# EUROPEAN PATENT SPECIFICATION

(11) **EP 3 374 504 B1**
(45) Date of publication and mention of the grant of the patent: **19.03.2025**
(21) Application number: 16794593.0
(22) Date of filing: 09.11.2016
(51) Int. Cl.: C12N 15/00, A61K 48/00, A61K 39/00

(54) **OPTIMIZED NUCLEIC ACID MOLECULES**
OPTIMIERTE NUKLEINSÄUREMOLEKÜLE
MOLÉCULES D'ACIDE NUCLÉIQUE OPTIMISÉES

(30) Priority: 09.11.2015 WO PCT/EP2015/002243; 20.11.2015 WO PCT/EP2015/002327
(43) Date of publication of application: 19.09.2018
(73) Proprietor: CureVac SE, 72076 Tübingen (DE)
(72) Inventor: BAUMHOF, Patrick, 72706 Tübingen (DE); RAUCH, Susanne, 72706 Tübingen (DE); KOWALCZYK, Aleksandra, 72706 Tübingen (DE); LUTZ, Johannes, 72706 Tübingen (DE); JASNY, Edith, 72706 Tübingen (DE); PETSCH, Benjamin, 72706 Tübingen (DE); THESS, Andreas, 72706 Tübingen (DE); SCHLAKE, Thomas, 72706 Tübingen (DE); FOTIN-MLECZEK, Mariola, 72706 Tübingen (DE); HEIDENREICH, Regina, 72706 Tübingen (DE); LAZZARO, Sandra, 72706 Tübingen (DE); DÖNER, Fatma, 72706 Tübingen (DE); GROßE, Wolfgang, 72706 Tübingen (DE)
(74) Representative: Graf von Stosch Patentanwaltsgesellschaft mbH
(86) International application number: PCT/EP2016/077145
(87) International publication number: WO 2017/081082

(56) References cited:
- EP-A1- 2 913 400
- WO-A1-2011/035004
- WO-A1-2012/071631
- WO-A1-2014/081507
- WO-A1-2014/081507
- WO-A1-2014/140176
- WO-A1-2014/144756
- WO-A1-2014/152940
- WO-A2-02/098443
- WO-A2-02/098443
- WO-A2-2005/039643
- WO-A2-2012/050923
- WO-A2-2012/050923
- WO-A2-2012/122334
- WO-A2-2012/165927
- DE-A1- 102013 111 099
- US-A- 6 099 847
- US-A1- 2003 054 337
- US-A1- 2005 032 730
- US-A1- 2010 183 652
- US-A1- 2013 316 952
- US-A1- 2013 316 952
- US-A1- 2014 255 377
- US-A1- 2014 255 377
- US-A1- 2015 064 236
- STORK R ET AL: "A novel tri-functional antibody fusion protein with improved pharmacokinetic properties generated by fusing a bispecific single-chain diabody with an albumin-binding domain from streptococcal protein G", PROTEIN ENGINEERING, DESIGN AND SELECTION, OXFORD JOURNAL, LONDON, GB, vol. 20, no. 11, 1 November 2007 (2007-11-01), pages 569 - 576, XP002515447, ISSN: 1741-0126, [retrieved on 20071103], DOI: 10.1093/PROTEIN/GZM061
- J. T. ANDERSEN ET AL: "Extending Half-life by Indirect Targeting of the Neonatal Fc Receptor (FcRn) Using a Minimal Albumin Binding Domain", JOURNAL OF BIOLOGICAL CHEMISTRY, vol. 286, no. 7, 18 February 2011 (2011-02-18), pages 5234 - 5241, XP055019819, ISSN: 0021-9258, DOI: 10.1074/jbc.M110.164848
- ODILE E. LEVY ET AL: "Novel Exenatide Analogs with Peptidic Albumin Binding Domains: Potent Anti-Diabetic Agents with Extended Duration of Action", PLOS ONE, vol. 9, no. 2, 4 February 2014 (2014-02-04), pages e87704, XP055232184, DOI: 10.1371/journal.pone.0087704
- KARIKÓ KATALIN ET AL: "Incorporation of pseudouridine into mRNA yields superior nonimmunogenic vector with increased translational capacity and biological stability", MOLECULAR THERAPY, NATURE PUBLISHING GROUP, GB, vol. 16, no. 11, 1 November 2008 (2008-11-01), pages 1833 - 1840, XP002598556, ISSN: 1525-0024, [retrieved on 20080916], DOI: 10.1038/MT.2008.200
- FREJD F: "Albumin binding domains and half life extension in vivo (chapter 14.3)", 1 January 2012, THERAPEUTIC PROTEINS: STRATEGIES TO MODULATE THEIR PLASMA HALF-LIVES,, PAGE(S) 272 - 283, XP009167422
- ROLAND E KONTERMANN ED - JIN YONG-SU ET AL: "Strategies for extended serum half-life of protein therapeutics", CURRENT OPINION IN BIOTECHNOLOGY, vol. 22, no. 6, 20 August 2011 (2011-08-20), pages 868 - 876, XP028397475, ISSN: 0958-1669, [retrieved on 20110729], DOI: 10.1016/J.COPBIO.2011.06.012
- STORK R ET AL: "A novel tri-functional antibody fusion protein with improved pharmacokinetic properties generated by fusing a bispecific single-chain diabody with an albumin-binding domain from streptococcal protein G", PROTEIN ENGINEERING, DESIGN AND SELECTION, OXFORD JOURNAL, LONDON, GB, vol. 20, no. 11, 1 November 2007 (2007-11-01), pages 569 - 576, XP002515447, ISSN: 1741-0126, [retrieved on 20071103], DOI: 10.1093/PROTEIN/GZM061
- J. T. ANDERSEN ET AL: "Extending Half-life by Indirect Targeting of the Neonatal Fc Receptor (FcRn) Using a Minimal Albumin Binding Domain", JOURNAL OF BIOLOGICAL CHEMISTRY, vol. 286, no. 7, 18 February 2011 (2011-02-18), pages 5234 - 5241, XP055019819, ISSN: 0021-9258, DOI: 10.1074/jbc.M110.164848
- ODILE E. LEVY ET AL: "Novel Exenatide Analogs with Peptidic Albumin Binding Domains: Potent Anti-Diabetic Agents with Extended Duration of Action", PLOS ONE, vol. 9, no. 2, 4 February 2014 (2014-02-04), pages e87704, XP055232184, DOI: 10.1371/journal.pone.0087704
- KARIKÓ KATALIN ET AL: "Incorporation of pseudouridine into mRNA yields superior nonimmunogenic vector with increased translational capacity and biological stability", MOLECULAR THERAPY, NATURE PUBLISHING GROUP, GB, vol. 16, no. 11, 1 November 2008 (2008-11-01), pages 1833 - 1840, XP002598556, ISSN: 1525-0024, [retrieved on 20080916], DOI: 10.1038/MT.2008.200
- ROLAND E KONTERMANN ED - JIN YONG-SU ET AL: "Strategies for extended serum half-life of protein therapeutics", CURRENT OPINION IN BIOTECHNOLOGY, vol. 22, no. 6, 20 August 2011 (2011-08-20), pages 868 - 876, XP028397475, ISSN: 0958-1669, [retrieved on 20110729], DOI: 10.1016/J.COPBIO.2011.06.012
- FREJD F: "Albumin binding domains and half life extension in vivo (chapter 14.3)", 1 January 2012, THERAPEUTIC PROTEINS: STRATEGIES TO MODULATE THEIR PLASMA HALF-LIVES,, PAGE(S) 272 - 283, XP009167422
- UGUR SAHIN ET AL: "mRNA-based therapeutics - developing a new class of drugs", NATURE REVIEWS DRUG DISCOVERY, vol. 13, no. 10, 19 September 2014 (2014-09-19), GB, pages 759 - 780, XP055544673, ISSN: 1474-1776, DOI: 10.1038/nrd4278
- ANONYMOUS: "GSN:AZZ98806", 13 September 2012 (2012-09-13), XP055342508, Retrieved from the Internet <URL:http://ibis.internal.epo.org/exam/dbfetch.jsp?id=GSN:AZZ98806> [retrieved on 20170206]
- ANONYMOUS: "GSN:AZF69177", 24 March 2011 (2011-03-24), XP055342536, Retrieved from the Internet <URL:http://ibis.internal.epo.org/exam/dbfetch.jsp?id=GSN:AZF69177> [retrieved on 20170206]
- ANONYMOUS: "GSP:BAI65492", 6 December 2012 (2012-12-06), XP055342459, Retrieved from the Internet <URL:http://ibis.internal.epo.org/exam/dbfetch.jsp?id=GSP:BAI65492> [retrieved on 20170206]
- KIM S J ET AL: "Enhanced immunogenicity of human papillomavirus 16 L1 genetic vaccines fused to an ER-targeting secretory signal peptide and RANTES", GENE THERAPY, vol. 10, no. 15, 1 August 2003 (2003-08-01), GB, pages 1268 - 1273, XP055200266, ISSN: 0969-7128, DOI: 10.1038/sj.gt.3301997
- ANONYMOUS: "GSN:AZX04661", 7 June 2012 (2012-06-07), XP055342492, Retrieved from the Internet <URL:http://ibis.internal.epo.org/exam/dbfetch.jsp?id=GSN:AZX04661> [retrieved on 20170206]
- ANONYMOUS: "GSP:AZX04662", 7 June 2012 (2012-06-07), XP055342497, Retrieved from the Internet <URL:http://ibis.internal.epo.org/exam/dbfetch.jsp?id=GSP:AZX04662> [retrieved on 20170206]
- ANONYMOUS: "GSP:BBF74823", 1 May 2014 (2014-05-01), XP055342463, Retrieved from the Internet <URL:http://ibis.internal.epo.org/exam/dbfetch.jsp?id=GSP:BBF74823> [retrieved on 20170206]
- DU L ET AL: "A Recombinant Vaccine of H5N1 HA1 Fused with Foldon and Human IgG Fc Induced Complete Cross-Clade Protection against Divergent H5N1 Viruses", PLOS ONE, vol. 6, no. 1, January 2011 (2011-01-01), XP002753614, ISSN: 1932-6203
- GRODELAND G ET AL: "DNA Vaccine that Targets Hemagglutinin to MHC Class II Molecules Rapidly Induces Antibody-Mediated Protection against Influenza", THE JOURNAL OF IMMUNOLOGY, vol. 191, no. 6, 15 September 2013 (2013-09-15), pages 3221 - 3231, XP055120524, ISSN: 0022-1767, DOI: 10.4049/jimmunol.1300504
- NASKALSKA A ET AL: "Towards a novel influenza vaccine: engineering of hemagglutinin on a platform of adenovirus dodecahedron", BMC BIOTECHNOLOGY, vol. 13, June 2013 (2013-06-01), XP002753615, ISSN: 1472-6750
- ANONYMOUS: "GSP:AYG57506", 22 July 2010 (2010-07-22), XP055389031, Retrieved from the Internet <URL:http://ibis.internal.epo.org/exam/dbfetch.jsp?id=GSP:AYG57506> [retrieved on 20170707]
- ANONYMOUS: "GSP:AYG57791", 22 June 2010 (2010-06-22), XP055389089, Retrieved from the Internet <URL:http://ibis.internal.epo.org/exam/dbfetch.jsp?id=GSP:AYG57791> [retrieved on 20170707]
- ANONYMOUS: "USPOP:ABA64804", 7 October 2005 (2005-10-07), XP055388678, Retrieved from the Internet <URL:http://ibis.internal.epo.org/exam/dbfetch.jsp?id=USPOP:ABA64804> [retrieved on 20170706]
- ANONYMOUS: "GSP:BBN38267", 18 September 2014 (2014-09-18), XP055388967, Retrieved from the Internet <URL:http://ibis.internal.epo.org/exam/dbfetch.jsp?id=GSP:BBN38267> [retrieved on 20170707]
- ANONYMOUS: "GSP:BBN38268", 18 September 2014 (2014-09-18), XP055388976, Retrieved from the Internet <URL:http://ibis.internal.epo.org/exam/dbfetch.jsp?id=GSP:BBN38268> [retrieved on 20170707]
- ANONYMOUS: "GSP:BBN38275", 18 September 2014 (2014-09-18), XP055388971, Retrieved from the Internet <URL:http://ibis.internal.epo.org/exam/dbfetch.jsp?id=GSP:BBN38275> [retrieved on 20170707]
- ANONYMOUS: "GSP:BBN38276", 18 September 2014 (2014-09-18), XP055388973, Retrieved from the Internet <URL:http://ibis.internal.epo.org/exam/dbfetch.jsp?id=GSP:BBN38276> [retrieved on 20170707]
- ANONYMOUS: "GSP:BBN38281", 18 September 2014 (2014-09-18), XP055388966, Retrieved from the Internet <URL:http://ibis.internal.epo.org/exam/dbfetch.jsp?id=GSP:BBN38281> [retrieved on 20170707]
- ANONYMOUS: "GSP:BBN38283", 18 September 2014 (2014-09-18), XP055388969, Retrieved from the Internet <URL:http://ibis.internal.epo.org/exam/dbfetch.jsp?id=GSP:BBN38283> [retrieved on 20170707]
- ANONYMOUS: "GSP:BBN38292", 18 September 2014 (2014-09-18), XP055388974, Retrieved from the Internet <URL:http://ibis.internal.epo.org/exam/dbfetch.jsp?id=GSP:BBN38292> [retrieved on 20170707]
- ROBERT M-A ET AL: "Efficient Delivery of Nuclear and Cytoplasmic Proteins Fused to HIV-1 Gag Polypeptide By Means of Virus-Like Particles", MOLECULAR THERAPY. SUPPLEMENT 1, 1 May 2015 (2015-05-01), pages S122, XP055388527
- ZLOTNICK A ET AL: "Localization of the C terminus of the assembly domain of hepatitis B virus capsid protein: Implications for morphogenesis and organization of encapsidated RNA", PROCEEDINGS NATIONAL ACADEMY OF SCIENCES PNAS, NATIONAL ACADEMY OF SCIENCES, US, vol. 94, 1 September 1997 (1997-09-01), pages 9556 - 9561, XP002982400, ISSN: 0027-8424, DOI: 10.1073/PNAS.94.18.9556

## Description

The present invention concerns optimized nucleic acid molecules, methods for optimization of nucleic acid molecules and uses of optimized nucleic acid molecules, as well as biological entities comprising optimized nucleic acid molecules. Various aspects relating to optimization and to optimized nucleic acid molecules are subject of the present invention.

In general, deoxyribonucleic acid (DNA) and ribonucleic acid (RNA) are nucleic acid molecules which encode genetic information. In living cells of unicellular or multicellular organisms (*in vivo*), as well as in living cells isolated from multicellular organisms (*in vitro*), the encoded genetic information is translated into polypeptides and proteins by ribosomes. *In vitro* translation can also be achieved in cell-free systems comprising ribosomes, and appropriate reagents. These effects are standardly exploited in modern molecular biology, so that desired genetic information, in the form of DNA or RNA, can be added or provided to *in vitro* or *in vivo* environments containing ribosomes. In vivo uses in modern medicine include administration of nucleic acid molecules for therapeutic purposes, particularly in the context of gene therapy or genetic vaccination. The RNA species encoding the genetic information for translation into polypeptides and proteins by ribosomes is called messenger RNA (mRNA).

DNA is known to be relatively stable and easy to handle. However, the use of DNA bears the risk of undesired insertion of the administered DNA-fragments into the target cell's or target subject's (patient's) genome, potentially resulting mutagenic events such as in loss of function of the impaired genes. As a further risk of *in vivo* uses, the undesired generation of anti-DNA antibodies has emerged. Another drawback is the limited expression level of the encoded peptide or protein that is achievable upon DNA administration because the DNA must enter the nucleus in order to be transcribed before the resulting mRNA can be translated. Among other reasons, the expression level of the administered DNA will be dependent on the presence of specific transcription factors which regulate DNA transcription. In the absence of such factors, DNA transcription will not yield satisfying amounts of RNA. As a result, the level of translated peptide or protein obtained is limited.

By using RNA instead of DNA, the risk of undesired genomic integration and generation of anti-DNA antibodies is minimized or avoided. However, RNA has been traditionally considered to be a rather unstable molecular species which may readily be degraded by ubiquitous RNAses. Typically, RNA degradation contributes to the regulation of the RNA half-life time. That effect was considered and proven to fine tune the regulation of eukaryotic gene expression (Friedel et al., 2009. Conserved principles of mammalian transcriptional regulation revealed by RNA half-life, Nucleic Acid Research 37(17): 1-12). Accordingly, each naturally occurring mRNA has its individual half-life depending on the gene from which the mRNA is derived and in which cell type it is expressed. It contributes to the regulation of the expression level of this gene. Unstable RNAs are important to realize transient gene expression at distinct points in time. However, long-lived RNAs may be associated with accumulation of distinct proteins or continuous expression of genes. *In vivo,* the half-life of mRNAs may also be dependent on environmental factors, such as hormonal treatment, as has been shown, e.g., for insulin-like growth factor I, actin, and albumin mRNA (Johnson et al., Newly synthesized RNA: Simultaneous measurement in intact cells of transcription rates and RNA stability of insulin-like growth factor I, actin, and albumin in growth hormone-stimulated hepatocytes, Proc. Natl. Acad. Sci., Vol. 88, pp. 5287-5291, 1991).

For gene therapy and genetic vaccination, usually stable RNA is desired. This is, on the one hand, due to the fact that it is usually desired that the product encoded by the RNA sequence accumulates *in vivo.* On the other hand, the RNA has to maintain its structural and functional integrity when prepared for a suitable dosage form, in the course of its storage, and when administered. Thus, efforts were made to provide stable RNA molecules for gene therapy or genetic vaccination in order to prevent them from being subject to early degradation or decay.

It has been reported that the G/C-content of nucleic acid molecules may influence their stability. Thus, nucleic acids comprising an increased amount of guanine (G) and/or cytosine (C) residues may be functionally more stable than nucleic acids containing a large amount of adenine (A) and thymine (T) or uracil (U) nucleotides. In this context, WO02/098443 provides a pharmaceutical composition containing an mRNA that is stabilised by sequence modifications in the coding region. Such a sequence modification takes advantage of the degeneracy of the genetic code. Accordingly, codons which contain a less favourable combination of nucleotides (less favourable in terms of RNA stability) may be substituted by alternative codons without altering the encoded amino acid sequence. This method of RNA stabilization is limited by the provisions of the specific nucleotide sequence of each single RNA molecule which is not allowed to leave the space of the desired amino acid sequence. Also, that approach is restricted to coding regions of the RNA.

As an alternative option for mRNA stabilisation, it has been found that naturally occurring eukaryotic mRNA molecules contain characteristic stabilising moieties. For example, they may comprise so-called untranslated regions (UTR) at their 5'-end (5'-UTR) and/or at their 3'-end (3'-UTR) as well as other structural features, such as a 5'-cap structure or a 3'-poly(A) tail. Both, 5'-UTR and 3'-UTR are typically transcribed from the genomic DNA and are, thus, a feature of the premature mRNA. Characteristic structural features of mature mRNA, such as the 5'-cap and the 3'-poly(A) tail (also called poly(A) tail or poly(A) sequence) are usually added to the transcribed (premature) mRNA during mRNA processing.

A 3'-poly(A) tail is typically a monotonous sequence stretch of adenosine nucleotides added to the 3'-end of the transcribed mRNA. It may comprise up to about 400 adenosine nucleotides. It was found that the length of such a 3'-poly(A) tail is potentially critical for the stability of individual mRNA.

Also, it was shown that the 3'-UTR of α-globin mRNA may be an important factor for the well-known stability of α-globin mRNA (Rodgers et al., Regulated α-globin mRNA decay is a cytoplasmic event proceeding through 3'-to-5' exosome-dependent decapping, RNA, 8, pp. 1526-1537, 2002). The 3'-UTR of α-globin mRNA is apparently involved in the formation of a specific ribonucleoprotein-complex, the α-complex, whose presence correlates with mRNA stability *in vitro* (Wang et al., An mRNA stability complex functions with poly(A)-binding protein to stabilize mRNA in vitro, Molecular and Cellular biology, Vol 19, No. 7, July 1999, p. 4552-4560).

An interesting regulatory function has further been demonstrated for the UTRs in ribosomal protein mRNAs: while the 5'-UTR of ribosomal protein mRNAs controls the growth-associated translation of the mRNA, the stringency of that regulation is conferred by the respective 3'-UTR in ribosomal protein mRNAs (Ledda et al., Effect of the 3'-UTR length on the translational regulation of 5'-terminal oligopyrimidine mRNAs, Gene, Vol. 344, 2005, p. 213-220). This mechanism contributes to the specific expression pattern of ribosomal proteins, which are typically transcribed in a constant manner so that some ribosomal protein mRNAs such as ribosomal protein S9 or ribosomal protein L32 are referred to as housekeeping genes (Janovick-Guretzky et al., Housekeeping Gene Expression in Bovine Liver is Affected by Physiological State, Feed Intake, and Dietary Treatment, J. Dairy Sci., Vol. 90, 2007, p. 2246-2252). The growth-associated expression pattern of ribosomal proteins is thus mainly due to regulation on the level of translation.

WO 2014/164253 A1 describes some specific nucleic acid molecules having 5'-UTRs and/or 3'-UTRs, without detailing on translation efficiency of such molecules.

US 2014/255377 describes pharmaceutical compositions containing albumin-binding arginine deiminase fusion protein (AAD) for treating cancer or other arginine-dependent diseases. Stork et al. (A novel tri-functional antibody fusion protein with improved pharmacokinetic properties generated by fusing a bispecific single-chain diabody with an albumin-binding domain from streptococcal protein G, Protein Engineering, Design and Selection, Oxford Journal, Vol. 20(11), 2007, p. 569-576) describe a tri-functional antibody fusion protein. Andersen et al. (Extending half-life by indirect targeting of the neonatal Fc receptor (FcRn) using a minimal albumin binding domain, Journal of Biological Chemistry, Vol. 286(7), 2011, p. 5234-5241) is directed to the extension of half-life by indirect targeting of neonatal FcRn. Levy et al. (Novel exenatide analogs with peptidic albumin binding domains: Potent anti-diabetic agents with extended duration of action, PLOS ONE, Vol. 9(2), 2014, p. e87704) relates to exenatide analogs with peptidic albumin binding domains. WO 02/098443 concerns pharmaceutical compositions comprising mRNA having sequence modifications in the translated region and being optimized for translation. WO 2014/081507 relates to compositions and methods for the manufacture and optimization of modified mRNA molecules via optimization of their terminal architecture. Karikó et al. (Incorporation of pseudouridine into mRNA yields superior nonimmunogenic vector with increased translational capacity and biological stability, Mol. Ther., Vol. 16(11), 2008, p. 1833-40) is directed to the incorporation of pseudouridine into mRNA. Frejd (Half-Life Extension by Binding to Albumin through an Albumin Binding Domain, Therapeutic Proteins: Strategies to Modulate Their Plasma Half-Lives, 2012, p. 272-283) concerns the extension of half-life of albumin through an albumin binding domain. Kontermann (Strategies for extended serum half-life of protein therapeutics, Curr Opin Biotechnol., Vol. 22(6), 2011, 868-876) relates to the half-life extension of protein therapeutics. WO 2012/050923 regards protein modifications for half-life extension. WO 2014/52940 relates to an mRNA for gene therapy or genetic vaccination with prolonged half-life, wherein the encoded polypeptide of interest is fused to a polypeptide capable of binding to an Fc receptor. US 2015/064236 relates to an mRNA for gene therapy or genetic vaccination with prolonged half-life, wherein the encoded polypeptide of interest is fused to a further polypeptide.

Irrespective of factors influencing mRNA stability, effective translation of the administered nucleic acid molecules by the target cells or tissue is crucial for any approach using nucleic acid molecules for gene therapy or genetic vaccination. As can be seen from the examples cited above, along with the regulation of stability, also translation of the majority of mRNAs is regulated by structural features like UTRs, 5'-cap and 3'-poly(A) tail. In this context, it has been reported that the length of the poly(A) tail may play an important role for translation efficiency as well. Stabilizing 3'-moieties, however, may also have an attenuating effect on translation.

There is therefore a need for optimized nucleic acid molecules, particularly optimized RNA molecules, in general.

There is also a specific need for optimized nucleic acid molecules, particularly optimized RNA molecules, which are suitable for medical applications; particularly applications which involve the introduction of nucleic acids, such as DNA or RNA, into a subject's cell or tissue, followed by the translation of the information coded by the nucleic acids into the desired peptides or proteins. Beneficial characteristics of mRNA were discovered in the recent years and clinical development of mRNA-based therapeutics is in progress (reviewed in Sahin et al. 2014. Nat Rev Drug Discov. 2014 Oct;13(10):759-80. doi: 10.1038/nrd4278. Epub 2014 Sep 19; Kallen and Thess 2014. Ther Adv Vaccines. 2014 Jan;2(1):10-31. doi: 10.1177/2051013613508729. Review).

In summary, mRNA represents a transient copy of the coded genetic information in all organisms. Hence, mRNA constructs may serve as a model for the synthesis of an unlimited variety of target proteins and, unlike DNA, represents all the necessary prerequisites for the preparation of a suitable vector for the transfer of exogenous genetic information *in vivo.*

It is an object of the invention to provide nucleic acid molecules which may be suitable for application in gene therapy and/or genetic vaccination. Particularly, it is the object of the invention to provide versatile RNA species which are stabilized against undesired degradation or decay. Another object of the present invention is to provide nucleic acid molecules coding for such a superior mRNA species which may be amenable for use in gene therapy and/or genetic vaccination. It is a further object of the present invention to provide a pharmaceutical composition for use in gene therapy and/or genetic vaccination. In summary, it is the object of the present invention to provide improved nucleic acid species which overcome the above discussed disadvantages of the prior art by a cost-effective and straight-forward approach.

The object underlying the present invention is solved by the claimed subject matter directed to an mRNA molecule suitable for application in gene therapy and/or genetic vaccination, the mRNA molecule comprising a 5'-untranslated region (5'-UTR) and a 3'-untranslated region (3'-UTR), wherein the mRNA comprises at least two modules, wherein each module is a ribonucleic acid moiety,wherein at least one module is an open reading frame (ORF) encoding a polypeptide or protein of interest, and wherein at least one module is a further coding ribonucleic acid moiety encoding at least one additional polypeptide or protein element encoded in the same open reading frame as the polypeptide or protein of interest, wherein the at least one additional polypeptide or protein element is an element that extends protein half-life, and wherein the polypeptide or protein element is characterized by at least 80% identity to SEQ ID NO. 1705, and wherein the open reading frame is or comprises a G/C-modified nucleic acid sequence, wherein the number of G and/or C nucleotides is increased as compared to the wild-type sequence. Also provided is a method for preparing an mRNA molecule suitable for application in gene therapy and/or genetic vaccination, comprising at least the step of combining at least two modules, wherein each module is a ribonucleic acid moiety, and thereby preparing an mRNA molecule comprising a 5'-untranslated region (5'-UTR), a 3'-untranslated region (3'-UTR) and said at least two modules, wherein at least one module is an open reading frame encoding a polypeptide or protein of interest, wherein at least one module is a further coding ribonucleic acid moiety encoding at least one polypeptide or protein element in the same open reading frame as the polypeptide or protein of interest, wherein the at least one polypeptide or protein element is an element that extends protein half-life, wherein the polypeptide or protein element is characterized by at least 80% identity to SEQ ID NO. 1705, and wherein the open reading frame is or comprises a G/C-modified nucleic acid sequence, wherein the number of G and/or C nucleotides is increased as compared to the wild-type sequence. Further provided is a vector encoding the claimed mRNA molecule, a cell comprising said mRNA molecule or said vector, a pharmaceutical composition comprising said mRNA molecule or said vector or said cell and a pharmaceutically acceptable carrier and provided is an mRNA molecule as defined above and in the claims for use in a method of treatment of the human or animal body by therapy, preferably, for use in a method of gene therapy or genetic vaccination.

In particular, the inventors identified structural and functional aspects related to optimization of nucleic acid molecules, particularly RNA molecules. Such aspects are provided herein. The invention also provides a modular system for combining aspects of mRNA molecules. The present invention therefore allows the versatile combination of nucleic acid sequences, and thus provides numerous mRNA molecules based on the general principles disclosed herein and as claimed. For example, mRNA constructs that may serve for information carriers in protein therapies can be designed in a way to obtain sufficient protein expression avoiding the activation of the immune system. In contrast, mRNA constructs that serve for information carriers in vaccination should be designed in a way to activate the immune system in the most efficient manner, that is e.g., to activate a strong cellular response for tumour vaccines or to induce a strong humoral response for prophylactic vaccines.

### Terms and Definitions

For the sake of clarity and readability the following definitions are provided. Any technical feature mentioned for these definitions may be read on each and every embodiment of the invention according to the claims. Additional definitions and explanations may be specifically provided in the context of these embodiments.

Adaptive immune response: The adaptive immune response is typically understood to be an antigen-specific response of the immune system. Antigen specificity allows for the generation of responses that are tailored to specific pathogens or pathogen-infected cells. The ability to mount these tailored responses is usually maintained in the body by "memory cells". Should a pathogen infect the body more than once, these specific memory cells are used to quickly eliminate it. In this context, the first step of an adaptive immune response is the activation of naïve antigen-specific T cells or different immune cells able to induce an antigen-specific immune response by antigen-presenting cells. This occurs in the lymphoid tissues and organs through which naive T cells are constantly passing. The three cell types that may serve as antigen-presenting cells are dendritic cells, macrophages, and B cells. Each of these cells has a distinct function in eliciting immune responses. Dendritic cells may take up antigens by phagocytosis and macropinocytosis and may become stimulated by contact with e.g. a foreign antigen to migrate to the local lymphoid tissue, where they differentiate into mature dendritic cells. Macrophages ingest particulate antigens such as bacteria and are induced by infectious agents or other appropriate stimuli to express MHC molecules. The unique ability of B cells to bind and internalize soluble protein antigens via their receptors may also be important to induce T cells. MHC-molecules are, typically, responsible for presentation of an antigen to T-cells. Therein, presenting the antigen on MHC molecules leads to activation of T cells which induces their proliferation and differentiation into armed effector T cells. The most important function of effector T cells is the killing of infected cells by CD8+ cytotoxic T cells and the activation of macrophages by Th1 cells which together make up cell-mediated immunity, and the activation of B cells by both Th2 and Th1 cells to produce different classes of antibody, thus driving the humoral immune response. T cells recognize an antigen by their T cell receptors which do not recognize and bind the antigen directly, but instead recognize short peptide fragments e.g. of pathogen-derived protein antigens, e.g. so-called epitopes, which are bound to MHC molecules on the surfaces of other cells.

Adaptive immune system: The adaptive immune system is essentially dedicated to eliminate or prevent pathogenic growth. It typically regulates the adaptive immune response by providing the vertebrate immune system with the ability to recognize and remember specific pathogens (to generate immunity), and to mount stronger attacks each time the pathogen is encountered. The system is highly adaptable because of somatic hypermutation (a process of accelerated somatic mutations), and V(D)J recombination (an irreversible genetic recombination of antigen receptor gene segments). This mechanism allows a small number of genes to generate a vast number of different antigen receptors, which are then uniquely expressed on each individual lymphocyte. Because the gene rearrangement leads to an irreversible change in the DNA of each cell, all of the progeny (offspring) of such a cell will then inherit genes encoding the same receptor specificity, including the Memory B cells and Memory T cells that are the keys to long-lived specific immunity.

Adjuvant/adjuvant component: An adjuvant or an adjuvant component in the broadest sense is typically a pharmacological and/or immunological agent that may modify, e.g. enhance, the effect of other agents, such as a drug or vaccine. It is to be interpreted in a broad sense and refers to a broad spectrum of substances. Typically, these substances are able to increase the immunogenicity of antigens. For example, adjuvants may be recognized by the innate immune systems and, e.g., may elicit an innate immune response. "Adjuvants" typically do not elicit an adaptive immune response. Insofar, "adjuvants" do not qualify as antigens. Their mode of action is distinct from the effects triggered by antigens resulting in an adaptive immune response.

Antigen: In the context of the present invention "antigen" refers typically to a substance which may be recognized by the immune system, preferably by the adaptive immune system, and is capable of triggering an antigen-specific immune response, e.g. by formation of antibodies and/or antigen-specific T cells as part of an adaptive immune response. Typically, an antigen may be or may comprise a peptide or protein which may be presented by the MHC to T-cells. In the sense of the present invention an antigen may be the product of translation of a provided nucleic acid molecule, preferably an mRNA as defined herein. In this context, also fragments, variants and derivatives of peptides and proteins comprising at least one epitope are understood as antigens. In the context of the present invention, tumour antigens and pathogenic antigens as defined herein are particularly preferred.

Artificial nucleic acid molecule: An artificial nucleic acid molecule may typically be understood to be a nucleic acid molecule - e.g. DNA or RNA - that does not occur naturally. In other words, an artificial nucleic acid molecule may be understood as a non-natural nucleic acid molecule. Such nucleic acid molecule may be non-natural due to its individual sequence (which does not occur naturally) and/or due to other modifications, e.g. structural modifications of nucleotides which do not occur naturally. An artificial nucleic acid molecule may be a DNA molecule, an RNA molecule or a hybrid-molecule comprising DNA and RNA portions. Typically, artificial nucleic acid molecules may be designed and/or generated by genetic engineering methods to correspond to a desired artificial sequence of nucleotides (heterologous sequence). In this context an artificial sequence is usually a sequence that may not occur naturally, i.e. it differs from the wild-type sequence by at least one nucleotide. The term "wild-type" may be understood as a sequence occurring in nature. When any particular "artificial nucleic acid molecule" is described herein to be "based on" any particular wild-type nucleic acid molecule, then said artificial nucleic acid molecule differs from said wild-type nucleic acid molecule by at least one nucleotide. Further, the term "artificial nucleic acid molecule" is not restricted to mean "one single molecule" but is, typically, understood to comprise an ensemble of identical molecules. Accordingly, it may relate to a plurality of identical molecules contained in an aliquot. Optimized nucleic acid molecules, as described herein, fall under the term "artificial nucleic acid molecules". Further properties of mRNA molecules of the invention are described herein below.

Bicistronic RNA multicistronic RNA: A bicistronic or multicistronic RNA is typically an RNA, preferably an mRNA that typically may have two (bicistronic) or more (multicistronic) open reading frames (ORF). An open reading frame in this context is a sequence of codons that is translatable into a peptide or protein.

Carrier / polymeric carrier: A carrier in the context of the invention is any compound that facilitates transport and/or complexation of another compound. Said other compound can be referred to as "cargo". A polymeric carrier is typically a carrier that is formed of a polymeric molecule. A carrier may be associated to its cargo by covalent or non-covalent interaction. A carrier may transport nucleic acids, e.g. RNA or DNA, to the target cells. The carrier may - in some embodiments - be a cationic component.

Cationic component: The term "cationic component" typically refers to a charged molecule, which is positively charged (cation) at a pH value typically from 1 to 9, preferably at a pH value of or below 9 (e.g. from 5 to 9), of or below 8 (e.g. from 5 to 8), of or below 7 (e.g. from 5 to 7), most preferably at a physiological pH, e.g. from 7.3 to 7.4. Accordingly, a cationic component may be any positively charged compound or polymer, preferably a cationic peptide or protein which is positively charged under physiological conditions, particularly under physiological conditions *in vivo.* A "cationic peptide or protein" may contain at least one positively charged amino acid, or more than one positively charged amino acid, e.g. selected from Arg, His, Lys or Orn. Accordingly, "polycationic" components are also within the scope exhibiting more than one positive charge under the conditions given.

5'-cap: A 5'-cap is an entity, typically a modified nucleotide entity, which generally "caps" the 5'-end of a mature mRNA. A 5'-cap may typically be formed by a modified nucleotide, particularly by a derivative of a guanine nucleotide. Preferably, the 5'-cap is linked to the 5'-terminus via a 5'-5'-triphosphate linkage. A 5'-cap may be methylated, e.g. m7GpppN, wherein N is the terminal 5' nucleotide of the nucleic acid carrying the 5'-cap, typically the 5'-end of an RNA. Further examples of 5'cap structures include glyceryl, inverted deoxy abasic residue (moiety), 4',5' methylene nucleotide, 1-(beta-D-erythrofuranosyl) nucleotide, 4'-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3,5 dihydroxypentyl nucleotide, 3'-3'-inverted nucleotide moiety, 3'-3'-inverted abasic moiety, 3'-2'-inverted nucleotide moiety, 3'-2'-inverted abasic moiety, 1,4-butanediol phosphate, 3'-phosphoramidate, hexylphosphate, aminohexyl phosphate, 3'-phosphate, 3'phosphorothioate, phosphorodithioate, or bridging or non-bridging methylphosphonate moiety.

Cellular immunity/cellular immune response: Cellular immunity relates typically to the activation of macrophages, natural killer cells (NK), antigen-specific cytotoxic T-lymphocytes, and the release of various cytokines in response to an antigen. In more general terms, cellular immunity is not based on antibodies, but on the activation of cells of the immune system. Typically, a cellular immune response may be characterized e.g. by activating antigen-specific cytotoxic T-lymphocytes that are able to induce apoptosis in cells, e.g. specific immune cells like dendritic cells or other cells, displaying epitopes of foreign antigens on their surface. Such cells may be virus-infected or infected with intracellular bacteria, or cancer cells displaying tumor antigens. Further characteristics may be activation of macrophages and natural killer cells, enabling them to destroy pathogens and stimulation of cells to secrete a variety of cytokines that influence the function of other cells involved in adaptive immune responses and innate immune responses.

DNA: DNA is the usual abbreviation for deoxy-ribonucleic acid. It is a nucleic acid molecule, i.e. a polymer consisting of nucleotides. These nucleotides are usually deoxy-adenosine-monophosphate, deoxy-thymidine-monophosphate, deoxy-guanosine-monophosphate and deoxy-cytidine-monophosphate monomers which are - by themselves - composed of a sugar moiety (deoxyribose), a base moiety and a phosphate moiety, and polymerise by a characteristic backbone structure. The backbone structure is, typically, formed by phosphodiester bonds between the sugar moiety of the nucleotide, i.e. deoxyribose, of a first and a phosphate moiety of a second, adjacent monomer. The specific order of the monomers, i.e. the order of the bases linked to the sugar/phosphate-backbone, is called the DNA sequence. DNA may be single stranded or double stranded. In the double stranded form, the nucleotides of the first strand typically hybridize with the nucleotides of the second strand, e.g. by A/T-base-pairing and G/C-base-pairing.

Element: An element, as used herein, generally refers to a polypeptide sub-sequence. Typically, more than one polypeptide sub-sequences are arranged in linear order, so that several same or different sub-sequences or elements are typically present in a polypeptide sequence. Without limiting the technical content, the term "element" is used herein to refer to a module on polypeptide or protein level. This use reflects the general use in the art for polypeptide or protein elements, as illustrated e.g. by the well-known term "transmembrane element". However, as used herein, the term "element" is not limited to those polypeptide or protein modules that have been termed "element" in the prior art, but generally refers to a polypeptide or protein sub-sequence or module, as defined herein. Typically, an element is encoded by a nucleic acid module (moiety), as defined herein.

Epitope: (also called "antigen determinant") can be distinguished in T cell epitopes and B cell epitopes. T cell epitopes or parts of the proteins in the context of the present invention may comprise fragments preferably having a length of about 6 to about 20 or even more amino acids, e.g. fragments as processed and presented by MHC class I molecules, preferably having a length of about 8 to about 10 amino acids, e.g. 8, 9, or 10, (or even 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T cells in form of a complex consisting of the peptide fragment and an MHC molecule, i.e. the fragments are typically not recognized in their native form. B cell epitopes are typically fragments located on the outer surface of (native) protein or peptide antigens as defined herein, preferably having 5 to 15 amino acids, more preferably having 5 to 12 amino acids, even more preferably having 6 to 9 amino acids, which may be recognized by antibodies, i.e. in their native form.

Such epitopes of proteins or peptides may furthermore be selected from any of the herein mentioned variants of such proteins or peptides. In this context antigenic determinants can be conformational or discontinuous epitopes which are composed of segments of the proteins or peptides as defined herein that are discontinuous in the amino acid sequence of the proteins or peptides as defined herein but are brought together in the three-dimensional structure or continuous or linear epitopes which are composed of a single polypeptide chain.

Fragment of a sequence: A fragment of a sequence may typically be a shorter portion of a full-length sequence of e.g. a nucleic acid molecule or an amino acid sequence. Accordingly, a fragment, typically, consists of a sequence that is identical to the corresponding stretch within the full-length sequence. A preferred fragment of a sequence in the context of the present invention, consists of a continuous stretch of entities, such as nucleotides or amino acids corresponding to a continuous stretch of entities in the molecule the fragment is derived from, which represents at least 5%, 10%, 20%, preferably at least 30%, more preferably at least 40%, more preferably at least 50%, even more preferably at least 60%, even more preferably at least 70%, and most preferably at least 80% of the total (i.e. full-length) molecule from which the fragment is derived.

G/C modified: A G/C-modified nucleic acid may typically be a nucleic acid, preferably an artificial nucleic acid molecule as defined herein, based on a modified wild-type sequence comprising a preferably increased number of guanosine and/or cytosine nucleotides as compared to the wild-type sequence. Such an increased number may be generated by substitution of codons containing adenosine or thymidine nucleotides by codons containing guanosine or cytosine nucleotides. If the enriched G/C content occurs in a coding region of DNA or RNA, it makes use of the degeneracy of the genetic code. Accordingly, the codon substitutions preferably do not alter the encoded amino acid residues, but exclusively increase the G/C content of the nucleic acid molecule. An artificial nucleic acid molecule, which is G/C modified and which therefore exhibits at least one superior property with respect to a non-G/C optimized nucleic acid molecule encoding the same polypeptide, is termed "optimized nucleic acid molecule".

Gene therapy: Gene therapy may typically be understood to mean a treatment of a patient's body or isolated elements of a patient's body, for example isolated tissues/cells, by nucleic acids encoding a peptide or protein. It typically may comprise at least one of the steps of a) administration of a nucleic acid, preferably an optimized nucleic acid molecule as defined herein, directly to the patient - by any suitable administration route - or *in vitro* to isolated cells/tissues of the patient, which results in transfection of the patient's cells either *in vivo*/*ex vivo* or *in vitro;* b) transcription and/or translation of the introduced nucleic acid molecule; and optionally c) re-administration of isolated, transfected cells to the patient, if the nucleic acid has not been administered directly to the patient.

Genetic vaccination: Genetic vaccination may typically be understood to be vaccination by administration of a nucleic acid molecule encoding an antigen or an immunogen or fragments thereof. The nucleic acid molecule may be administered to a subject's body or to isolated cells of a subject. Upon transfection of certain cells of the body or upon transfection of the isolated cells, the antigen or immunogen may be expressed by those cells and subsequently presented to the immune system, eliciting an adaptive, i.e. antigen-specific immune response. Accordingly, genetic vaccination typically comprises at least one of the steps of a) administration of a nucleic acid, preferably an optimized nucleic acid molecule as defined herein, to a subject, preferably a patient, or to isolated cells of a subject, preferably a patient, which usually results in transfection of the subject's cells either *in vivo* or *in vitro*; b) transcription and/or translation of the introduced nucleic acid molecule; and optionally c) re-administration of isolated, transfected cells to the subject, preferably the patient, if the nucleic acid has not been administered directly to the patient.

Heterologous sequence: Two sequences are typically understood to be 'heterologous' if they are not derivable from the same gene. I.e., although heterologous sequences may be derivable from the same organism, they naturally (in nature) do not occur in the same nucleic acid molecule, such as in the same mRNA.

Humoral immunity/humoral immune response: Humoral immunity refers typically to antibody production and optionally to accessory processes accompanying antibody production. A humoral immune response may be typically characterized, e.g., by Th2 activation and cytokine production, germinal centre formation and isotype switching, affinity maturation and memory cell generation. Humoral immunity also typically may refer to the effector functions of antibodies, which include pathogen and toxin neutralization, classical complement activation, and opsonin promotion of phagocytosis and pathogen elimination.

Immunogen: In the context of the present invention an immunogen may be typically understood to be a compound that is able to stimulate an immune response. Preferably, an immunogen is a peptide, polypeptide, or protein. In a particularly preferred embodiment, an immunogen in the sense of the present invention is the product of translation of a provided nucleic acid molecule, preferably an optimized nucleic acid molecule as defined herein. Typically, an immunogen elicits at least an adaptive immune response.

Immunostimulatory composition: An immunostimulatory composition may be typically understood to be a composition containing at least one component which is able to induce an immune response or from which a component which is able to induce an immune response is derivable. Such immune response may be preferably an innate immune response or a combination of an adaptive and an innate immune response. Preferably, an immunostimulatory composition contains at least one mRNA molecule as defined in the claims. The mRNA may be complexed with a suitable carrier. Thus, the immunostimulatory composition may comprise an mRNA/carrier-complex. Furthermore, the immunostimulatory composition may comprise an adjuvant and/or a suitable vehicle for the mRNA as defined in the claims.

Immune response: An immune response may typically be a specific reaction of the adaptive immune system to a particular antigen (so called specific or adaptive immune response) or an unspecific reaction of the innate immune system (so called unspecific or innate immune response), or a combination thereof.

Immune system: The immune system may protect organisms from infection. If a pathogen succeeds in passing a physical barrier of an organism and enters this organism, the innate immune system provides an immediate, but non-specific response. If pathogens evade this innate response, vertebrates possess a second layer of protection, the adaptive immune system. Here, the immune system adapts its response during an infection to improve its recognition of the pathogen. This improved response is then retained after the pathogen has been eliminated, in the form of an immunological memory, and allows the adaptive immune system to mount faster and stronger attacks each time this pathogen is encountered. According to this, the immune system comprises the innate and the adaptive immune system. Each of these two parts typically contains so called humoral and cellular components.

Immunostimulatory RNA: An immunostimulatory RNA (isRNA) may typically be an RNA that is able to induce an innate immune response. It usually does not have an open reading frame and thus does not provide a peptide-antigen or immunogen but elicits an immune response e.g. by binding to a specific kind of Toll-like-receptor (TLR) or other suitable receptors. However, of course also mRNAs having an open reading frame and coding for a peptide/protein may induce an innate immune response and, thus, may be immunostimulatory RNAs.

Innate immune system: The innate immune system, also known as non-specific (or unspecific) immune system, typically comprises the cells and mechanisms that defend the host from infection by other organisms in a non-specific manner. This means that the cells of the innate system may recognize and respond to pathogens in a generic way, but unlike the adaptive immune system, it does not confer long-lasting or protective immunity to the host. The innate immune system may be, e.g., activated by ligands of Toll-like receptors (TLRs) or other auxiliary substances such as lipopolysaccharides, TNF-alpha, CD40 ligand, or cytokines, monokines, lymphokines, interleukins or chemokines, IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta, TNF-alpha, growth factors, and hGH, a ligand of human Toll-like receptor TLR1, TLR2, TLR3, TLR4, TLR5, TLR6, TLR7, TLR8, TLR9, TLR10, a ligand of murine Toll-like receptor TLR1, TLR2, TLR3, TLR4, TLR5, TLR6, TLR7, TLR8, TLR9, TLR10, TLR11, TLR12 or TLR13, a ligand of a NOD-like receptor, a ligand of a RIG-I like receptor, an immunostimulatory nucleic acid, an immunostimulatory RNA (isRNA), a CpG-DNA, an antibacterial agent, or an anti-viral agent. The pharmaceutical composition as claimed comprises one or more such substances. Typically, a response of the innate immune system includes recruiting immune cells to sites of infection, through the production of chemical factors, including specialized chemical mediators, called cytokines; activation of the complement cascade; identification and removal of foreign substances present in organs, tissues, the blood and lymph, by specialized white blood cells; activation of the adaptive immune system; and/or acting as a physical and chemical barrier to infectious agents.

Cloning site: A cloning site is typically understood to be a segment of a nucleic acid molecule, which is suitable for insertion of a nucleic acid sequence, e.g., a nucleic acid sequence comprising an open reading frame. Insertion may be performed by any molecular biological method known to the one skilled in the art, e.g. by restriction and ligation. A cloning site typically comprises one or more restriction enzyme recognition sites (restriction sites). These one or more restrictions sites may be recognized by restriction enzymes which cleave the DNA at these sites. A cloning site which comprises more than one restriction site may also be termed a multiple cloning site (MCS) or a polylinker.

Module: A module, as used herein, generally refers to a polypeptide sub-sequence or a polynucleotide sub-sequence. A sub-sequence is a sequence forming part of a sequence. In the present invention, a modular design principle is provided that is suitable to generate a polypeptide sequence or a polynucleotide sequence comprising several (more than one) sub-sequences or modules. Typically, the respective sub-sequences are arranged in linear order. Thus, several same or different sub-sequences or modules are typically present in a polypeptide sequence or polynucleotide sequence, respectively. Without limiting the technical content, the term "moiety" is used herein to refer to a module on nucleic acid level, and the term "element" is used herein to refer to a module on polypeptide or protein level.

Moiety: A moiety, as used herein, generally refers to a polynucleotide sub-sequence. Typically, more than one polynucleotide sub-sequences are arranged in linear order, so that several same or different sub-sequences or moieties are typically present in a polynucleotide sequence. Without limiting the technical content, the term "moiety" is used herein to refer to a module on nucleic acid level. This is reflects the use of this term e.g. in the area of combinatorial chemistry, where said term is generally used to refer to one of the portions into which a given molecule can be (e.g. mentally) divided. Thus, herein, the term moiety refers to a portion of a nucleic acid molecule; the nucleic acid molecule can be (e.g. mentally) divided into several moieties. A moiety may encode a polypeptide or protein module (element), as defined herein, or may be a non-coding moiety.

Nucleic acid molecule: A nucleic acid molecule is a molecule comprising, preferably consisting of nucleic acid components. The term nucleic acid molecule preferably refers to DNA or RNA molecules. It is preferably used synonymous with the term "polynucleotide". Preferably, a nucleic acid molecule is a polymer comprising or consisting of nucleotide monomers which are covalently linked to each other by phosphodiester-bonds of a sugar/phosphate-backbone. The term "nucleic acid molecule" also encompasses modified nucleic acid molecules, such as base-modified, sugar-modified or backbone-modified etc. DNA or RNA molecules.

Open reading frame: An open reading frame (ORF) in the context of the invention may typically be a sequence of several nucleotide triplets which may be translated into a peptide or protein. An open reading frame preferably contains a start codon, i.e. a combination of three subsequent nucleotides coding usually for the amino acid methionine (ATG), at its 5'-end and a subsequent region which usually exhibits a length which is a multiple of 3 nucleotides. An ORF is preferably terminated by a stop-codon (e.g., TAA, TAG, and TGA). Typically, this is the only stop-codon of the open reading frame. Thus, an open reading frame in the context of the present invention is preferably a nucleotide sequence, consisting of a number of nucleotides that may be divided by three, which starts with a start codon (e.g. ATG) and which preferably terminates with a stop codon (e.g., TAA, TGA, or TAG). The open reading frame may be isolated or it may be incorporated in a longer nucleic acid sequence, for example in a vector or an mRNA. An open reading frame may also be termed "protein coding region" or "coding sequence" (cds).

Optimized nucleic acid molecule: In general, an optimized nucleic acid molecule is a nucleic acid molecule not found in nature. In other words, it is an artificial nucleic acid molecule, i.e. not a wild-type nucleic acid molecule. The nucleic acid molecule of the present invention is distinguished from a wild-type nucleic acid molecule by at least one structural feature. The distinguishing structural feature is selected from sequence modifications and base modifications. As described herein below, a sequence modification alters the polynucleotide sequence with respect to a wild-type nucleic acid molecule. Such sequence modification is typically selected among an addition, a deletion, an insertion and a substitution of one or more nucleic acid residues, with respect to a wild-type nucleic acid molecule. More than one such sequence modifications can be present in an optimized nucleic acid molecule. As described in detail below, the optimized nucleic acid molecules of the present invention, i.e. the mRNA molecule as defined in the claims, allow for the versatile combination of multiple polypeptide or protein elements, encoded by respective nucleic acid moieties. Thus, preferably, the optimized nucleic molecule of the present invention is characterized by at least one addition of one or more nucleic acid residues, in practice, addition of at least one nucleic acid moiety (coding or non-coding), as described herein. Said addition is preferably realized 5' or 3' with respect to a starting (e.g. wild-type) nucleic acid molecule. Base modification, as described below, means that at least one base of a nucleic acid or (deoxyribonucleic acid or ribonucleic acid) is altered. In any event, the at least one distinguishing structural feature provides - or contributes to - a functional property of the optimized nucleic acid molecule which is not exhibited by the non-optimized (wild-type) nucleic acid molecule. The mRNA molecule as defined in the claims and defined above is an optimized nucleic acid molecule.

Peptide: A peptide or oligopeptide or polypeptide is typically a polymer of at least two amino acid monomers, linked by peptide bonds. An oligopeptide typically contains less than 50 monomer units, although the term peptide or oligopeptide is not a disclaimer for molecules having more than 50 monomer units. Polypeptides typically have between 50 and 600 monomer units, although the term polypeptide is neither a disclaimer for molecules having more than 600 monomer units, nor for molecules having less than 50 monomer units. Large peptides, i.e. peptides typically having more than 50 monomer units, or even more than 600 monomer units, are also referred to as proteins.

Pharmaceutically effective amount: A pharmaceutically effective amount in the context of the invention is typically understood to be an amount that is sufficient to induce a pharmaceutical effect, such as an immune response, altering a pathological level of an expressed peptide or protein, or substituting a lacking gene product, e.g., in case of a pathological situation.

Protein: A protein typically comprises one or more peptides or polypeptides. A protein is typically folded into 3-dimensional form, which may be required for the protein to exert its biological function.

Poly(A) sequence: A poly(A) sequence, also called poly(A) tail or 3'-poly(A) tail, is typically understood to be a sequence of adenosine nucleotides, e.g., of up to about 400 adenosine nucleotides, e.g. from about 20 to about 400, preferably from about 50 to about 400, more preferably from about 50 to about 300, even more preferably from about 50 to about 250, most preferably from about 60 to about 250 adenosine nucleotides. A poly(A) sequence is typically located at the 3'end of an mRNA. In the context of the present invention, a poly(A) sequence may be located within an mRNA or any other nucleic acid molecule, such as, e.g., in a vector, for example, in a vector serving as template for the generation of an RNA, preferably an mRNA, e.g., by transcription of the vector.

Polyadenylation: Polyadenylation is typically understood to be the addition of a poly(A) sequence to a nucleic acid molecule, such as an RNA molecule, e.g. to a premature mRNA. Polyadenylation may be induced by a so called polyadenylation signal. This signal is preferably located within a stretch of nucleotides at the 3'-end of a nucleic acid molecule, such as an RNA molecule, to be polyadenylated. A polyadenylation signal typically comprises a hexamer consisting of adenine and uracil/thymine nucleotides, preferably the hexamer sequence AAUAAA. Other sequences, preferably hexamer sequences, are also conceivable. Polyadenylation typically occurs during processing of a pre-mRNA (also called premature-mRNA). Typically, RNA maturation (from pre-mRNA to mature mRNA) comprises the step of polyadenylation.

Restriction site: A restriction site, also termed restriction enzyme recognition site, is a nucleotide sequence recognized by a restriction enzyme. A restriction site is typically a short, preferably palindromic nucleotide sequence, e.g. a sequence comprising 4 to 8 nucleotides. A restriction site is preferably specifically recognized by a restriction enzyme. The restriction enzyme typically cleaves a nucleotide sequence comprising a restriction site at this site. In a double-stranded nucleotide sequence, such as a double-stranded DNA sequence, the restriction enzyme typically cuts both strands of the nucleotide sequence.

RNA mRNA: RNA is the usual abbreviation for ribonucleic acid. It is a nucleic acid molecule, i.e. a polymer consisting of nucleotides. These nucleotides are usually adenosine-monophosphate, uridine-monophosphate, guanosine-monophosphate and cytidine-monophosphate monomers which are connected to each other along a so-called backbone. The backbone is formed by phosphodiester bonds between the sugar, i.e. ribose, of a first and a phosphate moiety of a second, adjacent monomer. The specific succession of the monomers is called the RNA-sequence. Usually RNA may be obtainable by transcription of a DNA-sequence, e.g., inside a cell. In eukaryotic cells, transcription is typically performed inside the nucleus or the mitochondria. Typically, transcription of DNA usually results in the so-called premature RNA which has to be processed into so-called messenger-RNA, usually abbreviated as mRNA. Processing of the premature RNA, e.g. in eukaryotic organisms, comprises a variety of different posttranscriptional-modifications such as splicing, 5'-capping, polyadenylation, export from the nucleus or the mitochondria and the like. The sum of these processes is also called maturation of RNA. The mature messenger RNA usually provides the nucleotide sequence that may be translated into an amino-acid sequence of a particular peptide or protein. Typically, a mature mRNA comprises a 5'-cap, a 5'-UTR, an open reading frame, a 3'-UTR and a poly(A) sequence. Aside from messenger RNA, several non-coding types of RNA exist which may be involved in regulation of transcription and/or translation.

Sequence of a nucleic acid molecule: The sequence of a nucleic acid molecule is typically understood to be the particular and individual order, i.e. the succession of its nucleotides. The sequence of a protein or peptide is typically understood to be the order, i.e. the succession of its amino acids.

Sequence identity: Two or more sequences are identical if they exhibit the same length and order of nucleotides or amino acids. The percentage of identity typically describes the extent to which two sequences are identical, i.e. it typically describes the percentage of nucleotides that correspond in their sequence position with identical nucleotides of a reference-sequence. For determination of the degree of identity, the sequences to be compared are considered to exhibit the same length, i.e. the length of the longest sequence of the sequences to be compared. This means that a first sequence consisting of 8 nucleotides is 80% identical to a second sequence consisting of 10 nucleotides comprising the first sequence. In other words, in the context of the present invention, identity of sequences preferably relates to the percentage of nucleotides of a sequence which have the same position in two or more sequences having the same length. Gaps are usually regarded as non-identical positions, irrespective of their actual position in an alignment.

Stabilized nucleic acid molecule: A stabilized nucleic acid molecule is a nucleic acid molecule, preferably a DNA or RNA molecule that is modified such, that it is more stable to disintegration or degradation, e.g., by environmental factors or enzymatic digest, such as by an exo- or endonuclease degradation, than the nucleic acid molecule without the modification. Preferably, a stabilized nucleic acid molecule in the context of the present invention is stabilized in a cell, such as a prokaryotic or eukaryotic cell, preferably in a mammalian cell, such as a human cell. The stabilization effect may also be exerted outside of cells, e.g. in a buffer solution etc., for example, in a manufacturing process for a pharmaceutical composition comprising the stabilized nucleic acid molecule.

Transfection: The term "transfection" refers to the introduction of nucleic acid molecules, such as DNA or RNA (e.g. mRNA) molecules, into cells, preferably into eukaryotic cells. In the context of the present invention, the term "transfection" encompasses any method known to the skilled person for introducing nucleic acid molecules into cells, preferably into eukaryotic cells, such as into mammalian cells. Such methods encompass, for example, electroporation, lipofection, e.g. based on cationic lipids and/or liposomes, calcium phosphate precipitation, nanoparticle based transfection, virus based transfection, or transfection based on cationic polymers, such as DEAE-dextran or polyethylenimine etc. Preferably, the introduction is non-viral.

Vaccine: A vaccine is typically understood to be a prophylactic or therapeutic material providing at least one antigen, preferably an immunogen. The antigen or immunogen may be derived from any material that is suitable for vaccination. For example, the antigen or immunogen may be derived from a pathogen, such as from bacteria or virus particles etc., or from a tumor or cancerous tissue. The antigen or immunogen stimulates the body's adaptive immune system to provide an adaptive immune response.

Vector: The term "vector" refers to a nucleic acid molecule, preferably to an artificial nucleic acid molecule. A vector in the context of the present invention is suitable for incorporating or harbouring a desired nucleic acid sequence, such as preferably an optimized nucleic acid molecule as described herein, comprising at least one open reading frame (ORF). Such vectors may be storage vectors, expression vectors, cloning vectors, transfer vectors etc. A storage vector is a vector which allows the convenient storage of a nucleic acid molecule, for example, of an mRNA molecule. Thus, the vector may comprise a sequence corresponding, e.g., to a desired mRNA sequence or a part thereof, such as a sequence corresponding to the open reading frame and the 3'-UTR and/or the 5'-UTR of an mRNA. An expression vector may be used for production of expression products such as RNA, e.g. mRNA, or peptides, polypeptides or proteins. For example, an expression vector may comprise sequences needed for transcription of a sequence stretch of the vector, such as a promoter sequence, e.g. an RNA polymerase promoter sequence. A cloning vector is typically a vector that contains a cloning site, which may be used to incorporate nucleic acid sequences into the vector. A cloning vector may be, e.g., a plasmid vector or a bacteriophage vector. A transfer vector may be a vector which is suitable for transferring nucleic acid molecules into cells or organisms, for example, viral vectors. A vector in the context of the present invention may be, e.g., an RNA vector or a DNA vector. Preferably, a vector is a DNA molecule. Preferably, a vector in the sense of the present invention comprises a cloning site, a selection marker, such as an antibiotic resistance factor, and a sequence suitable for multiplication of the vector, such as an origin of replication. Preferably, a vector in the context of the present invention is a plasmid vector.

Vehicle: A vehicle is typically understood to be a material that is suitable for storing, transporting, and/or administering a compound, such as a pharmaceutically active compound. For example, it may be a physiologically acceptable liquid which is suitable for storing, transporting, and/or administering a pharmaceutically active compound.

3'-untranslated region (3'-UTR): Generally, the term "3'-UTR" refers to a part of the artificial nucleic acid molecule of the invention, which is located 3' (i.e. "downstream") of an open reading frame and which is not translated into protein. Typically, a 3'-UTR is the part of an mRNA which is located between the protein coding region (open reading frame (ORF) or coding sequence (CDS)) and the poly(A) sequence of the mRNA. In the context of the present invention, a 3'-UTR is suitably comprised in the optimized nucleic acid molecule. The term 3'-UTR may also comprise moieties, which are not encoded in the template, from which an RNA is transcribed, but which are added after transcription during maturation, e.g. a poly(A) sequence. A 3'-UTR of the mRNA is not translated into an amino acid sequence. The 3'-UTR sequence is generally encoded by the gene which is transcribed into the respective mRNA during the gene expression process. The genomic sequence is first transcribed into pre-mature mRNA, which comprises optional introns. The pre-mature mRNA is then further processed into mature mRNA in a maturation process. This maturation process comprises the steps of 5'capping, splicing the pre-mature mRNA to excise optional introns and modifications of the 3'-end, such as polyadenylation of the 3'-end of the pre-mature mRNA and optional endo-/ or exonuclease cleavages etc. In the context of the present invention, a 3'-UTR corresponds to the sequence of a mature mRNA which is located between the stop codon of the protein coding region, preferably immediately 3' to the stop codon of the protein coding region, and the poly(A) sequence of the mRNA. The term "corresponds to" means that the 3'-UTR sequence may be an RNA sequence, such as in the mRNA sequence used for defining the 3'-UTR sequence, or a DNA sequence which corresponds to such RNA sequence. In the context of the present invention, the term "a 3'-UTR of a gene", is the sequence which corresponds to the 3'-UTR of the mature mRNA derived from this gene, i.e. the mRNA obtained by transcription of the gene and maturation of the pre-mature mRNA. The term "3'-UTR of a gene" encompasses the DNA sequence and the RNA sequence (both sense and antisense strand and both mature and immature) of the 3'-UTR. Preferably, the 3'UTRs have a length of more than 20, 30, 40 or 50 nucleotides.

5'-untranslated region (5'-UTR): Generally, the term "5'-UTR" refers to a part of the artificial nucleic acid molecule, which is located 5' (i.e. "upstream") of an open reading frame (ORF) and which is not translated into protein. A 5'-UTR is typically understood to be a particular section of messenger RNA (mRNA), which is located 5' of the open reading frame of the mRNA. In the context of the present invention, a 5'-UTR is preferably present 5' of an open reading frame encoding a polypeptide comprising a polypeptide of interest. Typically, the 5'-UTR starts with the transcriptional start site and ends one nucleotide before the start codon of the open reading frame. Preferably, the 5'UTRs have a length of more than 20, 30, 40 or 50 nucleotides. The 5'-UTR may comprise moieties for controlling gene expression, also called regulatory moieties. Such regulatory moieties may be, for example, ribosomal binding sites. The 5'-UTR may be posttranscriptionally modified, for example by addition of a 5'-CAP. A 5'-UTR of the mRNA is not translated into an amino acid sequence. The 5'-UTR sequence is generally encoded by the gene which is transcribed into the respective mRNA during the gene expression process. The genomic sequence is first transcribed into pre-mature mRNA, which comprises optional introns. The pre-mature mRNA is then further processed into mature mRNA in a maturation process. This maturation process comprises the steps of 5'capping, splicing the pre-mature mRNA to excise optional introns and modifications of the 3'-end, such as polyadenylation of the 3'-end of the pre-mature mRNA and optional endo-/ or exonuclease cleavages etc.. In the context of the present invention, a 5'-UTR corresponds to the sequence of a mature mRNA which is located between the start codon and, for example, the 5'-CAP. Preferably, the 5'-UTR corresponds to the sequence which extends from a nucleotide located 3' to the 5'-CAP, more preferably from the nucleotide located immediately 3' to the 5'-CAP, to a nucleotide located 5' to the start codon of the protein coding region, preferably to the nucleotide located immediately 5' to the start codon of the protein coding region. The nucleotide located immediately 3' to the 5'-CAP of a mature mRNA typically corresponds to the transcriptional start site. The term "corresponds to" means that the 5'-UTR sequence may be an RNA sequence, such as in the mRNA sequence used for defining the 5'-UTR sequence, or a DNA sequence which corresponds to such RNA sequence. In the context of the present invention, the term "a 5'-UTR of a gene" is the sequence which corresponds to the 5'-UTR of the mature mRNA derived from this gene, i.e. the mRNA obtained by transcription of the gene and maturation of the pre-mature mRNA. The term "5'-UTR of a gene" encompasses the DNA sequence and the RNA sequence (both sense and antisense strand and both mature and immature) of the 5'-UTR.

5'Terminal Oligopyrimidine Tract (TOP): The 5'terminal oligopyrimidine tract (TOP) is typically a stretch of pyrimidine nucleotides located in the 5' terminal region of a nucleic acid molecule, such as the 5' terminal region of certain mRNA molecules or the 5' terminal region of a functional entity, e.g. the transcribed region, of certain genes. The sequence starts with a cytidine, which usually corresponds to the transcriptional start site, and is followed by a stretch of usually about 3 to 30 pyrimidine nucleotides. For example, the TOP may comprise 3, 4, 5, 6, 7, 8, 9, 10, 11, 12, 13, 14, 15, 16, 17, 18, 19, 20, 21, 22, 23, 24, 25, 26, 27, 28, 29, 30 or even more nucleotides. The pyrimidine stretch and thus the 5' TOP ends one nucleotide 5' to the first purine nucleotide located downstream of the TOP. Messenger RNA that contains a 5'terminal oligopyrimidine tract is often referred to as TOP mRNA. Accordingly, genes that provide such messenger RNAs are referred to as TOP genes. TOP sequences have, for example, been found in genes and mRNAs encoding peptide elongation factors and ribosomal proteins.

TOP motif: In the context of the present invention, a TOP motif is a nucleic acid sequence which corresponds to a 5'TOP as defined above. Thus, a TOP motif in the context of the present invention is preferably a stretch of pyrimidine nucleotides having a length of 3-30 nucleotides. Preferably, the TOP-motif consists of at least 3 pyrimidine nucleotides, preferably at least 4 pyrimidine nucleotides, preferably at least 5 pyrimidine nucleotides, more preferably at least 6 nucleotides, more preferably at least 7 nucleotides, most preferably at least 8 pyrimidine nucleotides, wherein the stretch of pyrimidine nucleotides preferably starts at its 5'end with a cytosine nucleotide. In TOP genes and TOP mRNAs, the TOP-motif preferably starts at its 5'end with the transcriptional start site and ends one nucleotide 5' to the first purin residue in said gene or mRNA. A TOP motif in the sense of the present invention is preferably located at the 5'end of a sequence which represents a 5'-UTR or at the 5'end of a sequence which codes for a 5'-UTR. Thus, preferably, a stretch of 3 or more pyrimidine nucleotides is called "TOP motif" in the sense of the present invention if this stretch is located at the 5'end of a respective sequence, such as an artificial nucleic acid molecule (e.g. the optimized nucleic acid molecule of the present invention), the 5'-UTR moiety of said artificial nucleic acid molecule, or the nucleic acid sequence which is derived from the 5'-UTR of a TOP gene as described herein. In other words, a stretch of 3 or more pyrimidine nucleotides, which is not located at the 5'-end of a 5'-UTR or a 5'-UTR moiety but anywhere within a 5'-UTR or a 5'-UTR moiety, is preferably not referred to as "TOP motif".

TOP gene: TOP genes are typically characterised by the presence of a 5' terminal oligopyrimidine tract. Furthermore, most TOP genes are characterized by a growth-associated translational regulation. However, also TOP genes with a tissue specific translational regulation are known. As defined above, the 5'-UTR of a TOP gene corresponds to the sequence of a 5'-UTR of a mature mRNA derived from a TOP gene, which preferably extends from the nucleotide located 3' to the 5'-CAP to the nucleotide located 5' to the start codon. A 5'-UTR of a TOP gene typically does not comprise any start codons, preferably no upstream AUGs (uAUGs) or upstream open reading frames (uORFs). Therein, upstream AUGs and upstream open reading frames are typically understood to be AUGs and open reading frames that occur 5' of the start codon (AUG) of the open reading frame that should be translated. The 5'-UTRs of TOP genes are generally rather short. The lengths of 5'-UTRs of TOP genes may vary between 20 nucleotides up to 500 nucleotides, and are typically less than about 200 nucleotides, preferably less than about 150 nucleotides, more preferably less than about 100 nucleotides. Exemplary 5'-UTRs of TOP genes in the sense of the present invention are the nucleic acid sequences extending from the nucleotide at position 5 to the nucleotide located immediately 5' to the start codon (e.g. the ATG) in the sequences according to SEQ ID NOs. 1-1363 of the patent application WO2013/43700.

In this context a particularly preferred fragment of a 5'-UTR of a TOP gene is a 5'-UTR of a TOP gene lacking the 5'TOP motif. The terms "5'-UTR of a TOP gene" or "5'-TOP UTR" preferably refer to the 5'-UTR of a naturally occurring TOP gene. A preferred example is represented by SEQ ID NO: 1674 (5'-UTR of human ribosomal protein Large 32 lacking the 5' terminal oligopyrimidine tract); corresponding to SEQ ID NO. 1368 of the patent application WO2013/143700).

Wild-type, e.g. wild-type nucleic acid molecule: The term "wild-type" may be understood as a sequence occurring in nature. A wild-type nucleic molecule may typically be understood to be a nucleic acid molecule - e.g. a DNA or an RNA - that occurs naturally. In other words, a wild-type nucleic acid molecule may be understood as a natural nucleic acid molecule. Such nucleic acid molecule may be natural due to its individual sequence (which occurs naturally) and/or due to other modifications, e.g. structural modifications of nucleotides which occur naturally. A wild-type nucleic acid molecule may be a DNA molecule, an RNA molecule or a hybrid-molecule comprising DNA and RNA portions. Herein, the term "wild-type" refers to any sequence as long as it occurs in nature, reflection in publically accessible sequence collections such as GenBank is not required. The National Institute of Health (NIH) provides a publically accessible, annotated collection of publicly available nucleotide sequences ("GenBank", accessible through the NCBI Entrez retrieval system: http://www.ncbi.nlm.nih.gov), (Nucleic Acids Research, 2013; 41(D1):D36-42), including publicly available wild-type sequences. Each GenBank record is assigned a unique constant identifier called an accession number and appears on the ACCESSION line of a GenBank record; and changes to the sequence data are tracked by an integer extension of the accession number which appears on the VERSION line of the GenBank record. Further, the term "wild-type nucleic acid molecule" is not restricted to mean "one single molecule" but is, typically, understood to comprise an ensemble of identical molecules. Accordingly, it may relate to a plurality of identical molecules contained in an aliquot. The optimized nucleic acid molecules of the present invention are preferably not wild-type nucleic acid molecules.

### Detailed description

The present invention concerns optimized nucleic acid molecules, which are the mRNA molecules as defined in the claims, methods for optimization of mRNA molecules and uses of said molecules as defined in the claims, as well as biological entities as defined in the claims comprising optimized nucleic acid molecules as defined in the claims. In general, an optimized nucleic acid molecule is a nucleic acid molecule not found in nature. In other words, it is an artificial nucleic acid molecule, i.e. not a wild-type nucleic acid molecule. In the broadest sense, an optimized nucleic acid molecule is superior to a naturally occurring nucleic acid molecule. Various aspects relating to optimization are subject of the present invention, as detailed herein.

The mRNA molecule of the present invention is distinguished from a wild-type nucleic acid molecule by the features as defined in the claims.

A modular design principle is provided that is suitable to generate an mRNA construct tailored for a respective medical application. Thus, the optimized nucleic acid molecules allow for the versatile combination of multiple moieties or elements.

In general, the present invention relates to an mRNA molecule suitable for application in gene therapy and/or genetic vaccination, the mRNA molecule comprising a 5'-untranslated region (5'-UTR) and a 3'-untranslated region (3'-UTR), wherein the mRNA comprises at least two modules, wherein each module is a ribonucleic acid moiety, wherein at least one module is an open reading frame (ORF) encoding a polypeptide or protein of interest, and wherein at least one module is a further coding nucleic acid moiety encoding at least one additional polypeptide or protein element in the same open reading frame as the polypeptides of interest, wherein the at least one additional polypeptide or protein element is an element that extends protein half-life, and wherein the polypeptide or protein element is characterized by at least 80% identity to SEQ ID NO. 1705, and wherein the open reading frame is or comprises a G/C-modified nucleic acid sequence, wherein the number of G and/or C nucleotides is increased as compared to the wild-type sequence. For reference purposes, the at least two modules can be numbered, e.g. first module, second module... The terms first and second do not imply a specific arrangement; thus, the first module can be located either 5' Upstream) or 3' (downstream) of the second module.

Each module is a ribonucleic acid moiety. Without limiting the technical content, the term "moiety" is used herein to refer to a unit, or building block, or sub-sequence, on nucleic acid level, and the term "element" is used herein to refer to a unit, or building block, or sub-sequence on polypeptide or protein level. When a nucleic acid molecule comprises multiple (two or more) moieties, said moieties are arranged in linear order (5' to 3') and linked to each other by a nucleosidic bond, thereby forming a modular nucleic acid molecule. If a polypeptide or protein molecule comprises multiple (two or more) elements, said elements are arranged in linear order (from N-terminus to C-terminus), and linked to each other by a peptide bond, thereby forming a modular polypeptide or protein. Such modular polypeptide or protein comprises multiple elements in linear order, with respect to the polypeptide strand.

In this entire specification, when the word "comprising" or "comprises" is used with reference to an item or a group of items, this usually means that additional items are optionally present. For example, a protein comprising two specific elements can optionally comprise one or more further elements in addition to the two specific elements. However, in specific more defined embodiments of the present invention, the word "comprising" or "comprises" has a more narrow meaning, i.e. synonymous to "consisting of" or "consists of", thereby excluding the optional presence of additional (non-specified) items.

By the present invention, versatility is provided by modular design on ribonucleic acid level. In particular, for proteins or polypeptides comprised of more than one polypeptide or protein element, the coding region can be designed or tailored on ribonucleic acid level. A tailored or designed ribonucleic acid molecule, e.g. RNA, e.g. mRNA, consists of several moieties, each consisting of a ribonucleic acid sub-sequence. The tailored or designed mRNA molecule comprises (i) at least one ribonucleic acid moiety encoding at least one polypeptide or protein of interest (e.g. a protein potentially producing a therapeutic outcome) and (ii) at least one further ribonucleic acid moiety as defined in the claims. Said further ribonucleic acid moiety may be selected among coding moieties and non-coding moieties. More than one of such moieties can be present in an optimized nucleic acid molecule. In the mRNA molecule as claimed at least two modules are present. Thus, preferably, the mRNA molecule of the present invention is characterized by addition of at least one ribonucleic acid moiety (coding or non-coding), as claimed and described herein. Said addition is preferably realized 5' or 3' with respect to a starting (e.g. wild-type) ribonucleic acid molecule. Said further coding moiety encodes for an element conferring a feature that is beneficial in the context of the polypeptide of interest, e.g. for an envisaged therapeutic application, namely for at least one additional polypeptide or protein element encoded in the same open reading frame as the polypeptide or protein of interest, wherein the at least one additional polypeptide or protein element is an element that extends protein half-life, and wherein the polypeptide or protein element is characterized by at least 80% identity to SEQ ID NO. 1705. Further elements may be selected among a secretory signal peptide (SSP), a multimerization element, a virus like particle (VLP) forming element, a transmembrane element, a dendritic cell targeting element, an immunological adjuvant element, an element promoting antigen presentation; a 2A peptide; a peptide linker element, an element directing post-translational modification (e.g. glycosylation), and/or any other polypeptide or protein. Further non-coding moieties are selected from the group comprising 3'-UTR, 5'-UTR, IRES, miRNA binding site, histone stem loop, poly(A)-sequence and/or any other polynucleotide moiety. The polypeptide or protein element of interest may for example be selected from the group comprising therapeutic proteins, therapeutic polypeptides, allergens, autoimmune antigens, pathogenic antigens, and tumour antigens.

Alternatively or additionally (preferably additionally), the optimized nucleic acid molecule is (also) characterized by the presence of at least one chemical modification, e.g. at least one modified nucleoside. In such case, at least one nucleoside (deoxyribonucleoside or ribonucleoside) is altered. In any event, the chemical modification is a structural feature of such optimized nucleic acid molecule. In the mRNA molecule as claimed, the open reading frame is or comprises a G/C-modified nucleic acid sequence, wherein the number of G and/or C nucleotides is increased as compared to the wild-type sequence

In any event, the at least one distinguishing structural feature provides - or contributes to - a functional property of the optimized nucleic acid molecule which is not exhibited by the non-optimized (wild-type) nucleic acid molecule. Without limitation, such functional properties can be selected from the list comprising improved or increased RNA stability, improved or directed RNA localization, improved or increased RNA lifetime, improved or increased translation of the RNA, improved or increased stability of the encoded polypeptide or protein, tissue- or target cell-specific expression of the encoded polypeptide or protein, improved or target-compartment directed localization of the encoded polypeptide or protein, such as localization at a membrane or in soluble form, in a particular cell organelle, at the cell surface, in excreted form, and the like. Functional properties also include properties associated with multimerization or particle formation of the polypeptide. Further, functional properties may include an added function, such as mediated by a fusion protein, wherein the added function is provided by a second polypeptide element. For such purposes, the ribonucleic acid moiety encoding second polypeptide is fused in frame with respect to the polypeptide of interest. Any two or more such functional properties may be exhibited by an optimized ribonucleic acid molecule of the present invention.

### 1. Type of nucleic acid molecule of the present invention

The optimized nucleic acid molecule according to the present invention is mRNA. Viral RNA or a replicon, DNA, such as a DNA plasmid or viral DNA, or modified RNA (except modified mRNA) or DNA molecules are disclosed for reference. It may be provided as a double-stranded molecule having a sense strand and an anti-sense strand, for example, as a DNA molecule having a sense strand and an anti-sense strand.

In one embodiment, the invention provides an optimized nucleic acid molecule which is a DNA molecule. Such nucleic acid molecule may serve as a template for an RNA molecule, preferably for an mRNA molecule. In other words, the optimized nucleic acid molecule may be a DNA which may be used as a template for production of an RNA e.g. an mRNA or a replicon. An mRNA is preferable. The obtainable RNA, may, in turn, be translated for production of a desired peptide or protein encoded by the open reading frame. If the optimized nucleic acid molecule is a DNA, it may, for example, be used as a double-stranded storage form for continued and repetitive *in vitro* or *in vivo* production of RNA e.g. mRNA.

In all aspects of the present invention, RNA is the preferred nucleic acid molecule. Thus, in an alternative, and preferred, embodiment, the nucleic acid molecule of the invention is an RNA molecule. RNA molecules may be obtainable by transcription from a DNA molecule according to the present invention. Alternatively, RNA molecules may also be obtainable *in vitro* by common methods of chemical synthesis, without being necessarily transcribed from a DNA progenitor.

RNA has numerous advantages over DNA as the nucleic acid for a genetic vehicle, including:
i) The RNA introduced into the cell does not integrate into the genome (whereas DNA does integrate into the genome to a certain degree and can also be inserted into an intact gene of the genome of the host cell, causing a mutation of the respective gene, which can lead to a partial or total loss of the genetic information or to misinformation).
ii) No viral sequences, such as promoters etc., are required for the effective transcription of RNA (whereas a strong promoter (e.g. the viral CMV promoter) is required for the expression of DNA introduced into the cell). The integration of such promoters into the genome of the host cell can lead to undesirable changes in the regulation of gene expression.
iii) The degradation of RNA that has been introduced takes place in a limited period of time, so that it is possible to achieve transient gene expression, which can be discontinued after the required treatment period (whereas this is not possible in the case of DNA that has been integrated into the genome).
iv) RNA does not lead to the induction of pathogenic anti-RNA antibodies in the patient (whereas the induction of anti-DNA antibodies is known to cause an undesirable immune response).
v) RNA is widely applicable; any desired RNA for any desired protein of interest can be prepared in short period of time for therapeutic purposes, even on an individual patient basis (personalized medicine).

As described in detail below, the mRNA molecule comprises a 5'-untranslated region (5'-UTR) and a 3'-untranslated region (3'-UTR) and comprises at least one further coding or non-coding moiety, such as an untranslated region (UTR). Thus, the invention provides an optimized mRNA molecule.

The RNA of the present invention is messenger RNA (mRNA), i.e. RNA encoding at least one polypeptide or protein. An "mRNA species", as used herein, corresponds to a genomic transcription unit.

### 2. Modules (coding and non-coding nucleic acid moieties)

It is a key feature of the present invention that the optimized ribonucleic acid molecule can be designed by combination of more than one ribonucleic acid moieties. Without limiting the technical content, the term "moiety" or "nucleic acid moiety" is used herein to refer to a unit, or building block, on ribonucleic acid level, and the term "element" or "polypeptide element" or "polypeptide or protein element" is used herein to refer to a unit, or building block, on polypeptide or protein level.

The present invention allows to incorporate and recombine desired coding and non-coding moieties into a single ribonucleic acid molecule. At least one such moiety is a coding moiety, i.e. a ribonucleic acid moiety encoding a polypeptide element or protein element. Whether any particular moiety is desired or not depends on the circumstances. For example, if multimerization of an encoded polypeptide is intended, a multimerization element is a desired element of the encoded polypeptide, and thus the ribonucleic acid sequence coding therefor is a desired moiety of an optimized ribonucleic acid encoding such polypeptide capable of multimerization, and so on. Based on the common general knowledge and together with the information provided herein, the skilled person will recognize suitability of individual ribonucleic acid moieties, e.g. encoding polypeptide or protein elements, for the purposes of any particular optimized nucleic acid molecule in the context of the present invention.

Suitable moieties can generally be selected from coding moieties and from non-coding moieties. However, as the purpose of a nucleic acid molecule in the context of the present invention is typically the provision of genetic information encoding a polypeptide or protein, at least one coding moiety is typically comprised.

In accordance with the modular nature, the polynucleotide of the present invention is preferably artificial or chimeric. A chimeric molecule, e.g. polynucleotide, comprises typically sequence information originating from more than one protein and/or from more than one species.

Methods of generating ribonucleic acid molecules comprising several moieties are known to the person skilled in the art and include, without limitation, *in vitro* synthesis and molecular biological approaches, e.g. enzymatic linkage of nucleic acid fragments by the help of ligase enzymes.

### 2.1 List of elements encoded by the ribonucleic acid moieties of the present invention

At least one module is a further coding ribonucleic acid moiety encoding at least one additional polypeptide or protein element encoded in the same open reading frame as the polypeptide or protein of interest, wherein the at least one additional polypeptide or protein element is an element that extends protein half-life, and wherein the polypeptide or protein element is characterized by at least 80% identity to SEQ **ID** NO. 1705. Further coding moieties can be selected from ribonucleic acid sequences encoding one or more polypeptides from the following list:
- a ribonucleic acid sequence encoding a polypeptide or protein of interest;
- a ribonucleic acid sequence encoding a secretory signal peptide (SSP);
- a ribonucleic acid sequence encoding a multimerization element including dimerization, trimerization, tetramerization or oligomerization elements;
- a ribonucleic acid sequence encoding a virus like particle (VLP) forming element;
- a ribonucleic acid sequence encoding a transmembrane element;
- a ribonucleic acid sequence encoding a dendritic cell targeting element;
- a ribonucleic acid sequence encoding an immunological adjuvant element;
- a ribonucleic acid sequence encoding an element promoting antigen presentation;
- a ribonucleic acid sequence encoding a 2A peptide;
- a ribonucleic acid sequence encoding a peptide linker element;
- an elements that extends protein half-life;
- a ribonucleic acid sequence encoding an element for post-translational modification (e.g. glycosylation)
   and/or
- any other ribonucleic acid sequence encoding a polypeptide or protein.

While the above list provides items in the singular form, it is equally possible that more than one respective moiety is selected. Moieties not included in the above list may equally be selected. Preferably, at least one moiety is from the above list.

The above are generic terms for the polypeptide/protein elements encoded by the respective ribonucleic acid moieties. Specific elements falling under these generic terms are also provided by the present invention. Details of elements from the above list, including sequences pertaining to specific embodiments, are provided below.

Herein, "a polypeptide or protein of interest" is any polypeptide or protein that is of interest for the desired purpose. Alternatively, a polypeptide or protein of interest can be referred to herein as target protein/polypeptide For example, when the purpose is vaccination against a certain antigen, a polypeptide or protein of interest is a polypeptide or protein which possesses the respective antigenic determinant. The ribonucleic acid molecule of the present invention comprises at least one moiety encoding a polypeptide or protein of interest, and additionally one or more further moiety encoding a further elementas defined in the claims. The ribonucleic acid molecule encoding at least one additional polypeptide or protein element, is encoded in the same reading frame as the polypeptide or protein of interest. Proteins or polypeptides encoded by that type of ribonucleic acids are also referred to as fusion proteins. Thus, preferably, the ribonucleic acid molecule of the present invention comprises a fusion protein. A fusion protein can comprise two or more, three or more, four or more, five or more, six or more, seven or more, eight or more, nine or more, ten or more polypeptide elements or protein elements. The mRNA molecule as claimed comprises at least two modules, wherein each module is a ribonucleic acid moiety and at least one module encodes at least one additional polypeptide or protein element in the same open reading frame as the polypeptides of interest, wherein the at least one additional polypeptide or protein element is an element that extends protein half-life, and wherein the polypeptide or protein element is characterized by at least 80% identity to SEQ ID NO. 1705.

### 2.2 List of non-coding moieties of the optimized ribonucleic acid molecule

Non-coding moieties can be selected from nucleic acid sequences from one or more disclosed the following list:
- a 5'-UTR;
- a 3'-UTR;
- a miRNA moiety;
- a Cap;
- a poly(C) sequence
- a histone stem-loop sequence
- a poly(A) sequence or a polyadenylation signal;
- an IRES moiety
- a hairpin moiety
- moieties for RNA binding proteins
- a moiety that prevents 3'-5' degradation
- moieties that regulate RNA decay rates

The above are generic terms. Specific moieties falling under these generic terms are also provided by the present invention. Details of moieties from the above list, including sequences pertaining to specific embodiments, are provided below.

While the above list provides items in the singular form, it is equally possible that more than one respective moiety is selected. Ribonucleic acid moieties not included in the above list may equally be selected. Preferably, at least one module or moiety is from the above list.

The claimed mRNA molecule comprises a 5'-untranslated region (5'-UTR) and a 3'-untranslated region (3'-UTR).

### 2.3 Number of coding moieties and number of non-coding moieties

More than two coding moietyiescan be comprised in the optimized ribonucleic acid molecule, such as 3 coding moieties, 4 coding moieties, 5 coding moieties, 6 coding moieties, 7 coding moieties, 8 coding moieties, 9 coding moieties, 10 coding moieties or more than 10 coding moieties. It is also possible that 2 to 10 coding moieties, 3 to 9 coding moieties, four to eight coding moieties, five to seven coding moieties are comprised. Since the coding moieties encode the respective polypeptide elements or protein elements in the same open reading frame, translation of said open reading frame will result in the expression of a fusion protein. Such a fusion protein comprises the respective number of polypeptide or protein elements.

More than one non-coding moiety can be comprised, such as 2 non-coding moieties, 3 non-coding moieties, 4 non-coding moieties, 5 non-coding moieties, 6 non-coding moieties, 7 non-coding moieties, 8 non-coding moieties, 9 non-coding moieties, 10 non-coding moieties or more than 10 non-coding moieties. It is also possible that two to ten non-coding moieties, three to nine non-coding moieties, four to eight non-coding moieties, five to seven non-coding moieties are comprised. The claimed mRNA molecule comprises a 3'-UTR moiety and a 5'-UTR moiety.

### 2.4 Combination of coding moieties and non-coding moieties

In light with the disclosure herein, any combination of moieties (coding moieties and non-coding moieties) is possible in addition to the combination of moieties defined in the claims. Guided by the disclosure herein, the skilled person can routinely select appropriate moieties.

Thereby, the present invention allows for the versatile combination and recombination of nucleic acid moieties, and thus of polypeptide/protein elements. Thereby, a nucleic acid which is fit for any given purpose, can be designed and prepared. In other words, an optimal nucleic acid for any given purpose can be designed and prepared. Since such optimal nucleic acid, or optimized nucleic acid, is provided by the present invention, the present invention concerns not only such nucleic acid as such, but also methods for their preparation and apparatuses for their preparation.

The present invention thus allows for versatile recombination of nucleic acid moieties for any given purpose. Versatility is achieved by combination of moieties (coding moieties and non-coding moieties) as disclosed herein.

### 3. Details and embodiments of moieties of the nucleic acid molecules of the invention

Details and embodiments of these moieties are provided herein below.

The following provides specific sequences as illustrative examples. However, further sequences having same or similar or equivalent functions to the specific sequences used herein may be used as well. An equivalent function is a function with the same effect or function (qualitatively); the effect need not be quantitatively identical. For example any two secretory signal peptides are designated as "equivalent", as long as each of them provides the function described herein for secretory signal peptides, even if - as the case may be - not at a quantitatively identical level.

Insofar as species origin is given for polypeptide or polynucleotide sequences in connection with specific sequences of elements or moieties below, this serves for purposes of information rather than limiting the invention to a particular purpose. For example, when a specific sequence is from mouse (*Mus musculus*), it can be used for all aspects of the present invention, including nucleic acids for therapy of humans. In other words, indication of the species origin is not limiting.

### 3.1 Coding modules

### 3.1.1 Polypeptide or protein of interest

The polypeptide or protein of interest is not limited. Rather, in line with the general concept of the present invention, virtually any polypeptide or protein, or nucleic acid encoding such polypeptide or protein, can be used or employed. Non-limiting examples include proteins of human origin, proteins of animal origin, proteins of plant origin, proteins of protozoological origin, proteins of virus origin, proteins of bacterial or archaebacterial origin, chimeric proteins, artificial proteins. The polypeptide or protein is encoded by an open reading frame (ORF).

In some embodiments, the ORF does not encode a ribosomal protein of human or plant origin, in particular *Arabidopsis* origin, in particular does not encode human ribosomal protein S6 (RPS6), human ribosomal protein L36a-like (RPL36AL) or *Arabidopsis* ribosomal protein S16 (RPS16). In a further preferred embodiment, the open reading frame (ORF) does not encode ribosomal protein S6 (RPS6), ribosomal protein L36a-like (RPL36AL) or ribosomal protein S16 (RPS16) of whatever origin. In some embodiments, the open reading frame of the optimized nucleic acid molecule according to the present invention does not code for a reporter protein, e.g., a reporter protein selected from the group consisting of globin proteins (particularly beta-globin), luciferase protein, GFP proteins, glucuronidase proteins (particularly beta- glucuronidase) or variants thereof, for example, variants exhibiting at least 70% sequence identity to a globin protein, a luciferase protein, a GFP protein, or a glucuronidase protein.

In a preferred embodiment, the at least one open reading frame encodes a therapeutic protein or peptide. In another embodiment, an antigen is encoded by the at least one open reading frame, such as a pathogenic antigen, a tumour antigen, an allergenic antigen or an autoimmune antigen. In an alternative embodiment, an antibody or an antigen-specific T cell receptor or a fragment thereof is encoded by the at least one open reading frame of the optimized nucleic acid molecule according to the invention.

Specific examples of suitable polypeptides and proteins of interest include pathogenic antigens, tumour antigens, and therapeutic proteins. Such examples are described below.

### 3.1.1.1 Therapeutic proteins; therapeutic polypeptides

The protein or polypeptide may comprise or consist of a therapeutic protein, a fragment, variant or derivative of a protein or a peptide, which comprises a therapeutic protein or a fragment, variant or derivative thereof.

Therapeutic proteins as defined herein are peptides or proteins, which are beneficial for the treatment of any inherited or acquired disease or which improves the condition of an individual. Particularly, therapeutic proteins play an important role in the creation of therapeutic agents that could modify and repair genetic errors, destroy cancer cells or pathogen infected cells, treat immune system disorders, treat metabolic or endocrine disorders, among other functions. For instance, Erythropoietin (EPO), a protein hormone can be utilized in treating patients with erythrocyte deficiency, which is a common cause of kidney complications. Furthermore adjuvant proteins, therapeutic antibodies are encompassed by therapeutic proteins and also hormone replacement therapy which is e.g. used in the therapy of women in menopause. In more recent approaches, somatic cells of a patient are used to reprogram them into pluripotent stem cells, which replace the disputed stem cell therapy. Also these proteins used for reprogramming of somatic cells or used for differentiating of stem cells are defined herein as therapeutic proteins. Furthermore, therapeutic proteins may be used for other purposes, e.g. wound healing, tissue regeneration, angiogenesis, etc. Furthermore, antigen-specific B cell receptors and fragments and variants thereof are defined herein as therapeutic proteins.

Therefore therapeutic proteins can be used for various purposes including treatment of various diseases like e.g. infectious diseases, neoplasms (e.g. cancer or tumour diseases), diseases of the blood and blood-forming organs, endocrine, nutritional and metabolic diseases, diseases of the nervous system, diseases of the circulatory system, diseases of the respiratory system, diseases of the digestive system, diseases of the skin and subcutaneous tissue, diseases of the musculoskeletal system and connective tissue, and diseases of the genitourinary system, independently if they are inherited or acquired.

In this context, particularly preferred therapeutic proteins which can be used inter alia in the treatment of metabolic or endocrine disorders are selected from (in brackets the particular disease for which the therapeutic protein is used in the treatment): Acid sphingomyelinase (Niemann-Pick disease), Adipotide (obesity), Agalsidase-beta (human galactosidase A) (Fabry disease; prevents accumulation of lipids that could lead to renal and cardiovascular complications), Alglucosidase (Pompe disease (glycogen storage disease type II)), alpha-galactosidase A (alpha-GAL A, Agalsidase alpha) (Fabry disease), alpha-glucosidase (Glycogen storage disease (GSD), Morbus Pompe), alpha-L-iduronidase (mucopolysaccharidoses (MPS), Hurler syndrome, Scheie syndrome), alpha-N-acetylglucosaminidase (Sanfilippo syndrome), Amphiregulin (cancer, metabolic disorder), Angiopoietin ((Ang1, Ang2, Ang3, Ang4, ANGPTL2, ANGPTL3, ANGPTL4, ANGPTL5, ANGPTL6, ANGPTL7) (angiogenesis, stabilize vessels), Betacellulin (metabolic disorder), Beta-glucuronidase (Sly syndrome), Bone morphogenetic protein BMPs (BMP1, BMP2, BMP3, BMP4, BMP5, BMP6, BMP7, BMP8a, BMP8b, BMP10, BMP15) (regenerative effect, bone-related conditions, chronic kidney disease (CKD)), CLN6 protein (CLN6 disease - Atypical Late Infantile, Late Onset variant, Early Juvenile, Neuronal Ceroid Lipofuscinoses (NCL)), Epidermal growth factor (EGF) (wound healing, regulation of cell growth, proliferation, and differentiation), Epigen (metabolic disorder), Epiregulin (metabolic disorder), Fibroblast Growth Factor (FGF, FGF-1, FGF-2, FGF-3, FGF-4, FGF-5, FGF-6, FGF-7, FGF-8, FGF-9, FGF-10, FGF-11, FGF-12, FGF-13, FGF-14, FGF-16, FGF-17, FGF-17, FGF-18, FGF-19, FGF-20, FGF-21, FGF-22, FGF-23) (wound healing, angiogenesis, endocrine disorders, tissue regeneration), Galsulphase (Mucopolysaccharidosis VI), Ghrelin (irritable bowel syndrome (IBS), obesity, Prader-Willi syndrome, type II diabetes mellitus), Glucocerebrosidase (Gaucher's disease), GM-CSF (regenerative effect, production of white blood cells, cancer), Heparin-binding EGF-like growth factor (HB-EGF) (wound healing, cardiac hypertrophy and heart development and function), Hepatocyte growth factor HGF (regenerative effect, wound healing), Hepcidin (iron metabolism disorders, Beta-thalassemia), Human albumin (Decreased production of albumin (hypoproteinaemia), increased loss of albumin (nephrotic syndrome), hypovolaemia, hyperbilirubinaemia), Idursulphase (lduronate-2-sulphatase) (Mucopolysaccharidosis II (Hunter syndrome)), Integrins αVβ3, αVβ5 and α5β1 (Bind matrix macromolecules and proteinases, angiogenesis), luduronate sulfatase (Hunter syndrome), Laronidase (Hurler and Hurler-Scheie forms of mucopolysaccharidosis I), N-acetylgalactosamine-4-sulfatase (rhASB; galsulfase, Arylsulfatase A (ARSA), Arylsulfatase B (ARSB)) (arylsulfatase B deficiency, Maroteaux-Lamy syndrome, mucopolysaccharidosis VI), N-acetylglucosamine-6-sulfatase (Sanfilippo syndrome), Nerve growth factor (NGF), Brain-Derived Neurotrophic Factor (BDNF), Neurotrophin-3 (NT-3), and Neurotrophin 4/5 (NT-4/5) (regenerative effect, cardiovascular diseases, coronary atherosclerosis, obesity, type 2 diabetes, metabolic syndrome, acute coronary syndromes, dementia, depression, schizophrenia, autism, Rett syndrome, anorexia nervosa, bulimia nervosa, wound healing, skin ulcers, corneal ulcers, Alzheimer's disease), Neuregulin (NRG1, NRG2, NRG3, NRG4) (metabolic disorder, schizophrenia), Neuropilin (NRP-1, NRP-2) (angiogenesis, axon guidance, cell survival, migration), Obestatin (irritable bowel syndrome (IBS), obesity, Prader-Willi syndrome, type II diabetes mellitus), Platelet Derived Growth factor (PDGF (PDFF-A, PDGF-B, PDGF-C, PDGF-D) (regenerative effect, wound healing, disorder in angiogenesis, Arteriosclerosis, Fibrosis, cancer), TGF beta receptors (endoglin, TGF-beta 1 receptor, TGF-beta 2 receptor, TGF-beta 3 receptor) (renal fibrosis, kidney disease, diabetes, ultimately end-stage renal disease (ESRD), angiogenesis), Thrombopoietin (THPO) (Megakaryocyte growth and development factor (MGDF)) (platelets disorders, platelets for donation, recovery of platelet counts after myelosuppressive chemotherapy), Transforming Growth factor (TGF (TGF-alpha, TGF-beta (TGFbeta1, TGFbeta2, and TGFbeta3))) (regenerative effect, wound healing, immunity, cancer, heart disease, diabetes, Marfan syndrome, Loeys-Dietz syndrome), VEGF (VEGF-A, VEGF-B, VEGF-C, VEGF-D, VEGF-E, VEGF-F and PIGF) (regenerative effect, angiogenesis, wound healing, cancer, permeability), Nesiritide (Acute decompensated congestive heart failure), Trypsin (Decubitus ulcer, varicose ulcer, debridement of eschar, dehiscent wound, sunburn, meconium ileus), adrenocorticotrophic hormone (ACTH) ("Addison's disease, Small cell carcinoma, Adrenoleukodystrophy, Congenital adrenal hyperplasia, Cushing's syndrome, Nelson's syndrome, Infantile spasms), Atrial-natriuretic peptide (ANP) (endocrine disorders), Cholecystokinin (diverse), Gastrin (hypogastrinemia), Leptin (Diabetes, hypertriglyceridemia, obesity), Oxytocin (stimulate breastfeeding, non-progression of parturition), Somatostatin (symptomatic treatment of carcinoid syndrome, acute variceal bleeding, and acromegaly, polycystic diseases of the liver and kidney, acromegaly and symptoms caused by neuroendocrine tumours), Vasopressin (antidiuretic hormone) (diabetes insipidus), Calcitonin (Postmenopausal osteoporosis, Hypercalcaemia, Paget's disease, Bone metastases, Phantom limb pain, Spinal Stenosis), Exenatide (Type 2 diabetes resistant to treatment with metformin and a sulphonylurea), Growth hormone (GH), somatotropin (Growth failure due to GH deficiency or chronic renal insufficiency, Prader-Willi syndrome, Turner syndrome, AIDS wasting or cachexia with antiviral therapy), Insulin (Diabetes mellitus, diabetic ketoacidosis, hyperkalaemia), Insulin-like growth factor 1 IGF-1 (Growth failure in children with GH gene deletion or severe primary IGF1 deficiency, neurodegenerative disease, cardiovascular diseases, heart failure), Mecasermin rinfabate, IGF-1 analog (Growth failure in children with GH gene deletion or severe primary IGF1 deficiency, neurodegenerative disease, cardiovascular diseases, heart failure), Mecasermin, IGF-1 analog (Growth failure in children with GH gene deletion or severe primary IGF1 deficiency, neurodegenerative disease, cardiovascular diseases, heart failure), Pegvisomant (Acromegaly), Pramlintide (Diabetes mellitus, in combination with insulin), Teriparatide (human parathyroid hormone residues 1-34) (Severe osteoporosis), Becaplermin (Debridement adjunct for diabetic ulcers), Dibotermin-alpha (Bone morphogenetic protein 2) (Spinal fusion surgery, bone injury repair), Histrelin acetate (gonadotropin releasing hormone; GnRH) (Precocious puberty), Octreotide (Acromegaly, symptomatic relief of VIP-secreting adenoma and metastatic carcinoid tumours), and Palifermin (keratinocyte growth factor; KGF) (Severe oral mucositis in patients undergoing chemotherapy, wound healing).

These and other proteins are understood to be therapeutic, as they are meant to treat the subject by replacing its defective endogenous production of a functional protein in sufficient amounts. Accordingly, such therapeutic proteins are typically mammalian, in particular human proteins.

For the treatment of blood disorders, diseases of the circulatory system, diseases of the respiratory system, cancer or tumour diseases, infectious diseases or immunedeficiencies following therapeutic proteins may be used: Alteplase (tissue plasminogen activator; tPA) (Pulmonary embolism, myocardial infarction, acute ischaemic stroke, occlusion of central venous access devices), Anistreplase (Thrombolysis), Antithrombin III (AT-III) (Hereditary AT-III deficiency, Thromboembolism), Bivalirudin (Reduce blood-clotting risk in coronary angioplasty and heparin-induced thrombocytopaenia), Darbepoetin-alpha (Treatment of anaemia in patients with chronic renal insufficiency and chronic renal failure (+/- dialysis)), Drotrecogin-alpha (activated protein C) (Severe sepsis with a high risk of death), Erythropoietin, Epoetin-alpha, erythropoetin, erthropoyetin (Anaemia of chronic disease, myleodysplasia, anaemia due to renal failure or chemotherapy, preoperative preparation), Factor IX (Haemophilia B), Factor VIIa (Haemorrhage in patients with haemophilia A or B and inhibitors to factor VIII or factor IX), Factor VIII (Haemophilia A), Lepirudin (Heparin-induced thrombocytopaenia), Protein C concentrate (Venous thrombosis, Purpura fulminans), Reteplase (deletion mutein of tPA) (Management of acute myocardial infarction, improvement of ventricular function), Streptokinase (Acute evolving transmural myocardial infarction, pulmonary embolism, deep vein thrombosis, arterial thrombosis or embolism, occlusion of arteriovenous cannula), Tenecteplase (Acute myocardial infarction), Urokinase (Pulmonary embolism), Angiostatin (Cancer), Anti-CD22 immunotoxin (Relapsed CD33+ acute myeloid leukaemia), Denileukin diftitox (Cutaneous T-cell lymphoma (CTCL)), Immunocyanin (bladder and prostate cancer), MPS (Metallopanstimulin) (Cancer), Aflibercept (Non-small cell lung cancer (NSCLC), metastatic colorectal cancer (mCRC), hormone-refractory metastatic prostate cancer, wet macular degeneration), Endostatin (Cancer, inflammatory diseases like rheumatoid arthritis as well as Crohn's disease, diabetic retinopathy, psoriasis, and endometriosis), Collagenase (Debridement of chronic dermal ulcers and severely burned areas, Dupuytren's contracture, Peyronie's disease), Human deoxy-ribonuclease I, dornase (Cystic fibrosis; decreases respiratory tract infections in selected patients with FVC greater than 40% of predicted), Hyaluronidase (Used as an adjuvant to increase the absorption and dispersion of injected drugs, particularly anaesthetics in ophthalmic surgery and certain imaging agents), Papain (Debridement of necrotic tissue or liquefication of slough in acute and chronic lesions, such as pressure ulcers, varicose and diabetic ulcers, burns, postoperative wounds, pilonidal cyst wounds, carbuncles, and other wounds), L-Asparaginase (Acute lymphocytic leukaemia, which requires exogenous asparagine for proliferation), Peg-asparaginase (Acute lymphocytic leukaemia, which requires exogenous asparagine for proliferation), Rasburicase (Paediatric patients with leukaemia, lymphoma, and solid tumours who are undergoing anticancer therapy that may cause tumour lysis syndrome), Human chorionic gonadotropin (HCG) (Assisted reproduction), Human follicle-stimulating hormone (FSH) (Assisted reproduction), Lutropin-alpha (Infertility with luteinizing hormone deficiency), Prolactin (Hypoprolactinemia, serum prolactin deficiency, ovarian dysfunction in women, anxiety, arteriogenic erectile dysfunction, premature ejaculation, oligozoospermia, asthenospermia, hypofunction of seminal vesicles, hypoandrogenism in men), alpha-1-Proteinase inhibitor (Congenital antitrypsin deficiency), Lactase (Gas, bloating, cramps and diarrhoea due to inability to digest lactose), Pancreatic enzymes (lipase, amylase, protease) (Cystic fibrosis, chronic pancreatitis, pancreatic insufficiency, post-Billroth II gastric bypass surgery, pancreatic duct obstruction, steatorrhoea, poor digestion, gas, bloating), Adenosine deaminase (pegademase bovine, PEG-ADA) (Severe combined immunodeficiency disease due to adenosine deaminase deficiency), Abatacept (Rheumatoid arthritis (especially when refractory to TNFalpha inhibition)), Alefacept (Plaque Psoriasis), Anakinra (Rheumatoid arthritis), Etanercept (Rheumatoid arthritis, polyarticular-course juvenile rheumatoid arthritis, psoriatic arthritis, ankylosing spondylitis, plaque psoriasis, ankylosing spondylitis), Interleukin-1 (IL-1) receptor antagonist, Anakinra (inflammation and cartilage degradation associated with rheumatoid arthritis), Thymulin (neurodegenerative diseases, rheumatism, anorexia nervosa), TNF-alpha antagonist (autoimmune disorders such as rheumatoid arthritis, ankylosing spondylitis, Crohn's disease, psoriasis, hidradenitis suppurativa, refractory asthma), Enfuvirtide (HIV-1 infection), and Thymosin α1 (Hepatitis B and C). (In brackets is the particular disease for which a use of the therapeutic protein is indicated for treatment)

### 3.1.1.2 Pathogenic antigens:

The protein or a polypeptide of interest may consist or comprise of a pathogenic antigen or a fragment, variant or derivative thereof. Such pathogenic antigens are derived from pathogenic organisms, in particular bacterial, viral or protozoological pathogenic organisms, which evoke an immunological reaction in a subject, in particular a mammalian subject, more particularly a human. More specifically, pathogenic antigens are preferably surface antigens, e.g. proteins (or fragments of proteins, e.g. the exterior portion of a surface antigen) located at the surface of the virus or the bacterial or protozoological organism.

Pathogenic antigens are peptide or protein antigens preferably derived from a pathogen associated with infectious disease which are preferably selected from antigens derived from the pathogens Acinetobacter baumannii, Anaplasma genus, Anaplasma phagocytophilum, Ancylostoma braziliense, Ancylostoma duodenale, Arcanobacterium haemolyticum, Ascaris lumbricoides, Aspergillus genus, Astroviridae, Babesia genus, Bacillus anthracis, Bacillus cereus, Bartonella henselae, BK virus, Blastocystis hominis, Blastomyces dermatitidis, Bordetella pertussis, Borrelia burgdorferi, Borrelia genus, Borrelia spp, Brucella genus, Brugia malayi, Bunyaviridae family, Burkholderia cepacia and other Burkholderia species, Burkholderia mallei, Burkholderia pseudomallei, Caliciviridae family, Campylobacter genus, Candida albicans, Candida spp, Chlamydia trachomatis, Chlamydophila pneumoniae, Chlamydophila psittaci, CJD prion, Clonorchis sinensis, Clostridium botulinum, Clostridium difficile, Clostridium perfringens, Clostridium perfringens, Clostridium spp, Clostridium tetani, Coccidioides spp, coronaviruses, Corynebacterium diphtheriae, Coxiella burnetii, Crimean-Congo haemorrhagic fever virus, Cryptococcus neoformans, Cryptosporidium genus, Cytomegalovirus (CMV), Dengue viruses (DEN-1, DEN-2, DEN-3 and DEN-4), Dientamoeba fragilis, Ebolavirus (EBOV), Echinococcus genus, Ehrlichia chaffeensis, Ehrlichia ewingii, Ehrlichia genus, Entamoeba histolytica, Enterococcus genus, Enterovirus genus, Enteroviruses, mainly Coxsackie A virus and Enterovirus 71 (EV71), Epidermophyton spp, Epstein-Barr Virus (EBV), Escherichia coli O157:H7, O111 and O104:H4, Fasciola hepatica and Fasciola gigantica, FFI prion, Filarioidea superfamily, Flaviviruses, Francisella tularensis, Fusobacterium genus, Geotrichum candidum, Giardia intestinalis, Gnathostoma spp, GSS prion, Guanarito virus, Haemophilus ducreyi, Haemophilus influenzae, Helicobacter pylori, Henipavirus (Hendra virus Nipah virus), Hepatitis A Virus, Hepatitis B Virus (HBV), Hepatitis C Virus (HCV), Hepatitis D Virus, Hepatitis E Virus, Herpes simplex virus 1 and 2 (HSV-1 and HSV-2), Histoplasma capsulatum, HIV (Human immunodeficiency virus), Hortaea werneckii, Human bocavirus (HBoV), Human herpesvirus 6 (HHV-6) and Human herpesvirus 7 (HHV-7), Human metapneumovirus (hMPV), Human papillomavirus (HPV), Human parainfluenza viruses (HPIV), Japanese encephalitis virus, JC virus, Junin virus, Kingella kingae, Klebsiella granulomatis, Kuru prion, Lassa virus, Legionella pneumophila, Leishmania genus, Leptospira genus, Listeria monocytogenes, Lymphocytic choriomeningitis virus (LCMV), Machupo virus, Malassezia spp, Marburg virus, Measles virus, Metagonimus yokagawai, Microsporidia phylum, Molluscum contagiosum virus (MCV), Mumps virus, Mycobacterium leprae and Mycobacterium lepromatosis, Mycobacterium tuberculosis, Mycobacterium ulcerans, Mycoplasma pneumoniae, Naegleria fowleri, Necator americanus, Neisseria gonorrhoeae, Neisseria meningitidis, Nocardia asteroides, Nocardia spp, Onchocerca volvulus, Orientia tsutsugamushi, Orthomyxoviridae family (Influenza), Paracoccidioides brasiliensis, Paragonimus spp, Paragonimus westermani, Parvovirus B19, Pasteurella genus, Plasmodium genus, Pneumocystis jirovecii, Poliovirus, Rabies virus, Respiratory syncytial virus (RSV), Rhinovirus, rhinoviruses, Rickettsia akari, Rickettsia genus, Rickettsia prowazekii, Rickettsia rickettsii, Rickettsia typhi, Rift Valley fever virus, Rotavirus, Rubella virus, Sabia virus, Salmonella genus, Sarcoptes scabiei, SARS coronavirus, Schistosoma genus, Shigella genus, Sin Nombre virus, Hantavirus, Sporothrix schenckii, Staphylococcus genus, Staphylococcus genus, Streptococcus agalactiae, Streptococcus pneumoniae, Streptococcus pyogenes, Strongyloides stercoralis, Taenia genus, Taenia solium, Tick-borne encephalitis virus (TBEV), Toxocara canis or Toxocara cati, Toxoplasma gondii, Treponema pallidum, Trichinella spiralis, Trichomonas vaginalis, Trichophyton spp, Trichuris trichiura, Trypanosoma brucei, Trypanosoma cruzi, Ureaplasma urealyticum, Varicella zoster virus (VZV), Varicella zoster virus (VZV), Variola major or Variola minor, vCJD prion, Venezuelan equine encephalitis virus, Vibrio cholerae, West Nile virus, Western equine encephalitis virus, Wuchereria bancrofti, Yellow fever virus, Yersinia enterocolitica, Yersinia pestis, and Yersinia pseudotuberculosis.

In this context particularly preferred are antigens from the pathogens selected from Influenza virus, respiratory syncytial virus (RSV), Herpes simplex virus (HSV), human Papilloma virus (HPV), Human immunodeficiency virus (HIV), Plasmodium, Staphylococcus aureus, Dengue virus, Chlamydia trachomatis, Cytomegalovirus (CMV), Hepatitis B virus (HBV), Mycobacterium tuberculosis, Rabies virus, and Yellow Fever Virus.

### 3.1.1.3 Tumour antigens:

The protein or polypeptide may comprise or consist of a tumour antigen, a fragment, variant or derivative of a tumour antigen. Such nucleic acid molecules are particularly useful for therapeutic purposes, particularly genetic vaccination. Preferably, the tumour antigen is selected from the group comprising a melanocyte-specific antigen, a cancer-testis antigen or a tumour-specific antigen, preferably a CT-X antigen, a non-X CT-antigen, a binding partner for a CT-X antigen or a binding partner for a non-X CT-antigen or a tumour-specific antigen, more preferably a CT-X antigen, a binding partner for a non-X CT-antigen or a tumour-specific antigen or a fragment, variant or derivative of said tumour antigen; and wherein each of the nucleic acid sequences encodes a different peptide or protein; and wherein at least one of the nucleic acid sequences encodes for 5T4, 707-AP, 9D7, AFP, AlbZIP HPG1, alpha-5-beta-1-integrin, alpha-5-beta-6-integrin, alpha-actinin-4/m, alpha-methylacyl-coenzyme A racemase, ART-4, ARTC1/m, B7H4, BAGE-1, BCL-2, bcr/abl, beta-catenin/m, BING-4, BRCA1/m, BRCA2/m, CA 15-3/CA 27-29, CA 19-9, CA72-4, CA125, calreticulin, CAMEL, CASP-8/m, cathepsin B, cathepsin L, CD19, CD20, CD22, CD25, CDE30, CD33, CD4, CD52, CD55, CD56, CD80, CDC27/m, CDK4/m, CDKN2A/m, CEA, CLCA2, CML28, CML66, COA-1/m, coactosin-like protein, collage XXIII, COX-2, CT-9/BRD6, Cten, cyclin B1, cyclin D1, cyp-B, CYPB1, DAM-10, DAM-6, DEK-CAN, EFTUD2/m, EGFR, ELF2/m, EMMPRIN, EpCam, EphA2, EphA3, ErbB3, ETV6-AML1, EZH2, FGF-5, FN, Frau-1, G250, GAGE-1, GAGE-2, GAGE-3, GAGE-4, GAGE-5, GAGE-6, GAGE7b, GAGE-8, GDEP, GnT-V, gp100, GPC3, GPNMB/m, HAGE, HAST-2, hepsin, Her2/neu, HERV-K-MEL, HLA-A*0201-R17I, HLA-A11/m, HLA-A2/m, HNE, homeobox NKX3.1, HOM-TES-14/SCP-1, HOM-TES-85, HPV-E6, HPV-E7, HSP70-2M, HST-2, hTERT, iCE, IGF-1R, IL-13Ra2, IL-2R, IL-5, immature laminin receptor, kallikrein-2, kallikrein-4, Ki67, KIAA0205, KIAA0205/m, KK-LC-1, K-Ras/m, LAGE-A1, LDLR-FUT, MAGE-A1, MAGE-A2, MAGE-A3, MAGE-A4, MAGE-A6, MAGE-A9, MAGE-A10, MAGE-A12, MAGE-B1, MAGE-B2, MAGE-B3, MAGE-B4, MAGE-B5, MAGE-B6, MAGE-B10, MAGE-B16, MAGE-B17, MAGE-C1, MAGE-C2, MAGE-C3, MAGE-D1, MAGE-D2, MAGE-D4, MAGE-E1, MAGE-E2, MAGE-F1, MAGE-H1, MAGEL2, mammaglobin A, MART-1/melan-A, MART-2, MART-2/m, matrix protein 22, MC1R, M-CSF, ME1/m, mesothelin, MG50/PXDN, MMP11, MN/CA IX-antigen, MRP-3, MUC-1, MUC-2, MUM-1/m, MUM-2/m, MUM-31m, myosin class I/m, NA88-A, N-acetylglucosaminyltransferase-V, Neo-PAP, Neo-PAP/m, NFYC/m, NGEP, NMP22, NPM/ALK, N-Ras/m, NSE, NY-ESO-1, NY-ESO-B, OA1, OFA-iLRP, OGT, OGT/m, OS-9, OS-9/m, osteocalcin, osteopontin, p15, p190 minor bcr-abl, p53, p53/m, PAGE-4, PAI-1, PAI-2, PAP, PART-1, PATE, PDEF, Pim-1-Kinase, Pin-1, Pml/PARalpha, POTE, PRAME, PRDX5/m, prostein, proteinase-3, PSA, PSCA, PSGR, PSM, PSMA, PTPRK/m, RAGE-1, RBAF600/m, RHAMM/CD168, RU1, RU2, S-100, SAGE, SART-1, SART-2, SART-3, SCC, SIRT2/m, Sp17, SSX-1, SSX-2/HOM-MEL-40, SSX-4, STAMP-1, STEAP-1, survivin, survivin-2B, SYT-SSX-1, SYT-SSX-2, TA-90, TAG-72, TARP, TEL-AML1, TGFbeta, TGFbetaRII, TGM-4, TPI/m, TRAG-3, TRG, TRP-1, TRP-2/6b, TRP/INT2, TRP-p8, tyrosinase, UPA, VEGFR1, VEGFR-2/FLK-1, WT1 and a immunoglobulin idiotype of a lymphoid blood cell or a T cell receptor idiotype of a lymphoid blood cell, or a fragment, variant or derivative of said tumour antigen; preferably survivin or a homologue thereof, an antigen from the MAGE-family or a binding partner thereof or a fragment, variant or derivative of said tumour antigen. Particularly preferred in this context are the tumour antigens NY-ESO-1, 5T4, MAGE-C1, MAGE-C2, Survivin, Muc-1, PSA, PSMA, PSCA, STEAP and PAP.

### 3.1.2 Secretory signal peptides:

Secretory signal peptides (abbreviated SSPs) are typically relatively short peptide stretches that promote the secretion of a protein polypeptide.

When used in combination with a polypeptide or protein of interest in the context of the present invention, such signal sequence is typically placed N-terminal to the polypeptide or protein of interest. On nucleic acid level, the coding sequence for such signal sequence is typically placed in frame (i.e. in the same reading frame), 5' to the coding sequence for the polypeptide or protein of interest.

Preferred secretory signal sequences are those functional in eukaryotic cells.

In the eukaryotic cell, the secretory signal peptide is typically cleaved from the nascent polypeptide chain immediately after it has been translocated into the membrane of the endoplasmic reticulum. The translocation occurs co-translationally and is dependent on a cytoplasmic protein-RNA complex (signal recognition particle, SRP). Without wishing to be bound to any particular theory, within the endoplasmic reticulum, protein folding and certain post-translational modifications can occur (e.g., glycosylation). Then, the protein is typically transported into Golgi vesicles and eventually secreted.

There is no well-defined consensus sequence or sequence motif for signal peptides, but there is a common structure. Secretory signal sequences have a tripartite structure, consisting of a hydrophobic core region flanked by an n- and c-region. Typically, the n-region is one to five amino acids in length, carrying positively charged amino acids. Between the hydrophobic core region and the signal peptidase cleavage site is the c-region, which consists of three to seven polar, but mostly uncharged, amino acids. Close to the cleavage site a more specific pattern of amino acids, known as the (3,1)-rule, is found: the amino acid residues at positions 3 and 1 (relative to the cleavage site) must be small and neutral for cleavage to occur correctly. For target proteins such as antigens associated with infectious diseases, a proper secretion of the antigen is beneficial for the induction of an immune response, because secretion of the antigen mimics the "natural" way of a viral infection and cytoplasmic localization of the expressed antigenic peptides/proteins could strongly limit the exposure of antigens to professional immune cells required for an induction of a humoral immune response.

Secretory signal peptides (SSPs) may be used as additional elements to promote or improve the secretion of the target protein (protein of interest). Suitably, the polypeptide sequence of the SSP used in the present invention is selected from the following list of polypeptide sequences (SEQ ID NOs: 1-1115 and SEQ ID NO: 1728).

On nucleic acid level, particularly RNA level, any nucleotide sequence moiety can be employed that encodes any of SSP used in the present invention. In specific embodiments, such nucleotide sequence is selected to encode a polypeptide selected from the following list of polypeptide sequences SEQ ID NOs: 1-1115 and SEQ ID NO: 1728. Owing to the degenerated genetic code, in the case of most polypeptides SEQ ID NOs: 1-1115 and SEQ ID NO: 1728, more than one particular nucleic acid sequence is conceivable as encoding the respective polypeptide. While each and every such nucleic acid may generally be used in the context of the present invention, it is preferable that the nucleic acid sequence that encodes the polypeptide sequence is selected such that its sequence is codon-optimized according to the general guidance provided in this specification.

Alternatively, any polypeptide element may be selected which is characterized by at least 80 % identity, at least 85 % identity, preferably at least 90 % identity, and more preferably at least 95 % identity to any of the sequences SEQ ID NOs: 1-1115 and SEQ ID NO: 1728). On nucleic acid level, any polynucleotide (e.g. RNA) moiety may be selected which encodes such polypeptide element.

### 3.1.3 Multimerization elements

For target proteins, such as antigens associated with infectious diseases, multimerization of the encoded antigen may be beneficial for the induction of an immune response. Fusion of the target antigen to multimerization elements (e.g., dimerization elements, trimerization elements, tetramerization elements, and oligomerization elements) may lead to the formation of multimeric antigen-complexes. This potentially increases immunogenicity of the respective antigen because such antigen-complexes may mimic a "natural" infection with an exogenous pathogen (e.g., virus) where a plurality of potential antigens is commonly located at the envelope of the pathogen (e.g., hemagglutinin (HA) antigen of the influenza virus).

When used in combination with a polypeptide or protein of interest in the context of the present invention, such multimerization element can be placed N-terminal or C-terminal to the polypeptide of interest. On nucleic acid level, the coding sequence for such multimerization element is typically placed in frame (i.e. in the same reading frame), 5' or 3' to the coding sequence for the polypeptide or protein of interest.

Particular multimerization elements are oligomerization elements, tetramerization elements, trimerization elements or dimerization elements.

Dimerization elements may be selected from e.g. dimerization elements/domains of heat shock proteins, immunoglobulin Fc domains and leucine zippers (dimerization domains of the basic region leucine zipper class of transcription factors). Specific elements are provided in SEQ ID NOs. 1116 - 1120.

Trimerization and tetramerization elements may be selected from e.g. engineered leucine zippers (engineered α-helical coiled coil peptide that adopt a parallel trimeric state), fibritin foldon domain from enterobacteria phage T4, GCN4pll, GCN4-pLl, and p53. Specific elements are provided in SEQ ID NOs. 1121-1145 (trimerization elements) and SEQ ID NOs. 1146-1149 (tetramerization elements).

Oligomerization elements may be selected from e.g. ferritin, surfactant D, oligomerization domains of phosphoproteins of paramyxoviruses, complement inhibitor C4 binding protein (C4bp) oligomerization domains, Viral infectivity factor (Vif) oligomerization domain, sterile alpha motif (SAM) domain, and von Willebrand factor type D domain.

Ferritin forms oligomers and is a highly conserved protein found in all animals, bacteria, and plants. Ferritin is a protein that spontaneously forms nanoparticles of 24 identical subunits. Ferritin-antigen fusion constructs potentially form oligomeric aggregates or "clusters" of antigens that may enhance the immune response.

Surfactant D protein (SPD) is a hydrophilic glycoprotein that spontaneously self-assembles to form oligomers. An SPD-antigen fusion constructs may form oligomeric aggregates or "clusters" of antigens that may enhance the immune response.

Phosphoprotein of paramyxoviruses (negative sense RNA viruses) functions as a transcriptional transactivator of the viral polymerase. Oligomerization of the phosphoprotein is critical for viral genome replication. A phosphoprotein-antigen fusion constructs may form oligomeric aggregates or "clusters" of antigens that may enhance the immune response.

Complement inhibitor C4 binding Protein (C4bp) may also be used as a fusion partner to generate oligomeric antigen aggregates. The C-terminal domain of C4bp (57 amino acid residues in humans and 54 amino acid residues in mice) is both necessary and sufficient for the oligomerization of C4bp or other polypeptides fused to it. A C4bp-antigen fusion constructs may form oligomeric aggregates or "clusters" of antigens that may enhance the immune response.

Viral infectivity factor (Vif) multimerization domain has been shown to form oligomers both *in vitro* and *in vivo.* The oligomerization of Vif involves a sequence mapping between residues 151 to 164 in the C-terminal domain, the 161PPLP164 motif (for human HIV-1: TPKKIKPPLP). A Vif-antigen fusion constructs may form oligomeric aggregates or "clusters" of antigens that may enhance the immune response.

The sterile alpha motif (SAM) domain is a protein interaction module present in a wide variety of proteins involved in many biological processes. The SAM domain that spreads over around 70 residues is found in diverse eukaryotic organisms. SAM domains have been shown to homo- and hetero-oligomerise, forming multiple self-association oligomeric architectures. A SAM-antigen fusion constructs may form oligomeric aggregates or "clusters" of antigens that may enhance the immune response.

von Willebrand factor (vWF) contains several type D domains: D1 and D2 are present within the N-terminal propeptide whereas the remaining D domains are required for oligomerization. The vWF domain is found in various plasma proteins: complement factors B, C2, CR3 and CR4; the Integrins (I-domains); collagen types VI, VII, XII and XIV; and other extracellular proteins. A vWF-antigen fusion constructs may form oligomeric aggregates or "clusters" of antigens that may enhance the immune response.

Specific elements suitable for oligomerization are provided in SEQ ID NOs. 1150 - 1167.

Multimerization elements useful in the present invention are provided in SEQ ID NOs: 1116-1167. Multimerization elements fused to respective target proteins (antigens) may be used to form antigen nanoparticles.

Suitably, the polypeptide sequence of the multimerization element used in the present invention is selected from the following list of polypeptide sequences (SEQ ID NOs: 1116-1167).

On nucleic acid level, particularly RNA level, any nucleotide sequence moiety can be employed that encodes any of oligomerization element used in the present invention. In specific embodiments, such nucleotide sequence is selected to encode a polypeptide selected from the following list of polypeptide sequences SEQ ID NOs: 1116-1167. Owing to the degenerated genetic code, in the case of most polypeptides SEQ ID NOs: 1116-1167, more than one particular nucleic acid sequence is conceivable as encoding the respective polypeptide. While each and every such nucleic acid may generally be used in the context of the present invention, it is preferable that the nucleic acid sequence that encodes the polypeptide sequence is selected such that its sequence is codon-optimized according to the general guidance provided in this specification.

Alternatively, any polypeptide element may be selected which is characterized by at least 80 % identity, at least 85 % identity, preferably at least 90 % identity, and more preferably at least 95 % identity to any of the sequences SEQ ID NOs: 1116-1167. On nucleic acid level, any polynucleotide (e.g. RNA) moiety may be selected which encodes such polypeptide element.

### 3.1.4 Virus like particle (VLP) forming elements

VLPs are self-assembled viral structural proteins (envelope proteins or capsid proteins) that structurally resemble viruses (without containing viral genetic material). VLPs contain repetitive high density displays of antigens which present conformational epitopes that can elicit strong T cell and B cell immune responses.

When used in combination with a polypeptide or protein of interest in the context of the present invention, such VLP forming element can be placed N-terminal or C-terminal to the polypeptide of interest. On nucleic acid level, the coding sequence for such VLP forming element is typically placed in frame (i.e. in the same reading frame), 5' or 3' to the coding sequence for the polypeptide or protein of interest.

For nucleic acid (e.g. RNA) encoding a polypeptide or protein of interest, particularly antigenic polypeptides or proteins associated with infectious (e.g. viral) diseases, it may be beneficial to introduce a VLP forming element into the respective constructs. In addition to the "clustering" of epitopes, an improved secretion of the VLP particle may also increase the immunogenicity of the respective antigen.

VLP forming elements fused to an antigen may generate virus like particles containing repetitive high density displays of antigens. VLP forming elements may be selected e.g. from any one of SEQ ID NOs: 1168-1227. Essentially, such VLP forming elements can be chosen from any viral or phage capsid or envelope protein.

VLP forming elements may be used as additional elements to promote or improve the particle formation of the target protein. Suitably, the polypeptide sequence of the VLP forming element used in the present invention is selected from the following list of polypeptide sequences (SEQ ID NOs: 1168-1227).

On nucleic acid level, particularly RNA level, any nucleotide sequence moiety can be employed that encodes any of VLP forming element used in the present invention. In specific embodiments, such nucleotide sequence is selected to encode a polypeptide selected from the following list of polypeptide sequences SEQ ID NOs: 1168-1227. Owing to the degenerated genetic code, in the case of most polypeptides SEQ ID NOs: 1168-1227, more than one particular nucleic acid sequence is conceivable as encoding the respective polypeptide of the below list. While each and every such nucleic acid may generally be used in the context of the present invention, it is preferable that the nucleic acid sequence that encodes the polypeptide sequence is selected such that its sequence is codon-optimized according to the general guidance provided in this specification.

Alternatively, any polypeptide element may be selected which is characterized by at least 80 % identity, at least 85 % identity, preferably at least 90 % identity, and more preferably at least 95 % identity to any of the sequences SEQ ID NOs: 1168-1227. On nucleic acid level, any polynucleotide (e.g. RNA) moiety may be selected which encodes such polypeptide element.

### 3.1.5 Transmembrane elements

Transmembrane elements or membrane spanning polypeptide elements are present in proteins that are integrated or anchored in plasma membranes of cells. Typical transmembrane elements are alpha-helical transmembrane elements. Such transmembrane elements are composed essentially of amino acids with hydrophobic side chains, because the interior of a cell membrane (lipid bilayer) is also hydrophobic. From the structural perspective, transmembrane elements are commonly single hydrophobic alpha helices or beta barrel structures; whereas hydrophobic alpha helices are usually present in proteins that are present in membrane anchored proteins (e.g., seven transmembrane domain receptors), beta-barrel structures are often present in proteins that generate pores or channels.

For target proteins, such as antigens associated with infectious (e.g. viral) diseases, it may be beneficial to introduce a transmembrane element into the respective constructs. By addition of a transmembrane element to the target peptide/protein it may be possible to further enhance the immune response, wherein the translated target peptide/protein, e.g. a viral antigen, anchors to a target membrane, e.g. the plasma membrane of a cell, thereby increasing immune responses. This effect is also referred to as antigen clustering.

When used in combination with a polypeptide or protein of interest in the context of the present invention, such transmembrane element can be placed N-terminal or C-terminal to the polypeptide of interest. On nucleic acid level, the coding sequence for such transmembrane element is typically placed in frame (i.e. in the same reading frame), 5' or 3' to the coding sequence for the polypeptide or protein of interest.

The transmembrane domain may be selected from the transmembrane domain of Hemagglutinin (HA) of Influenza virus, Env of HIV-1, EIAV (equine infectious anaemia virus), MLV (murine leukaemia virus), mouse mammary tumor virus, G protein of VSV (vesicular stomatitis virus), Rabies virus, or a transmembrane element of a seven transmembrane domain receptor. Specific elements are provided in the Table below.

As shown in the examples of the present invention, it is equally possible by the present invention to remove a transmembrane (TM) element from a polypeptide or protein. Thereby, an optimized nucleic acid can be prepared which does not code for the respective transmembrane (TM) element.

Suitably, the polypeptide sequence of the transmembrane (TM) domain used in the present invention is selected from the following list of polypeptide sequences (SEQ ID NOs: 1228-1343).

On nucleic acid level, particularly RNA level, any nucleotide sequence moiety can be employed that encodes any transmembrane (TM) domain used in the present invention. In specific embodiments, such nucleotide sequence is selected to encode a polypeptide selected from the following list of polypeptide sequences SEQ ID NOs: 1228-1343. Owing to the degenerated genetic code, in the case of most polypeptides SEQ ID NOs: 1228-1343, more than one particular nucleic acid sequence is conceivable as encoding the respective polypeptide. While each and every such ribonucleic acid may generally be used in the context of the present invention, it is preferable that the ribonucleic acid sequence that encodes the polypeptide sequence is selected such that its sequence is codon-optimized according to the general guidance provided in this specification.

Alternatively, any polypeptide element may be selected which is characterized by at least 80 % identity, at least 85 % identity, preferably at least 90 % identity, and more preferably at least 95 % identity to any of the sequences SEQ ID NOs: 1228-1343. On nucleic acid level, any polynucleotide (e.g. RNA) moiety may be selected which encodes such polypeptide element.

### 3.1.6 Dendritic cell targeting elements

Dendritic cells (DCs), the most potent antigen presenting cells (APCs), link the innate immune response to the adaptive immune response. They bind and internalize pathogens/antigens and display fragments of the antigen on their membrane (via MHC molecules) to stimulate T-cell responses against those pathogens/antigens. Polypeptide elements capable of targeting to dendritic cells are referred to as dendritic cell targeting elements.

When used in combination with a polypeptide or protein of interest in the context of the present invention, such dendritic cell targeting element can be placed N-terminal or C-terminal to the polypeptide of interest. On nucleic acid level, the coding sequence for such dendritic cell element is typically placed in frame (i.e. in the same reading frame), 5' or 3' to the coding sequence for the polypeptide or protein of interest.

Targeting antigens to DCs is an appropriate method to stimulate and induce effective antitumor and antiviral immune responses. To achieve dendritic cell targeting, proteins/peptides (e.g., antibody fragments, receptor ligands) that bind to DC surface receptors have to be fused to the respective antigen/target protein. Such DC receptors include C-type lectins (mannose receptors (e.g., MR1, DEC-205 (CD205)), CD206, DC-SIGN (CD209), Clec9a, DCIR, Lox-1, MGL, MGL-2, Clec12A, Dectin-1, Dectin-2, langerin (CD207)), scavenger receptors, F4/80 receptors (EMR1), DC-STAMP, receptors for the Fc portion of antibodies (Fc receptors), toll-like receptors (e.g., TLR2, 5, 7, 8, 9) and complement receptors (e.g., CR1, CR2).

An antigen may be fused to the following elements to obtain targeting of dendritic cells: anti-DC-SIGN antibody, CD11c specific single chain fragments (scFV), DEC205-specific single chain fragments (scFV), soluble PD-1, chemokine (C motif) ligand XCL1, CD40 ligand, human IgG1, murine IgG2a, anti Celec 9A, anti MHCII scFV.

Essentially, any other protein/peptide element that binds to a receptor localized on dendritic cells may be used as an element (Apostolopoulos, Vasso, et al. "Targeting antigens to dendritic cell receptors for vaccine development." Journal of drug delivery 2013 (2013); Kastenmüller, Wolfgang, et al. "Dendritic cell-targeted vaccines - hope or hype?" Nature Reviews Immunology 14.10 (2014): 705-711). Such dendritic cell antigens are also contemplated in the present invention.

Suitably, the polypeptide sequence of the dendritic cell targeting element used in the present invention is selected from the following list of polypeptide sequences (SEQ ID NOs: 1344-1359).

On nucleic acid level, particularly RNA level, any nucleotide sequence moiety can be employed that encodes any of dendritic cell targeting elements used in the present invention. In specific embodiments, such nucleotide sequence is selected to encode a polypeptide selected from the following list of polypeptide sequences SEQ ID NOs: 1344-1359. Owing to the degenerated genetic code, in the case of most polypeptides SEQ ID NOs: 1344-1359, more than one particular nucleic acid sequence is conceivable as encoding the respective polypeptide. While each and every such nucleic acid may generally be used in the context of the present invention, it is preferable that the nucleic acid sequence that encodes the polypeptide sequence is selected such that its sequence is codon-optimized according to the general guidance provided in this specification.

Alternatively, any polypeptide element may be selected which is characterized by at least 80 % identity, at least 85 % identity, preferably at least 90 % identity, and more preferably at least 95 % identity to any of the sequences SEQ ID NOs: 1344-1359. On nucleic acid level, any polynucleotide (e.g. RNA) moiety may be selected which encodes such polypeptide element.

### 3.1.7 Immunologic adjuvant elements

Immunologic adjuvant elements, or adjuvant elements, may comprise peptide or protein elements that potentiate or "govern" the immune response. Such elements may include peptides/proteins that trigger a danger response (e.g., damage-associated molecular pattern molecules (DAMPs)), elements that activate the complement system (e.g., peptides/proteins involved in the classical complement pathway, the alternative complement pathway, and the lectin pathway) or elements that activate an innate immune response (e.g., pathogen-associated molecular pattern molecules, PAMPs).

When used in combination with a polypeptide or protein of interest in the context of the present invention, such immunologic adjuvant element can be placed N-terminal or C-terminal to the polypeptide of interest. On nucleic acid level, the coding sequence for such immunologic adjuvant element is typically placed in frame (i.e. in the same reading frame), 5' or 3' to the coding sequence for the polypeptide or protein of interest.

For target peptides/proteins such as antigens associated with infectious diseases or antigens associated with tumor diseases it may be beneficial to fuse the respective target peptide/protein to elements that potentiate the immune response against the target peptide/protein or shunts the immune response against the target peptide/protein towards a desired response (e.g., humoral or cellular response).

Immunologic adjuvant elements that may be fused to a target protein, may be selected from heat shock proteins (e.g., HSP60, HSP70, gp96), flagellin FliC, high mobility group box 1 proteins (e.g., HMGN1), extra domain A of fibronectin (EDA), C3 protein fragments (e.g. C3d), transferrin, β-defensin, or any other peptide/protein PAMP-receptor (PRs) ligand, DAMP or element that activates the complement system. Specific elements are provided in the table below (polypeptide sequences). Thus, suitably, the polypeptide sequence of the adjuvant element used in the present invention is selected from the following list of polypeptide sequences (SEQ ID NOs: 1360-1421).

On RNA level any ribonucleotide sequence moiety can be employed that encodes any of adjuvant element used in the present invention. In specific embodiments, such ribonucleotide sequence is selected to encode a polypeptide selected from the following list of polypeptide sequences SEQ ID NOs: 1360-1421. Owing to the degenerated genetic code, in the case of most polypeptides SEQ ID NOs: 1360-1421, more than one particular nucleic acid sequence is conceivable as encoding the respective polypeptide. While each and every such nucleic acid may generally be used in the context of the present invention, it is preferable that the nucleic acid sequence that encodes the polypeptide sequence is selected such that its sequence is codon-optimized according to the general guidance provided in this specification.

Alternatively, any polypeptide element may be selected which is characterized by at least 80 % identity, at least 85 % identity, preferably at least 90 % identity, and more preferably at least 95 % identity to any of the sequences SEQ ID NOs: 1360-1421. On nucleic acid level, any polynucleotide (e.g. RNA) moiety may be selected which encodes such polypeptide element.

### 3.1.8 Elements promoting antigen presentation

Many pathogens are too large to be recognized directly by immune cells. Therefore, they have to be internalized and digested into smaller fragments by specialized antigen-presenting cells (APCs), e.g. dendritic cells. The digestion of larger proteins occurs in a dedicated cellular compartment, the lysosome or the proteasome. After digestion, the smaller fragments are transported via exosome trafficking to the cell surface where the fragments are presented by major histocompatibility complex (MHC) molecules on the cell surface.

For target peptides/proteins such as antigens associated with infectious diseases or antigens associated with tumor diseases it may be beneficial to fuse the respective target peptide/protein to elements that promote antigen presentation. Such elements may comprise peptides/proteins that trigger the entry into the lysosome/proteasome pathway or that promote the entry into the exosome. In general, when used in combination with a polypeptide or protein of interest in the context of the present invention, such immunologic adjuvant element can be placed N-terminal or C-terminal to the polypeptide of interest. However, in practice, some particular elements may be particularly functional when they are present either at the N-terminus, or at the C-terminus, i.e. at the same terminus at which the respective particular elements are found in the wild-type context. In general, on nucleic acid level, the coding sequence for such immunologic adjuvant element is typically placed in frame (i.e. in the same reading frame), 5' or 3' (in analogy to the respective wild-type context) to the coding sequence for the polypeptide or protein of interest.

Such elements promoting antigen presentation may be selected e.g. from MHC invariant chain (li), invariant chain (li) lysosome targeting signal, sorting signal of the lysosomal-associated membrane protein LAMP-1, lysosomal integral membrane protein-II (LIMP-II), C1C2 Lactadherin domain. Specific elements are provided in the table below.

Elements promoting antigen presentation (or antigen-presentation promoting elements) may be used as additional elements to promote or improve the secretion of the target protein. Suitably, the polypeptide sequence of the antigen-presentation promoting element used in the present invention is selected from the following list of polypeptide sequences (SEQ ID NOs: 1422-1433).

On RNA level any nucleotide sequence moiety can be employed that encodes any of the antigen-presentation promoting elements used in the present invention. In specific embodiments, such nucleotide sequence is selected to encode a polypeptide selected from the following list of polypeptide sequences SEQ ID NOs: 1422-1433. Owing to the degenerated genetic code, in the case of most polypeptides SEQ ID NOs: 1422-1433, more than one particular nucleic acid sequence is conceivable as encoding the respective polypeptide. While each and every such nucleic acid may generally be used in the context of the present invention, it is preferable that the nucleic acid sequence that encodes the polypeptide sequence is selected such that its sequence is codon-optimized according to the general guidance provided in this specification.

Alternatively, any polypeptide element may be selected which is characterized by at least 80 % identity, at least 85 % identity, preferably at least 90 % identity, and more preferably at least 95 % identity to any of the sequences SEQ ID NOs: 1422-1433. On nucleic acid level, any RNA moiety may be selected which encodes such polypeptide element.

### 3.1.9 2A peptides

Viral 2A peptides ("self-cleaving" peptides) allow the expression of multiple proteins from a single open reading frame. The terms 2A peptide and 2A element are used interchangeably herein. The mechanism by the 2A sequence for generating two proteins from one transcript is by ribosome skipping - a normal peptide bond is impaired at 2A, resulting in two discontinuous protein fragments from one translation event.

When used in the context of the present invention, such 2A peptides are particularly useful when encoded by a nucleic acid encoding at least two functional protein elements. In general, a 2A element is useful when the nucleic acid molecule encodes at least one polypeptide or protein of interest and at least one further protein element. In a preferred embodiment, a 2A element is present when the polynucleotide of the invention encodes two proteins or polypeptides of interest, e.g. two antigens.

The coding sequence for such 2A peptide is typically located in between the coding sequence of the polypeptide of interest and the coding sequence of the least one further protein element (which may also be a polypeptide of interest), so that cleavage of the 2A peptide leads to two separate polypeptide molecules, at least one of them being a polypeptide or protein of interest.

For example, for expressing target proteins that are composed of several polypeptide chains, such as antibodies, it may be beneficial to provide coding information for both polypeptide chains on a single nucleic acid molecule, separated by a nucleic acid sequence encoding a 2A peptide. 2A peptides may also be beneficial when cleavage of the protein of interest from another encoded polypeptide element is desired.

2A peptides may be derived from foot-and-mouth diseases virus, from equine rhinitis A virus, Thosea asigna virus, Porcine teschovirus-1. Specific elements are provided in the table below. Suitably, the polypeptide sequence of the 2A peptide used in the present invention is selected from the following list of polypeptide sequences (SEQ ID NOs: 1434-1508).

On nucleic acid level, particularly RNA level, any nucleotide sequence moiety can be employed that encodes any of 2A peptide used in the present invention. In specific embodiments, such nucleotide sequence is selected to encode a polypeptide selected from the following list of polypeptide sequences SEQ ID NOs: 1434-1508. Owing to the degenerated genetic code, in the case of most polypeptides SEQ ID NOs: 1434-1508, more than one particular nucleic acid sequence is conceivable as encoding the respective polypeptide. While each and every such nucleic acid may generally be used in the context of the present invention, it is preferable that the nucleic acid sequence that encodes the polypeptide sequence is selected such that its sequence is codon-optimized according to the general guidance provided in this specification.

Alternatively, any polypeptide element may be selected which is characterized by at least 80 % identity, at least 85 % identity, preferably at least 90 % identity, and more preferably at least 95 % identity to any of the sequences SEQ ID NOs: 1434-1508. On nucleic acid level, any polynucleotide (e.g. RNA) moiety may be selected which encodes such polypeptide element.

### 3.1.10 Peptide linker elements

In protein constructs composed of several elements (e.g., target protein fused to a transmembrane domain), the protein elements are often separated by peptide linker elements. The same applies for polypeptides of interest having various domains. Such elements may be beneficial because they allow for a proper folding of the individual elements and thereby the proper functionality of each element. Alternatively, the term "spacer" or "peptide spacer" is used herein.

When used in the context of the present invention, such linkers or spacers are particularly useful when encoded by a nucleic acid encoding at least two functional protein elements, such as at least one polypeptide or protein of interest and at least one further protein or polypeptide element, preferably also selected from the list of coding moieties of the present invention. In that case, the linker is typically located on the polypeptide chain in between the polypeptide of interest and the at least one further protein element. On nucleic acid level, the coding sequence for such linker is typically placed in the reading frame, 5' or 3' to the coding sequence for the polypeptide or protein of interest, or placed between coding regions for individual polypeptide domains of a given protein of interest.

Peptide linkers are preferably composed of small, non-polar (e.g. Gly) or polar (e.g. Ser or Thr) amino acids. The small size of these amino acids provides flexibility, and allows for mobility of the connecting functional domains, as described by Chen et al. (Adv Drug Deliv Reb. 2013; 65(10): 1357-1369). The incorporation of Ser or Thr can maintain the stability of the linker in aqueous solutions by forming hydrogen bonds with the water molecules, and therefore reduces an interaction between the linker and the protein moieties. Rigid linkers generally maintain the distance between the protein domains and they may be based on helical structures and/or they have a sequence that is rich in proline. Cleavable linkers (also termed "cleavage linkers") allow for *in vivo* separation of the protein domains. The mechanism of cleavage may be based e.g. on reduction of disulfide bonds within the linker sequence or proteolytic cleavage. The cleavage may be mediated by an enzyme (enzymatic cleavage), e.g. the cleavage linker may provide a protease sensitive sequence (e.g., furin cleavage).

A typical sequence of a flexible linker is composed of repeats of the amino acids Glycine (G) and Serine (S). For instance, the linker may have the following sequence: GS, GSG, SGG, SG, GGS, SGS, GSS, SSG. In some embodiments, the same sequence is repeated multiple times (e.g. two, three, four, five or six times) to create a longer linker. In other embodiments, a single amino acid residue such as S or G can be used as a linker.

Peptide linkers, including cleavage linkers, flexible linkers and rigid linkers, or spacers, may be selected from the ones shown in the table below.

Linkers or spacers may be used as additional elements to promote or improve the secretion of the target protein. Suitably, the polypeptide sequence of the linker or spacer used in the present invention is selected from the following list of polypeptide sequences (SEQ ID NOs: 1509-1565).

On RNA level any ribonucleotide sequence moiety can be employed that encodes any of linker or spacer used in the present invention. In specific embodiments, such nucleotide sequence is selected to encode a polypeptide selected from the following list of polypeptide sequences SEQ ID NOs: 1509-1565. Owing to the degenerated genetic code, in the case of most polypeptides of SEQ ID NOs: 1509-1565, more than one particular nucleic acid sequence is conceivable as encoding the respective polypeptide list. While each and every such nucleic acid may generally be used in the context of the present invention, it is preferable that the nucleic acid sequence that encodes the polypeptide sequence is selected such that its sequence is codon-optimized according to the general guidance provided in this specification.

Alternatively, any polypeptide element may be selected which is characterized by at least 80 % identity, at least 85 % identity, preferably at least 90 % identity, and more preferably at least 95 % identity to any of the sequences SEQ ID NOs: 1509-1565. On nucleic acid level, any polynucleotide (e.g. RNA) moiety may be selected which encodes such polypeptide element.

### 3.1.11 Elements that extend protein half-life

The plasma half-life of therapeutic proteins is a critical factor in many clinical applications. When extension of protein half-life is desired, protein elements described herein may be incorporated. Such half-life extending elements are particularly useful for target proteins that are smaller than the kidney filtration cutoff of around 70 kDa and/or are subject to metabolic turnover by peptidases, which significantly limits their plasma half-life *in vivo.*

The mRNA molecule as defined in the claims is suitable for application in gene therapy and/or genetic vaccination and comprises a 5'-untranslated region (5'-UTR) and a 3'-untranslated region (3'-UTR), the mRNA further comprises at least two modules, wherein each module is a ribonucleic acid moiety, wherein at least one module is an open reading frame (ORF) encoding a polypeptide or protein of interest, and wherein at least one module is a further coding ribonucleic acid moiety encoding at least one additional polypeptide or protein element encoded in the same open reading frame as the polypeptide or protein of interest, wherein the at least one additional polypeptide or protein element is an element that extends protein half-life, and wherein the polypeptide or protein element is characterized by at least 80% identity to SEQ ID NO. 1705, and wherein the open reading frame is or comprises a G/C-modified nucleic acid sequence, wherein the number of G and/or C nucleotides is increased as compared to the wild-type sequence.

Elements that extend protein half-life may be derived from homo-amino acid polymer (HAPylation), albumin, the Fc portion of immmunoglobulins, albumin binding domains, albumin binding peptide, poly-glycine elements, elastin-like elements, transferrin, proline-alanine-serine polymers (PASylation), HCG beta-subunit CTP elements, XTEN derived elements, ELP elements (ELPylation), gelatin-like protein polymers, IgG1, IgG2, Ig binding domain of Staphylococcus, etc. Specific additional elements are provided in SEQ ID NOs: 1671 - 1727. Any other element that extends the half-life of the respective target protein may be suitable in additional to what is defined in the claims. Owing to the degenerated genetic code, in the case of most polypeptides of SEQ ID NOs: 1671 -1727, more than one particular nucleic acid sequence is conceivable as encoding the respective polypeptide. While each and every such nucleic acid may generally be used in the context of the present invention, it is preferable that the nucleic acid sequence that encodes the polypeptide sequence is selected such that its sequence is codon-optimized according to the general guidance provided in this specification. Alternatively, any polypeptide element may be selected additionally which is characterized by at least 80 % identity, at least 85 % identity, preferably at least 90 % identity, and more preferably at least 95 % identity to any of the sequences SEQ ID NOs: 1671 - 1727.

On nucleic acid level, any RNA moiety may be selected which encodes such polypeptide element.

### 3.1.11 Additional coding modules

The protein of interest is fused or comprises additional coding modules, wherein at least one module is a further coding ribonucleic acid moiety encoding at least one additional polypeptide or protein element encoded in the same open reading frame as the polypeptide or protein of interest, wherein the at least one additional polypeptide or protein element is an element that extends protein half-life, and wherein the polypeptide or protein element is characterized by at least 80% identity to SEQ ID NO. 1705. Further coding modules may be as listed below:
- Elements encoding for cellular localisation signals including but not limited to membrane insertion or nuclear import signals
- Elements suitable for targeting intracellular or extracellular proteins including but not limited to cellular receptors
- Element suitable for targeting cell surface molecules including but not limited to glycans and cellular matrix components
- Elements bearing mutations to stabilise defined folding states
- Elements bearing mutations to enhance a multimeric assembly
- Element generally stabilizing the protein
- element enhancing protein solubility by altering hydrophobicity/hydrophilicity of the target protein
- Elements suitable for recruiting parts of the cellular machinery

### 3.2 Non-coding modules

At least one non-coding nucleic acid moiety is present in the optimized nucleic acid molecule of the present invention, namely the mRNA molecule comprises a 5'-untranslated region (5'-UTR) and a 3'-untranslated region (3'-UTR).

### 3.2.1 UTRs

Untranslated regions (UTRs) are non-coding moieties of a nucleic acid sequence, particularly of an RNA sequence, preferably mRNA, sequence.

The mRNA molecule comprises a 5'-untranslated region (5'-UTR) and a 3'-untranslated region (3'-UTR). Suitable UTR moieties are selected from 5'-UTR moieties and 3'-UTR moieties. Moreover, the mRNA molecule comprises at least one open reading frame, at least one 3'-UTR (moiety) and at least one 5'-UTR (moiety).

Preferably, the at least one 3'-UTR moiety and/or the at least one 5'-UTR moiety in the mRNA molecule according to the present invention comprises or consists of a nucleic acid sequence which is derived from the 3'-UTR and/or the 5'-UTR of a eukaryotic protein coding gene, preferably from the 3'-UTR and/or the 5'-UTR of a vertebrate protein coding gene, more preferably from the 3'-UTR and/or the 5'-UTR of a mammalian protein coding gene, e.g. from mouse and human protein coding genes, even more preferably from the 3'-UTR and/or the 5'-UTR of a primate or rodent protein coding gene, in particular the 3'-UTR and/or the 5'-UTR of a human or murine protein coding gene.

In general, it is understood that the at least one 3'-UTR moiety in the mRNA molecule according to the present invention comprises or consists of a ribonucleic acid sequence which is preferably derived from a naturally (in nature) occurring 3'-UTR, whereas the at least one 5'-UTR moiety in the mRNA molecule as claimed comprises or consists of a ribonucleic acid sequence which is preferably derived from a naturally (in nature) occurring 5'-UTR.

Preferably, the at least one open reading frame is heterologous to the at least one 3'-UTR moiety and/or to the at least one 5'-UTR moiety. The term "heterologous" in this context means that two sequence moieties comprised by the mRNA molecule, such as the open reading frame and the 3'-UTR moiety and/or the open reading frame and the 5'-UTR moiety, do not occur naturally (in nature) in this combination. They are typically recombinant. Preferably, the 3'-UTR moiety and/or the 5'-UTR moiety are/is derived from a different gene than the open reading frame. For example, the ORF may be derived from a different gene than the 3'-UTR moiety and/or to the at least one 5'-UTR moiety, e.g. encoding a different protein or the same protein but of a different species etc. I.e. the open reading frame is derived from a gene which is distinct from the gene from which the 3'-UTR moiety and/or to the at least one 5'-UTR moiety is derived. In a preferred embodiment, the ORF does not encode a human or plant (e.g., Arabidopsis) ribosomal protein, preferably does not encode human ribosomal protein S6 (RPS6), human ribosomal protein L36a-like (RPL36AL) or Arabidopsis ribosomal protein S16 (RPS16). In a further preferred embodiment, the open reading frame (ORF) does not encode ribosomal protein S6 (RPS6), ribosomal protein L36a-like (RPL36AL) or ribosomal protein S16 (RPS16).

In specific embodiments it is preferred that the open reading frame does not code for a reporter protein, e.g., selected from the group consisting of globin proteins (particularly beta-globin), luciferase protein, GFP proteins or variants thereof, for example, variants exhibiting at least 70% sequence identity to a globin protein, a luciferase protein, or a GFP protein. Thereby, it is particularly preferred that the open reading frame does not code for a GFP protein. It is also particularly preferred that the open reading frame (ORF) does not encode a reporter gene or is not derived from a reporter gene, wherein the reporter gene is preferably not selected from group consisting of globin proteins (particularly beta-globin), luciferase protein, beta-glucuronidase (GUS) and GFP proteins or variants thereof, preferably not selected from EGFP, or variants of any of the above genes, typically exhibiting at least 70% sequence identity to any of these reporter genes, preferably a globin protein, a luciferase protein, or a GFP protein.

Even more preferably, the 3'-UTR moiety and/or the 5'-UTR moiety is heterologous to any other moiety comprised in the optimized nucleic acid as defined herein. For example, if the mRNA according to the invention comprises a 3'-UTR moiety from a given gene, it does preferably not comprise any other nucleic acid sequence, in particular no functional nucleic acid sequence (e.g. coding or regulatory sequence moiety) from the same gene, including its regulatory sequences at the 5' and 3' terminus of the gene's ORF. Accordingly, for example, if the mRNA according to the invention comprises a 5'-UTR moiety from a given gene, it does preferably not comprise any other nucleic acid sequence, in particular no functional nucleic acid sequence (e.g. coding or regulatory sequence moiety) from the same gene, including its regulatory sequences at the 5' and 3' terminus of the gene's ORF.

Preferably, the at least one 3'-UTR moiety and/or the at least one 5'-UTR moiety is functionally linked to an open reading frame (ORF) of the mRNA molecule. This means preferably that the 3'-UTR moiety and/or to the at least one 5'-UTR moiety is associated with the ORF such that it may exert a function, such as an enhancing or stabilizing function on the expression of the encoded peptide or protein or a stabilizing function on the optimized nucleic acid molecule. Preferably, the ORF and the 3'-UTR moiety are associated in 5'→3' direction and/or the 5'-UTR moiety and the ORF are associated in 5'→3' direction. Thus, preferably, the optimized nucleic acid molecule comprises in general the structure 5'-[5'-UTR moiety]-(optional)-linker-ORF-(optional)-linker-[3'-UTR moiety]-3', wherein the optimized nucleic acid molecule may comprise only a 5'-UTR moiety and no 3'-UTR moiety, only a 3'-UTR moiety and no 5'-UTR moiety, or both, a 3'-UTR moiety and a 5'-UTR moiety. Furthermore, the linker may be present or absent. For example, the linker may be one or more nucleotides, such as a stretch of 1-50 or 1-20 nucleotides, e.g., comprising or consisting of one or more restriction enzyme recognition sites (restriction sites).

Preferably, the at least one 3'-UTR moiety and/or the at least one 5'-UTR moiety comprises or consists of a nucleic acid sequence which is derived from the 3'-UTR and/or the 5'-UTR of a transcript of a gene. Preferably, the at least one 3'-UTR moiety and/or the at least one 5'-UTR moiety of the mRNA molecule according to the present invention comprises or consists of a "functional fragment", a "functional variant" or a "functional fragment of a variant" of the 3'-UTR and/or the 5'-UTR of a transcript of a gene.

The phrase "nucleic acid sequence which is derived from the 3'-UTR and/or the 5'-UTR of a of a transcript of a gene" preferably refers to a nucleic acid sequence which is based on the 3'-UTR sequence and/or on the 5'-UTR sequence of a transcript of a gene or a fragment or part thereof, preferably a naturally occurring gene or a fragment or part thereof. In this context, the term naturally occurring is used synonymously with the term wild-type. This phrase includes sequences corresponding to the entire 3'-UTR sequence and/or the entire 5'-UTR sequence, i.e. the full length 3'-UTR and/or 5'-UTR sequence of a transcript of a gene, and sequences corresponding to a fragment of the 3'-UTR sequence and/or the 5'-UTR sequence of a transcript of a gene. Preferably, a fragment of a 3'-UTR and/or a 5'-UTR of a transcript of a gene consists of a continuous stretch of nucleotides corresponding to a continuous stretch of nucleotides in the full-length 3'-UTR and/or 5'-UTR of a transcript of a gene, which represents at least 5%, 10%, 20%, preferably at least 30%, more preferably at least 40%, more preferably at least 50%, even more preferably at least 60%, even more preferably at least 70%, even more preferably at least 80%, and most preferably at least 90% of the full-length 3'-UTR and/or 5'-UTR of a transcript of a gene. Such a fragment is preferably a functional fragment as described herein. Preferably, the fragment retains a regulatory function for the translation of the ORF linked to the 3'-UTR and/or 5'-UTR or fragment thereof.

The terms "variant of the 3'-UTR and/or variant of the 5'-UTR of a of a transcript of a gene" and "variant thereof" in the context of a 3'-UTR and/or a 5'-UTR of a transcript of a gene refers to a variant of the 3'-UTR and/or 5'-UTR of a transcript of a naturally occurring gene, preferably to a variant of the 3'-UTR and/or 5'-UTR of a transcript of a vertebrate gene, more preferably to a variant of the 3'-UTR and/or 5'-UTR of a transcript of a mammalian gene, even more preferably to a variant of the 3'-UTR and/or 5'-UTR of a transcript of a primate gene, in particular a human gene as described above. Such variant may be a modified 3'-UTR and/or 5'-UTR of a transcript of a gene. For example, a variant 3'-UTR and/or a variant of the 5'-UTR may exhibit one or more nucleotide deletions, insertions, additions and/or substitutions compared to the naturally occurring 3'-UTR and/or 5'-UTR from which the variant is derived. Preferably, a variant of a 3'-UTR and/or variant of the 5'-UTR of a of a transcript of a gene is at least 40%, preferably at least 50%, more preferably at least 60%, more preferably at least 70%, even more preferably at least 80%, even more preferably at least 90%, most preferably at least 95% identical to the naturally occurring 3'-UTR and/or 5'-UTR the variant is derived from. Preferably, the variant is a functional variant as described herein.

The terms "functional variant", "functional fragment", and "functional fragment of a variant" (also termed "functional variant fragment") in the context of the present invention, mean that the fragment of the 3'-UTR and/or the 5'-UTR, the variant of the 3'-UTR and/or the 5'-UTR, or the fragment of a variant of the 3'-UTR and/or the 5'-UTR of a transcript of a gene fulfils at least one, preferably more than one function of the naturally occurring 3'-UTR and/or 5'-UTR of a transcript of a gene of which the variant, the fragment, or the fragment of a variant is derived. Such function may be, for example, stabilizing mRNA and/or enhancing, stabilizing and/or prolonging protein production from an mRNA and/or increasing protein expression or total protein production from an mRNA, preferably in a mammalian cell, such as in a human cell. Preferably, the function of the 3'-UTR and/or the 5'-UTR concerns the translation of the protein encoded by the ORF. More preferably, the function comprises enhancing translation efficiency of the ORF linked to the 3'-UTR and/or the 5'-UTR or fragment or variant thereof. It is particularly preferred that the variant, the fragment, and the variant fragment in the context of the present invention fulfil the function of stabilizing an mRNA, preferably in a mammalian cell, such as a human cell, compared to an mRNA comprising a reference 3'-UTR and/or a reference 5'-UTR or lacking a 3'-UTR and/or a 5'-UTR, and/or the function of enhancing, stabilizing and/or prolonging protein production from an mRNA, preferably in a mammalian cell, such as in a human cell, compared to an mRNA comprising a reference 3'-UTR and/or a reference 5'-UTR or lacking a 3'-UTR and/or a 5'-UTR, and/or the function of increasing protein production from an mRNA, preferably in a mammalian cell, such as in a human cell, compared to an mRNA comprising a reference 3'-UTR and/or a reference 5'-UTR or lacking a 3'-UTR and/or a 5'-UTR. A reference 3'-UTR and/or a reference 5'-UTR may be, for example, a 3'-UTR and/or a 5'-UTR naturally occurring in combination with the ORF. Furthermore, a functional variant, a functional fragment, or a functional variant fragment of a 3'-UTR and/or a 5'-UTR of a transcript of a gene preferably does not have a substantially diminishing effect on the efficiency of translation of the mRNA which comprises such variant, fragment, or variant fragment of a 3'-UTR and/or a 5'-UTR compared to the wild-type 3'-UTR and/or the wild-type 5'-UTR from which the variant, the fragment, or the variant fragment is derived. A particularly preferred function of a "functional fragment", a "functional variant" or a "functional fragment of a variant" of the 3'-UTR and/or the 5'-UTR of a transcript of a gene in the context of the present invention is the enhancement, stabilization and/or prolongation of protein production by expression of an mRNA carrying the functional fragment, functional variant or functional fragment of a variant as described above. In the context of the present invention, the functional fragment of the 3'-UTR and/or of the 5'-UTR preferably exhibits a length of at least about 3 nucleotides, preferably of at least about 5 nucleotides, more preferably of at least about 10, 15, 20, 25 or 30 nucleotides, even more preferably of at least about 50 nucleotides, most preferably of at least about 70 nucleotides. In a preferred embodiment, the 3'-UTR and/or the 5'-UTR of a transcript of a gene or a fragment or variant thereof exhibits a length of between 3 and about 500 nucleotides, preferably of between 5 and about 150 nucleotides, more preferably of between 10 and 100 nucleotides, even more preferably of between 15 and 90, most preferably of between 20 and 70. Typically, the 5'-UTR moiety and/or the 3'-UTR moiety is characterized by less than 500, 400, 300, 200, 150 or less than 100 nucleotides.

The present invention comprises the association of such 5'-UTRs and 3'-UTRs with a nucleic acid molecule of interest, e.g. an ORF. The terms "associating the nucleic acid molecule or the vector with a 3'-UTR moiety and/or a 5'-UTR moiety" or "associating the optimized nucleic acid molecule or the vector with a 3'-UTR moiety and/or a 5'-UTR moiety", or the like, in the context of the present invention preferably means functionally associating or functionally combining the artificial (optimized) nucleic acid molecule or the vector with the 3'-UTR moiety and/or with the 5'-UTR moiety. Thereby, further optimization (i.e. gain of additional desired functional properties) may be achieved. This means that the artificial (optimized) nucleic acid molecule and the 3'-UTR moiety and/or the 5'-UTR moiety, preferably the 3'-UTR moiety and/or the 5'-UTR moiety, are associated or coupled such that the function of the 3'-UTR moiety and/or of the 5'-UTR moiety, e.g., the RNA and/or protein production prolonging and/or increasing function, is exerted. Typically, this means that the 3'-UTR moiety and/or the 5'-UTR moiety is integrated into the artificial (optimized) nucleic acid molecule, preferably the mRNA molecule, 3' and/or 5', respectively, to an open reading frame (ORF), preferably immediately 3' to an open reading frame and/or immediately 5' to an open reading frame, the 3'-UTR moiety preferably between the open reading frame and a poly(A) sequence or a polyadenylation signal. Preferably, the 3'-UTR moiety and/or the 5'-UTR moiety is integrated into the artificial (optimized) nucleic acid molecule or the vector, preferably the mRNA, as 3'-UTR and/or as 5'-UTR respectively, i.e. such that the 3'-UTR moiety and/or the 5'-UTR moiety is the 3'-UTR and/or the 5'-UTR, respectively, of the artificial (optimized) nucleic acid molecule or the vector, preferably the mRNA, i.e., such that the 5'-UTR ends immediately before the 5'-end of the ORF and the 3'-UTR extends from the 3'-side of the open reading frame to the 5'-side of a poly(A) sequence or a polyadenylation signal, optionally connected via a short linker, such as a sequence comprising or consisting of one or more restriction sites. Thus, preferably, the terms "associating the artificial nucleic acid molecule or the vector with a 3'-UTR moiety and/or a 5'-UTR moiety" or associating the optimized nucleic acid molecule or the vector with a 3'-UTR moiety and/or a 5'-UTR moiety" mean functionally associating the 3'-UTR moiety and/or the 5'-UTR moiety with an open reading frame located within the artificial (optimized) nucleic acid molecule or the vector, preferably within the mRNA molecule. Thereby, further optimization may be achieved. The association with a 3'-UTR moiety and/or a 5'-UTR moiety can either be achieved by *de novo* association of individual moieties, or by modifying a pre-existing nucleic acid (template). Thus, the present invention comprises a method of associating an open reading frame (ORF) encoding a polypeptide or protein of interest and optional further element(s) with a 3'-UTR moiety and/or with a 5'-UTR moiety.

It is particularly preferred that the mRNA molecule comprises both (i) at least one preferred 5'-UTR and (ii) at least one preferred 3'-UTR, each as described herein. Furthermore, the mRNA molecule according to the present invention may comprise more than one 3'-UTR moieties and/or more than one 5'-UTR moieties as described herein. For example, the mRNA molecule according to the present invention may comprise one, two, three, four or more 3'-UTR moieties, and/or one, two, three, four or more 5'-UTR moieties, wherein the individual 3'-UTR moieties may be the same or they may be different, and similarly, the individual 5'-UTR moieties may be the same or they may be different. For example, the mRNA molecule according to the present invention may comprise two essentially identical 3'-UTR moieties. Accordingly, for example, the mRNA molecule according to the present invention may comprise two essentially identical 5'-UTR moieties.

The term "3'-UTR moiety" refers to a nucleic acid sequence which comprises or consists of a nucleic acid sequence that is derived from a 3'-UTR or from a variant or a fragment of a 3'-UTR. A "3'-UTR moiety" preferably refers to a nucleic acid sequence which is comprised by a 3'-UTR of an optimized nucleic acid sequence, such as an optimized mRNA, e.g. as claimed. Accordingly, in the sense of the present invention, preferably, a 3'-UTR moiety may be comprised by the 3'-UTR of an mRNA, preferably of an optimized mRNA, e.g. as claimed, or a 3'-UTR moiety may be comprised by the 3'-UTR of the respective transcription template. Preferably, a 3'-UTR moiety is a nucleic acid sequence which corresponds to the 3'-UTR of an mRNA, preferably to the 3'-UTR of an optimized mRNA, such as an mRNA obtained by transcription of a genetically engineered vector construct. Preferably, a 3'-UTR moiety in the sense of the present invention functions as a 3'-UTR or codes for a nucleotide sequence that fulfils the function of a 3'-UTR.

Accordingly, the term "5'-UTR moiety" refers to a nucleic acid sequence which comprises or consists of a nucleic acid sequence that is derived from a 5'-UTR or from a variant or a fragment of a 5'-UTR. A "5'-UTR moiety" preferably refers to a nucleic acid sequence which is comprised by a 5'-UTR of an optimized nucleic acid sequence, such as an optimized mRNA, e.g. as claimed. Accordingly, in the sense of the present invention, preferably, a 5'-UTR moiety may be comprised by the 5'-UTR of an mRNA, preferably of an optimized mRNA, e.g. as claimed, or a 5'-UTR moiety may be comprised by the 5'-UTR of the respective transcription template. Preferably, a 5'-UTR moiety is a nucleic acid sequence which corresponds to the 5'-UTR of an mRNA, preferably to the 5'-UTR of an optimized mRNA, such as an mRNA obtained by transcription of a genetically engineered vector construct. Preferably, a 5'-UTR moiety in the sense of the present invention functions as a 5'-UTR or codes for a nucleotide sequence that fulfils the function of a 5'-UTR.

The 3'-UTR moiety and/or the 5'-UTR moiety in the mRNA molecule according to the present invention provides one or more beneficial UTR property to said mRNA molecule. Thus, the mRNA molecule according to the present invention may in particular comprise:
- a 3'-UTR moiety which provides one or more beneficial UTR property to said mRNA molecule,
- a 5'-UTR moiety which provides one or more beneficial UTR property to said mRNA molecule,
- a 3'-UTR moiety which provides one or more beneficial UTR property to said optimized nucleic acid molecule and a 5'-UTR moiety which provides one or more beneficial UTR property to said mRNA molecule.

As described in detail below, said at least one 3'-UTR moiety which provides one or more beneficial UTR property to said mRNA molecule or said at least one 5'-UTR moiety which provides one or more beneficial UTR property to said mRNA molecule can be selected from naturally occurring (preferably heterologous) 3'-UTR moieties and 5'-UTR moieties (together naturally occurring UTR moieties or wild-type UTR moieties), and from optimized 3'-UTR moieties and optimized 5'-UTR moieties (together optimized UTR moieties). Wild-type UTR moieties can be selected from the group comprising wild-type UTR moieties published in the literature and in publically accessible databases, such as GenBank (NCBI), and wild-type UTR moieties not previously published. The latter can be identified by sequencing mRNAs found in cells, preferably mammalian cells. Using this approach, the present inventors identified several wild-type UTR moieties not previously published, and UTR moieties of this type are provided in the present invention. The term artificial UTR moiety is not particularly limited and can refer to any nucleic acid sequence not found in nature, i.e. nonidentical to a wild-type UTR moiety. In preferred embodiments, however, the artificial UTR moiety used in the present invention is a ribonucleic acid sequence which shows a certain degree of sequence identity to a wild-type UTR moiety, such as 10 to 99.9 %, 20 to 99 %, 30 to 98 %, 40 to 97 %, 50 to 96 %, 60 to 95 %, 70 to 90 %. In preferred embodiments, artificial UTR moiety used in the present invention is identical to a wild-type UTR moiety, except that one, or two, or three, or four, or five, or more than five nucleotides have been substituted by the same number of nucleotides (e.g. one nucleotide being substituted by one nucleotide). Preferably, substitution of one nucleotide is a substitution by the respective complementary nucleotide. Preferred artificial UTR moieties correspond to wild-type UTR moieties, except that (i) some or all ATG triplets in a wild-type 5'-UTR moiety (if present) are converted to the triplet TAG; and/or (ii) selected cleavage site(s) for a particular restriction enzyme in a wild-type 5'-UTR moiety or in a in a wild-type 3'-UTR moiety (if present) are eliminated by substituting one nucleotide within the cleavage site for said specific restriction enzyme by the complementary nucleotide, thereby removing the cleavage sites for said specific restriction enzyme. The latter is usually desired when a (e.g. wild-type) UTR moiety comprises a cleavage site for said specific restriction enzyme, and when said particular restriction enzyme is (planned to be) used in subsequent cloning steps. Since such internal cleavage of 5'-UTR moieties and 3'-UTR moieties is undesired, an artificial UTR moiety can be generated in which the restriction cleavage site for said specific restriction enzyme is eliminated. Such substitution can be done by any suitable method known to the person skilled in the art, e.g. use of modified primers by PCR.

Preferably, the mRNA molecule according to the present invention comprises a 3'-UTR moiety which provides one or more beneficial UTR property to said mRNA molecule and/or a 5'-UTR moiety which provides one or more beneficial UTR property to said mRNA molecule.

The mRNA molecule according to the present invention comprises at least one 3'-UTR moiety and at least one 5'-UTR moiety, i.e. at least one 3'-UTR moiety which provides one or more beneficial UTR property to said optimized nucleic acid molecule and at least one 5'-UTR moiety which provides one or more beneficial UTR property to said optimized nucleic acid molecule.

Specific useful UTRs useful for the present invention may be selected from the specific 5'-UTRs and the specific 3'-UTRs described in the following.

### 3.2.1.1 5'-UTRs

Specific 5'-UTRs useful in the context of the present invention may be selected from the ones described in the following.

In some embodiments, the 5'-UTR moiety used in the present invention differs from a wild-type 5'-UTR moiety. Such 5'-UTR moieties are designated "artificial 5'-UTR moieties". Typically the artificial 5'-UTR moiety differs from the wild-type 5'-UTR moiety it is based on in that at least one nucleotide, such as two nucleotides, three nucleotides, four nucleotides, five nucleotides, six nucleotides, seven nucleotides, eight nucleotides, nine nucleotides, ten nucleotides, or more than ten nucleotides, is/are exchanged. For example, such a nucleotide exchange may be recommendable in case the wild-type 5'-UTR moiety comprises a nucleotide moiety which is considered disadvantageous. For example, in some embodiments a nucleotide moiety which is considered disadvantageous is selected from (i) an internal ATG triplet (i.e. an ATG triplet other than the start codon of the open reading frame of the nucleic acid of the invention) or (ii) a restriction enzyme recognition site (cleavage site), particularly the restriction enzyme recognition site (cleavage site) which is recognized (cleavable) by a restriction enzyme used in the process of making (cloning) the optimized nucleic acid of the present invention. Hence, it is possible to specifically introduce certain base(s) (in exchange for the respective wild-type base(s)), so that the artificial 5'-UTR moiety does not contain a nucleotide moiety which is considered disadvantageous.

Optionally, the 5'-UTR comprises or consists of a nucleic acid sequence which is derived from the 5'-UTR of a TOP gene or which is derived from a fragment, homolog or variant of the 5'-UTR of a TOP gene.

The nucleic acid sequence which is derived from the 5'-UTR of a TOP gene is derived from a eukaryotic TOP gene, preferably a plant or animal TOP gene, more preferably a chordate TOP gene, even more preferably a vertebrate TOP gene, most preferably a mammalian TOP gene, such as a human TOP gene.

For example, the 5'-UTR is preferably selected from 5'-UTR moieties comprising or consisting of a nucleic acid sequence which is derived from a nucleic acid sequence selected from the group consisting of SEQ ID NOs. 1-1363, SEQ ID NO. 1395, SEQ ID NO. 1421 and SEQ ID NO. 1422 of the patent application WO2013/143700, from the homologs of SEQ ID NOs. 1-1363, SEQ ID NO. 1395, SEQ ID NO. 1421 and SEQ ID NO. 1422 of the patent application WO2013/143700, from a variant thereof, or preferably from a corresponding RNA sequence. The term "homologs of SEQ ID NOs. 1-1363, SEQ ID NO. 1395, SEQ ID NO. 1421 and SEQ ID NO. 1422 of the patent application WO2013/143700" refers to sequences of other species than *Homo sapiens,* which are homologous to the sequences according to SEQ ID NOs. 1-1363, SEQ ID NO. 1395, SEQ ID NO. 1421 and SEQ ID NO. 1422 of the patent application WO2013/143700.

Optionally, the 5'-UTR comprises or consists of a nucleic acid sequence which is derived from a nucleic acid sequence extending from nucleotide position 5 (i.e. the nucleotide that is located at position 5 in the sequence) to the nucleotide position immediately 5' to the start codon (located at the 3' end of the sequences), e.g. the nucleotide position immediately 5' to the ATG sequence, of a nucleic acid sequence selected from SEQ ID NOs. 1-1363, SEQ ID NO. 1395, SEQ ID NO. 1421 and SEQ ID NO. 1422 of the patent application WO2013/143700, from the homologs of SEQ ID NOs. 1-1363, SEQ ID NO. 1395, SEQ ID NO. 1421 and SEQ ID NO. 1422 of the patent application WO2013/143700, from a variant thereof, or a corresponding RNA sequence. It is particularly preferred that the 5'-UTR is derived from a nucleic acid sequence extending from the nucleotide position immediately 3' to the 5'TOP to the nucleotide position immediately 5' to the start codon (located at the 3' end of the sequences), e.g. the nucleotide position immediately 5' to the ATG sequence, of a nucleic acid sequence selected from SEQ ID NOs. 1-1363, SEQ ID NO. 1395, SEQ ID NO. 1421 and SEQ ID NO. 1422 of the patent application WO2013/143700, from the homologs of SEQ ID NOs. 1-1363, SEQ ID NO. 1395, SEQ ID NO. 1421 and SEQ ID NO. 1422 of the patent application WO2013/143700, from a variant thereof, or a corresponding RNA sequence.

In a particularly preferred embodiment, the further 5'-UTR comprises or consists of a nucleic acid sequence which is derived from a 5'-UTR of a TOP gene encoding a ribosomal protein or from a variant of a 5'-UTR of a TOP gene encoding a ribosomal protein. For example, the 5'-UTR moiety comprises or consists of a nucleic acid sequence which is derived from a 5'-UTR of a nucleic acid sequence according to any of SEQ ID NOs: 170, 232, 244, 259, 1284, 1285, 1286, 1287, 1288, 1289, 1290, 1291, 1292, 1293, 1294, 1295, 1296, 1297, 1298, 1299, 1300, 1301, 1302, 1303, 1304, 1305, 1306, 1307, 1308, 1309, 1310, 1311, 1312, 1313, 1314, 1315, 1316, 1317, 1318, 1319, 1320, 1321, 1322, 1323, 1324, 1325, 1326, 1327, 1328, 1329, 1330, 1331, 1332, 1333, 1334, 1335, 1336, 1337, 1338, 1339, 1340, 1341, 1342, 1343, 1344, 1346, 1347, 1348, 1349, 1350, 1351, 1352, 1353, 1354, 1355, 1356, 1357, 1358, 1359, or 1360 of the patent application WO2013/143700, a corresponding RNA sequence, a homolog thereof, or a variant thereof as described herein, optionally lacking the 5'-TOP motif. As described above, the sequence extending from position 5 to the nucleotide immediately 5' to the ATG (which is located at the 3'end of the sequences) corresponds to the 5'-UTR of said sequences.

Optionally, the 5'-UTR comprises or consists of a nucleic acid sequence which is derived from a 5'-UTR of a TOP gene encoding a ribosomal Large protein (RPL) or from a homolog or variant of a 5'-UTR of a TOP gene encoding a ribosomal Large protein (RPL). For example, the 5'-UTR moiety comprises or consists of a nucleic acid sequence which is derived from a 5'-UTR of a nucleic acid sequence according to any of SEQ ID NOs: SEQ ID NOs: 67, 259, 1284-1318, 1344, 1346, 1348-1354, 1357, 1358, 1421 and 1422 of the patent application WO2013/143700, a corresponding RNA sequence, a homolog thereof, or a variant thereof as described herein, optionally lacking the 5'TOP motif.

Optionally, the 5'-UTR moiety comprises or consists of a nucleic acid sequence which is derived from the 5'-UTR of a ribosomal protein Large 32 gene, preferably from a vertebrate ribosomal protein Large 32 (L32) gene, more preferably from a mammalian ribosomal protein Large 32 (L32) gene, most preferably from a human ribosomal protein Large 32 (L32) gene, or from a variant of the 5'-UTR of a ribosomal protein Large 32 gene, preferably from a vertebrate ribosomal protein Large 32 (L32) gene, more preferably from a mammalian ribosomal protein Large 32 (L32) gene, most preferably from a human ribosomal protein Large 32 (L32) gene, wherein preferably the further 5'-UTR does not comprise the 5'TOP of said gene.

Accordingly, the 5'-UTR moiety can comprise or consist of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO: 1804 (5'-UTR of human ribosomal protein Large 32 lacking the 5' terminal oligopyrimidine tract; corresponding to SEQ ID NO. 1368 of the patent application WO2013/143700) or to a corresponding RNA sequence, or wherein the 5'-UTR moiety comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO: 1804 or more preferably to a corresponding RNA sequence. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

In some embodiments, the mRNA molecule comprises a 5'-UTR which comprises or consists of a nucleic acid sequence which is derived from the 5'-UTR of a vertebrate TOP gene, such as a mammalian, e.g. a human TOP gene, selected from RPSA, RPS2, RPS3, RPS3A, RPS4, RPS5, RPS6, RPS7, RPS8, RPS9, RPS10, RPS11, RPS12, RPS13, RPS14, RPS15, RPS15A, RPS16, RPS17, RPS18, RPS19, RPS20, RPS21, RPS23, RPS24, RPS25, RPS26, RPS27, RPS27A, RPS28, RPS29, RPS30, RPL3, RPL4, RPL5, RPL6, RPL7, RPL7A, RPL8, RPL9, RPL10, RPL10A, RPL11, RPL12, RPL13, RPL13A, RPL14, RPL15, RPL17, RPL18, RPL18A, RPL19, RPL21, RPL22, RPL23, RPL23A, RPL24, RPL26, RPL27, RPL27A, RPL28, RPL29, RPL30, RPL31, RPL32, RPL34, RPL35, RPL35A, RPL36, RPL36A, RPL37, RPL37A, RPL38, RPL39, RPL40, RPL41, RPLP0, RPLP1, RPLP2, RPLP3, RPLP0, RPLP1, RPLP2, EEF1A1, EEF1B2, EEF1D, EEF1G, EEF2, EIF3E, EIF3F, EIF3H, EIF2S3, EIF3C, EIF3K, EIF3EIP, EIF4A2, PABPC1, HNRNPA1, TPT1, TUBB1, UBA52, NPM1, ATP5G2, GNB2L1, NME2, UQCRB or from a homolog or variant thereof, wherein preferably the further 5'-UTR does not comprise a TOP-motif or the 5'TOP of said genes, and wherein optionally the further 5'-UTR starts at its 5'-end with a nucleotide located at position 1, 2, 3, 4, 5, 6, 7, 8, 9 or 10 downstream of the 5'terminal oligopyrimidine tract (TOP) and wherein further optionally the further 5'-UTR which is derived from a 5'-UTR of a TOP gene terminates at its 3'-end with a nucleotide located at position 1, 2, 3, 4, 5, 6, 7, 8, 9 or 10 upstream of the start codon (A(U/T)G) of the gene it is derived from.

Alternatively, any polynucleotide moiety may be selected which is characterized by at least 80 % identity, at least 85 % identity, preferably at least 90 % identity, and more preferably at least 95 % identity to any of the above-described 5'-UTR sequences.

### 3.2.1.2 3'-UTRs

Specific 3'-UTRs useful in the context of the present invention may be selected from the ones described in the following.

The 3'-UTR can comprise or consist of a nucleic acid sequence which is derived from a 3'-UTR of a gene selected from the group consisting of an albumin gene, an α-globin gene, a β-globin gene, a tyrosine hydroxylase gene, a lipoxygenase gene, and a collagen alpha gene, such as a collagen alpha 1(I) gene, or from a variant of a 3'-UTR of a gene selected from the group consisting of an albumin gene, an α-globin gene, a β-globin gene, a tyrosine hydroxylase gene, a lipoxygenase gene, and a collagen alpha gene, such as a collagen alpha 1(I) gene according to SEQ ID NO: 1369-1390 of the patent application WO2013/ 43700.

In this context the nucleic acid molecule of the present invention can comprises a 3'-UTR moiety derived from the nucleic acids according to SEQ ID NO. 1369-1390 of the patent application WO2013/143700 or a fragment, homolog or variant thereof.

In a particularly preferred embodiment, the further 3'-UTR comprises or consists of a nucleic acid sequence which is derived from a 3'-UTR of an albumin gene, preferably a vertebrate albumin gene, more preferably a mammalian albumin gene, most preferably a human albumin gene according to SEQ ID NO: 1728 (Human albumin 3'-UTR; corresponding to SEQ ID NO: 1369 of the patent application WO2013/143700).

The 3'-UTR may comprise a nucleic acid sequence derived from a fragment of the human albumin gene according to SEQ ID NO: 1729 (albumin7 3'-UTR; corresponding to SEQ ID NO: 1376 of the patent application WO2013/143700).

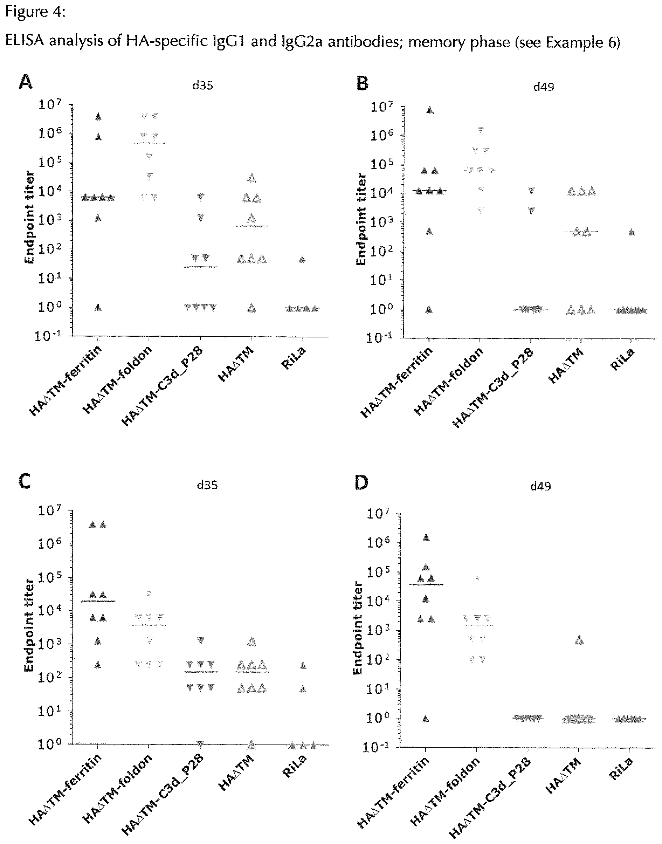

Thus, in embodiments, the 3'-UTR of the mRNA molecule comprises or consists of the corresponding RNA sequence of a nucleic acid sequence according to SEQ ID NO: 1729. The 3'-UTR may also comprise or consist of a nucleic acid sequence derived from a ribosomal protein coding gene, whereby ribosomal protein coding genes from which a further 3'-UTR may be derived include, but are not limited to, ribosomal protein L9 (RPL9), ribosomal protein L3 (RPL3), ribosomal protein L4 (RPL4), ribosomal protein L5 (RPL5), ribosomal protein L6 (RPL6), ribosomal protein L7 (RPL7), ribosomal protein L7a (RPL7A), ribosomal protein L11 (RPL11), ribosomal protein L12 (RPL12), ribosomal protein L13 (RPL13), ribosomal protein L23 (RPL23), ribosomal protein L18 (RPL18), ribosomal protein L18a (RPL18A), ribosomal protein L19 (RPL19), ribosomal protein L21 (RPL21), ribosomal protein L22 (RPL22), ribosomal protein L23a (RPL23A), ribosomal protein L17 (RPL17), ribosomal protein L24 (RPL24), ribosomal protein L26 (RPL26), ribosomal protein L27 (RPL27), ribosomal protein L30 (RPL30), ribosomal protein L27a (RPL27A), ribosomal protein L28 (RPL28), ribosomal protein L29 (RPL29), ribosomal protein L31 (RPL31), ribosomal protein L32 (RPL32), ribosomal protein L35a (RPL35A), ribosomal protein L37 (RPL37), ribosomal protein L37a (RPL37A), ribosomal protein L38 (RPL38), ribosomal protein L39 (RPL39), ribosomal protein, large, P0 (RPLP0), ribosomal protein, large, P1 (RPLP1), ribosomal protein, large, P2 (RPLP2), ribosomal protein S3 (RPS3), ribosomal protein S3A (RPS3A), ribosomal protein S4, X-linked (RPS4X), ribosomal protein S4, Y-linked 1 (RPS4Y1), ribosomal protein S5 (RPS5), ribosomal protein S6 (RPS6), ribosomal protein S7 (RPS7), ribosomal protein S8 (RPS8), ribosomal protein S9 (RPS9), ribosomal protein S10 (RPS10), ribosomal protein S11 (RPS11), ribosomal protein S12 (RPS12), ribosomal protein S13 (RPS13), ribosomal protein S15 (RPS15), ribosomal protein S15a (RPS15A), ribosomal protein S16 (RPS16), ribosomal protein S19 (RPS19), ribosomal protein S20 (RPS20), ribosomal protein S21 (RPS21), ribosomal protein S23 (RPS23), ribosomal protein S25 (RPS25), ribosomal protein S26 (RPS26), ribosomal protein S27 (RPS27), ribosomal protein S27a (RPS27a), ribosomal protein S28 (RPS28), ribosomal protein S29 (RPS29), ribosomal protein L15 (RPL15), ribosomal protein S2 (RPS2), ribosomal protein L14 (RPL14), ribosomal protein S14 (RPS14), ribosomal protein L10 (RPL10), ribosomal protein L10a (RPL10A), ribosomal protein L35 (RPL35), ribosomal protein L13a (RPL13A), ribosomal protein L36 (RPL36), ribosomal protein L36a (RPL36A), ribosomal protein L41 (RPL41), ribosomal protein S18 (RPS18), ribosomal protein S24 (RPS24), ribosomal protein L8 (RPL8), ribosomal protein L34 (RPL34), ribosomal protein S17 (RPS17), ribosomal protein SA (RPSA), ubiquitin A-52 residue ribosomal protein fusion product 1 (UBA52), Finkel-Biskis-Reilly murine sarcoma virus (FBR-MuSV) ubiquitously expressed (FAU), ribosomal protein L22-like 1 (RPL22L1), ribosomal protein S17 (RPS17), ribosomal protein L39-like (RPL39L), ribosomal protein L10-like (RPL10L), ribosomal protein L36a-like (RPL36AL), ribosomal protein L3-like (RPL3L), ribosomal protein S27-like (RPS27L), ribosomal protein L26-like 1 (RPL26L1), ribosomal protein L7-like 1 (RPL7L1), ribosomal protein L13a pseudogene (RPL13AP), ribosomal protein L37a pseudogene 8 (RPL37AP8), ribosomal protein S10 pseudogene 5 (RPS10P5), ribosomal protein S26 pseudogene 11 (RPS26P11), ribosomal protein L39 pseudogene 5 (RPL39P5), ribosomal protein, large, P0 pseudogene 6 (RPLP0P6) and ribosomal protein L36 pseudogene 14 (RPL36P14). Preferably the 3'-UTR comprises or consists of a nucleic acid sequence according to any one of SEQ ID NOs: 10 to 205 of WO2015/101414. Preferably, the at least one 3'-untranslated region element (3'-UTR element) comprises or consists of a nucleic acid sequence which is derived from the 3'-UTR of a FIG4 gene or from a variant of the 3'-UTR of a FIG4 gene. The term "a FIG4 gene" generally refers to a gene encoding FIG4, which is also known as, for instance, Sac Domain-Containing Inositol Phosphatase 3, SAC3, S. cerevisiae Homolog of Fig4 (see, for instance, Minagawa et al., 2001. Identification and Characterization of a Sac Domain-containing Phosphoinositide 5-Phosphatase, J. Biol. Chem., Vol. 276, p. 22011-22015; Takasuga and Sasaki, Phosphatidylinositol-3,5-biphosphate: metabolism and physiological functions, Journal of Biochemistry, Vol. 154, No. 3, 2013, p. 211-218). As used herein, the term "a FIG4 gene" refers to any FIG4 gene, irrespective of the species, from which it is derived. Specifically, the term refers to a mammalian FIG4 gene. Further, the term "a FIG4 gene" comprises any paralogs and orthologs of a mammalian FIG4 gene. Moreover, any sequence, which is characterized by substantial sequence similarity or identity is referred to as FIG4 gene in the context of the present invention. Fig4 genes and corresponding 3'-UTRs are disclosed in WO201S/101415.

In particular, any 3'-UTR of a FIG4 gene according to the disclosure of WO2015/101415 can be selected as a nucleic acid moiety according to the present invention. Preferred moieties are represented by SEQ ID NO: 1 and 2 of WO2015/101415.

The suitability of a particular UTR can be detected by comparison of expression of a gene carrying the particular UTR to expression of a housekeeping gene. Respective sequences are disclosed in Table 1 of WO_2007_068265. Respective sequences are also disclosed in WO2014164253A1. These respective sequences are incorporated herein.

Alternatively, any polynucleotide moiety may be selected which is characterized by at least 80 % identity, at least 85 % identity, preferably at least 90 % identity, and more preferably at least 95 % identity to any of the above-described 3'-UTR sequences.

### 3.2.2 miRNA moieties

A miRNA may also be selected as a moiety in the present invention. Any miRNA moiety known in the art may be selected. Such a moiety can be selected from microRNA target sequences, microRNA seqences, or microRNA seeds. For example, miRNA sequences (microRNA target sequences, microRNA seqences, or microRNA seeds) are described in WO 2015085318 A2, US 2005/0261218 and US2005/0059005. Identification of microRNA, microRNA target regions, and their expression patterns and role in biology have been reported (Bonauer et al., Curr Drug Targets 2010 11 :943-949; Anand and Cheresh, Curr Opin Hematol 2011, 18: 171-176; Contreras and Rao Leukemia, 2012 26:404-413; Barrel, Cell, 2009 136:215-233; Landgraf et al., Cell, 2007 129: 1401-1414.

In general, microRNAs (or miRNA) are 19-25 nucleotide long noncoding RNAs. miRNAs bind to 3'-UTR of nucleic acid molecules. This causes down-regulation of gene expression, either by reducing nucleic acid molecule stability or by inhibiting translation. As a module of the present invention, the polynucleotides of the present invention may comprise one or more microRNA target sequences, microRNA seqences, or microRNA seeds.

As used herein, the term "microRNA site" refers to a polynucleotide sequence to which a microRNA can bind or otherwise associate. "binding" typically occurs by Watson-Crick hybridization; but any otherwise stable association of the microRNA with the target sequence at or adjacent to the microRNA site is also comprised in the concept of a "microRNA site" according to the present invention.

In general, a microRNA sequence comprises a "seed" region, i.e., a sequence typically in the region of positions 2-8 of a mature microRNA. The seed region sequence has perfect Watson-Crick complementarity to the miRNA target sequence. Such a microRNA seed may comprise positions 2-8, or alternatively 2-7 of the mature microRNA. Thus, in one embodiment, a microRNA seed comprises 7 nucleotides (e.g., nucleotides 2-8 of the mature microRNA), wherein the seed-complementary site in the corresponding miRNA target is flanked by an adenine (A) opposed to microRNA position 1. In another embodiment, a microRNA seed comprises 6 nucleotides (e.g., nucleotides 2-7 of the mature microRNA), wherein the seed-complementary site in the corresponding miRNA target is flanked byan adenine (A) opposed to microRNA position 1. Respective nucleic acid modules are disclosed in Grimson et al.; Mol Cell. 2007 Jul 6;27(1):91-105

In the present invention, a microRNA target sequence is typically designed to be comprised in a 3'-UTR or otherwise 3' (upstream) of an open reading frame. In such case, the miRNA target sequence is thought to target the molecule for degradation or reduced translation, provided that a corresponding microRNA in question is available. This allows to control any undesired off-target effects upon delivery of the nucleic acid molecule of the present invention.

In case it is not desired to translate an mRNA in the liver, but the mRNA is transported to the liver or otherwise ends up there, then miR-122, a microRNA abundant in liver, can inhibit the expression of the nucleic acid of the present invention, if one or multiple target sites of miR-122 are present (e.g. designed) in the 3'-UTR region of the polynucleotide of the present inveniton. Introduction of one or multiple binding sites for different microRNA can be engineered to further influence (e.g. decrease) the longevity, stability, and protein translation of polynucleotides.

In contrast, in case it is indeed desired to translate an mRNA, microRNA binding sites can be engineered out of (i.e. removed from) sequences in which they occur, e.g., in order to increase protein expression in specific tissues. For example, one or more miR-122 binding sites may be removed to improve protein expression in the liver.

Thereby, regulation of expression in specific tissues can be accomplished through introduction or removal or one or several microRNA binding sites. For examples microRNAs are known to regulate mRNA, and thereby protein expression, without limitation in liver (miR-122), heart (miR-ld, miR-149), endothelial cells (miR- 17-92, miR-126), adipose tissue (let-7, miR-30c), kidney (miR-192, miR-194, miR-204), myeloid cells (miR-142-3p, miR-142-5p, miR-16, miR-21, miR-223, miR-24, miR-27),muscle (miR-133, miR-206, miR-208), and lung epithelial cells (let-7, miR-133, miR-126). MicroRNA can also regulate complex biological processes such as angiogenesis (miR-132) (e.g. Anand and Cheresh, Curr. Opin. Hematol. 2011, 18: 171-176;).

Thus, in general, according to the modular design principle of the present invention, binding sites for microRNAs may be removed or introduced, in order to tailor the expression of the polynucleotides expression to desired cell types or tissues, or to the context of relevant biological processes. Listings of miRNA sequences and binding sites areavailable to the public. Any sequence disclosed in the literature discussed herein may be used in the context of the present invention: examples of microRNA that drive tissue- or disease-specific gene expression are listed in Getner and Naldini, Tissue Antigens. 2012, 80:393-403. An example of incorporation of microRNA seed sites is incorporation of miR-142 sites into a UGT1A1-expressing lentiviral vector, which causes reduced expression in antigen-presentating cells, leading to the absence of an immune response against the virally expressed UGT1A1 as disclosed in Schmitt et al., Gastroenterology 2010; 139:999-1007; Gonzalez-Asequinolaza et al. Gastroenterology 2010, 139:726-729. Thus, incorporation of one or more miR-142 seed sites into mRNA is thought to be be important in the case of treatment of patients with complete protein deficiencies (UGT1A1 type I, LDLR-deficient patients, CRIM-negative Pompe patients, etc.). Thereby, the mRNA molecule of the present invention can be designed to fit such purposes.

Any polynucleotide may be selected which is characterized by at least 80 % identity, at least 85 % identity, preferably at least 90 % identity, and more preferably at least 95 % identity to any of such miRNA sequences.

Owing to the different expression patterns of microRNA in different cell types, the present invention allows to specifically design polynucleotide molecules for targeted expression in specific cell types, or under specific biological conditions. Through introduction of tissue-specific microRNA binding sites, polynucleotides can be designed that for protein expression in a tissue or in the context of a biological condition.

### 3.2.3 IRES moieties

An internal ribosome entry site, abbreviated IRES, is a nucleotide sequence that allows for translation initiation in the middle of a messenger RNA (mRNA) sequence as part of the greater process of protein synthesis. While translation in eukaryotes is usually initiated only at the 5' end of the mRNA molecule, presence of an IRES allows translation of the RNAs in a cap-independent manner. In nature, it is common that IRESes are located in the 5'UTR of RNA viruses. Presence of an IRES can allow for translation of two proteins from a single transcript (RNA): for such purposes, part of the present invention, an IRES is present downstream of the coding region of a first polypeptide element, but upstream of the coding region of a second polypeptide element on the same transcript. Translation of the first coding region is initiated at the normal 5' cap, and the translation of the second coding region at the IRES. In general, IRES allow the expression of multiple proteins from a single nucleic acid molecule.

In the present invention, an internal ribosome entry site (IRES) sequence or IRES-motif may separate several open reading frames, for example if the optimized nucleic acid molecule encodes for two or more peptides or proteins. An IRES-sequence may be particularly helpful if the optimized nucleic acid molecule is a bi- or multicistronic nucleic acid molecule.

When used in the context of the present invention, such IRES are particularly useful when present in a nucleic acidencoding at least two functional protein moieties, such as at least one polypeptide or protein of interest and at least one further polypeptide or protein element, preferably also selected from the list of coding moieties of the present invention. In that case, the IRES is typically located on the polynucleotide chain in between the coding region for the protein of interest and the coding region for the at least one further protein element, so that translation leads to two separate polypeptide molecules, at least one of them being a polypeptide or protein of interest.

For example, for expressing target proteins that are composed of several polypeptide chains, such as antibodies, it may be beneficial to provide coding information for both peptide chains on a single nucleic acid molecule, separated by an IRES.

Suitably, the nucleotide sequence of the IRES used in the present invention is selected from the following list of nucleotide sequences (SEQ ID NOs: 1566-1662).

Alternatively, any polynucleotide may be selected which is characterized by at least 80 % identity, at least 85 % identity, preferably at least 90 % identity, and more preferably at least 95 % identity to any of the IRES sequences SEQ ID NOs: 1566-1662.

### 3.2.4 Histone stem-loop

Preferably, the mRNA molecule may additionally comprise a histone stem-loop. A histone-stem-loop, if present, is preferably localized 3' (downstream) of a 3'UTR moiety, and upstream of a poly(A) sequence or polyadenylation signal (if present). Thus, an mRNA molecule according to the present invention may, for example, comprise in 5'-to-3'-direction an ORF encoding a polypeptide of interest and optionally further element(s), a 3'-UTR moiety, an optional histone stem-loop sequence, an optional poly(A) sequence or polyadenylation signal and an optional poly(C) sequence. In another example, the optimized nucleic acid molecule according to the present invention may, for example, comprise in 5'-to-3'-direction an 5'-UTR moiety, an ORF encoding a polypeptide of interest and optionally further element(s), an optional histone stem-loop sequence, an optional poly(A) sequence or polyadenylation signal and an optional poly(C) sequence. In another example, the optimized nucleic acid molecule according to the present invention may, for example, comprise in 5'-to-3'-direction an 5'-UTR moiety, an ORF encoding a polypeptide of interest and optionally further element(s), a 3'-UTR moiety, an optional histone stem-loop sequence, an optional poly(A) sequence or polyadenylation signal and an optional poly(C) sequence. It may also comprise in 5'-to-3'-direction an ORF, an 3'-UTR moiety, an optional poly(A) sequence, an optional poly (C) sequence and an optional histone stem-loop sequence, or in 5'-to-3'-direction an 5'-UTR moiety, an ORF, an optional poly(A) sequence, an optional poly(C) sequence and an optional histone stem-loop sequence, or in 5'-to-3'-direction an 5'-UTR element, an ORF, a 3'-UTR element, an optional poly(A) sequence, an optional poly(C) sequence and an optional histone stem-loop sequence.

In a preferred embodiment, the mRNA molecule according to the invention further comprises at least one histone stem-loop sequence.

Such histone stem-loop sequences are preferably selected from histone stem-loop sequences as disclosed in WO 2012/01 9780.

Alternatively, such histone stem-loop sequences are preferably selected from histone stem-loop sequences as disclosed in WO 2013/143699.

A histone stem-loop sequence, suitable to be used within the present invention, is preferably selected from at least one of the following formulae (I) or (II):
formula (I) (stem-loop sequence without stem bordering elements): 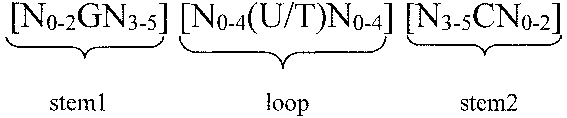
formula (II) (stem-loop sequence with stem bordering elements): 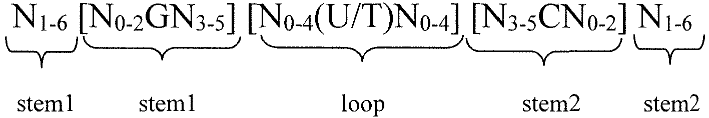 bordering element bordering element wherein:
   - stem1 or stem2 bordering elements N₁₋₆: is a consecutive sequence of 1 to 6, preferably of 2 to 6, more preferably of 2 to 5, even more preferably of 3 to 5, most preferably of 4 to 5 or 5 N, wherein each N is independently from another selected from a nucleotide selected from A, U, T, G and C, or a nucleotide analogue thereof;
   - stem1 [N₀₋₂GN₃₋₅]: is reverse complementary or partially reverse complementary with element stem2, and is a consecutive sequence between of 5 to 7 nucleotides; wherein N₀₋₂ is a consecutive sequence of 0 to 2, preferably of 0 to 1, more preferably of 1 N, wherein each N is independently from another selected from a nucleotide selected from A, U, T, G and C or a nucleotide analogue thereof; wherein N₃₋₅ is a consecutive sequence of 3 to 5, preferably of 4 to 5, more preferably of 4 N, wherein each N is independently from another selected from a nucleotide selected from A, U, T, G and C or a nucleotide analogue thereof, and wherein G is guanosine or an analogue thereof, and may be optionally replaced by a cytidine or an analogue thereof, provided that its complementary nucleotide cytidine in stem2 is replaced by guanosine;
   - loop sequence [N₀₋₄(U/T)N₀₋₄]: is located between elements stem1 and stem2, and is a consecutive sequence of 3 to 5 nucleotides, more preferably of 4 nucleotides; wherein each N₀₋₄ is independent from another a consecutive sequence of 0 to 4, preferably of 1 to 3, more preferably of 1 to 2 N, wherein each N is independently from another selected from a nucleotide selected from A, U, T, G and C or a nucleotide analogue thereof; and wherein U/T represents uridine, or optionally thymidine;
   - stem2 [N₃₋₅CN₀₋₂]: is reverse complementary or partially reverse complementary with element stem1, and is a consecutive sequence between of 5 to 7 nucleotides; wherein N₃₋₅ is a consecutive sequence of 3 to 5, preferably of 4 to 5, more preferably of 4 N, wherein each N is independently from another selected from a nucleotide selected from A, U, T, G and C or a nucleotide analogue thereof; wherein N₀₋₂ is a consecutive sequence of 0 to 2, preferably of 0 to 1, more preferably of 1 N, wherein each N is independently from another selected from a nucleotide selected from A, U, T, G or C or a nucleotide analogue thereof; and wherein C is cytidine or an analogue thereof, and may be optionally replaced by a guanosine or an analogue thereof provided that its complementary nucleoside guanosine in stem1 is replaced by cytidine;
   wherein
   stem1 and stem2 are capable of base pairing with each other forming a reverse complementary sequence, wherein base pairing may occur between stem1 and stem2, e.g. by Watson-Crick base pairing of nucleotides A and U/T or G and C or by non-Watson-Crick base pairing e.g. wobble base pairing, reverse Watson-Crick base pairing, Hoogsteen base pairing, reverse Hoogsteen base pairing or are capable of base pairing with each other forming a partially reverse complementary sequence, wherein an incomplete base pairing may occur between stem1 and stem2, on the basis that one or more bases in one stem do not have a complementary base in the reverse complementary sequence of the other stem.

According to a further preferred embodiment, the histone stem-loop sequence may be selected according to at least one of the following specific formulae (Ia) or (IIa):
formula (la) (stem-loop sequence without stem bordering elements): 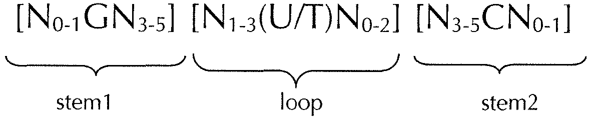
formula (Ila) (stem-loop sequence with stem bordering elements): 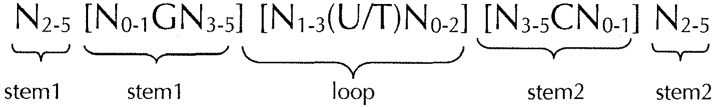
bordering element bordering element
wherein:
   - N, C, G, T and U: are as defined above.

According to a further more particularly preferred embodiment of the first aspect, the optimized nucleic acid molecule sequence may comprise at least one histone stem-loop sequence according to at least one of the following specific formulae (Ib) or (IIb):
formula (Ib) (stem-loop sequence without stem bordering elements): 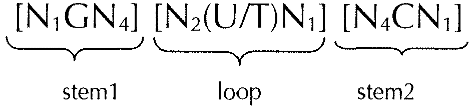
formula (IIb) (stem-loop sequence with stem bordering elements): 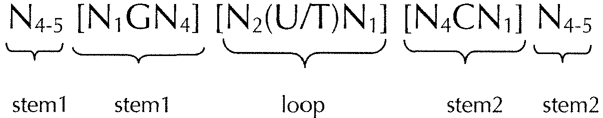 bordering element bordering element
wherein:
   - N, C, G, T and U: are as defined above.

A particular preferred histone stem-loop sequence is the corresponding RNA sequence sequence according to SEQ ID NO: 1731, or more preferably.

### 3.2.5 Cap

The optimized nucleic acid molecule according to the present invention may further comprise optionally a 5'-cap. The optional 5'-cap is preferably located 5' to the ORF, more preferably 5' to the at least one 5'-UTR (if present) within the optimized nucleic acid molecule according to the present invention. This embodiment is particularly useful when the nucleic acid molecule is an RNA molecule. A 5'-cap may be added in a cell, or may alternatively be added *in vitro.* Further details of a 5'-cap useful in the present invention are described below in the context of chemical modifications.

### 3.2.6 Polyadenylation signal or poly(A) sequence

Preferably, the optimized nucleic acid molecule according to the present invention further comprises a poly(A) sequence and/or a polyadenylation signal. A poly(A) sequence is particularly useful when the nucleic acid molecule is an RNA molecule, and is preferably present in an RNA molecule comprising a 3'-UTR. Preferably, the poly(A) sequence is located 3' to the 3'-UTR moiety, more preferably the poly(A) sequence is connected to the 3'-end of a 3'-UTR moiety. The connection may be direct or indirect, for example, via a stretch of 2, 4, 6, 8, 10, 20 etc. nucleotides, such as via a linker of 1-50, preferably of 1-20 nucleotides, e.g. comprising or consisting of one or more restriction sites. However, even if the optimized nucleic acid molecule according to the present invention does not comprise a 3'-UTR, for example if it only comprises at least one 5'-UTR moiety, it preferably still comprises a poly(A) sequence and/or a polyadenylation signal.

In one embodiment, a DNA molecule comprising an ORF, optionally followed by a 3' UTR, may contain a stretch of thymidine nucleotides which can be transcribed into a poly(A) sequence in the resulting mRNA. The length of the poly(A) sequence may vary. For example, the poly(A) sequence may have a length of about 20 adenine nucleotides up to about 300 adenine nucleotides, preferably of about 40 to about 200 adenine nucleotides, more preferably from about 50 to about 100 adenine nucleotides, such as about 60, 70, 80, 90 or 100 adenine nucleotides. Most preferably, the nucleic acid of the invention comprises a poly(A) sequence of about 60 to about 70 nucleotides, most preferably 64 adenine nucleotides.

In one embodiment, the optional polyadenylation signal is located downstream of the 3' of the 3'-UTR moiety. Preferably, the polyadenylation signal comprises the consensus sequence NN(U/T)ANA, with N = A or U, preferably AA(U/T)AAA or A(U/T)(U/T)AAA. Such consensus sequence may be recognised by most animal and bacterial cell-systems, for example by the polyadenylation-factors, such as cleavage/polyadenylation specificity factor (CPSF) cooperating with CstF, PAP, PAB2, CFI and/or CFII. Preferably, the polyadenylation signal, preferably the consensus sequence NNUANA, is located less than about 50 nucleotides, more preferably less than about 30 bases, most preferably less than about 25 bases, for example 21 bases, downstream of the 3'-end of the 3'-UTR moiety or of the ORF, if no 3'-UTR moiety is present.

Transcription of an optimized nucleic acid molecule according to the present invention, e.g. of an artificial DNA molecule, comprising a polyadenylation signal downstream of the 3'-UTR moiety (or of the ORF) will result in a premature-RNA containing the polyadenylation signal downstream of its 3'-UTR moiety (or of the ORF).

Using an appropriate transcription system will then lead to attachment of a poly(A) sequence to the premature-RNA. For example, the inventive optimized nucleic acid molecule may be a DNA molecule comprising a 3'-UTR moiety as described above and a polyadenylation signal, which may result in polyadenylation of an RNA upon transcription of this DNA molecule. Accordingly, a resulting RNA may comprise a combination of a 3'-UTR moiety and a poly(A) sequence.

Potential transcription systems are *in vitro* transcription systems or cellular transcription systems etc. Accordingly, transcription of an optimized nucleic acid molecule according to the invention, e.g. transcription of an optimized nucleic acid molecule comprising an open reading frame, a 3'-UTR moiety and/or a 5'-UTR moiety and optionally a polyadenylation-signal, may result in an mRNA molecule comprising an open reading frame, a 3'-UTR moiety and optionally a poly(A) sequence.

Accordingly, the invention also provides an optimized nucleic acid molecule, which is an mRNA molecule comprising an open reading frame, a 3'-UTR moiety as described above and/or a 5'-UTR moiety as described above and optionally a poly(A) sequence.

In another embodiment, the 3'-UTR of the optimized nucleic acid molecule according to the invention does not comprise a polyadenylation signal or a poly(A) sequence. Further preferably, the optimized nucleic acid molecule according to the invention does not comprise a polyadenylation signal or a poly(A) sequence. More preferably, the 3'-UTR of the optimized nucleic acid molecule, or the inventive optimized nucleic acid molecule as such, does not comprise a polyadenylation signal, in particular it does not comprise the polyadenylation signal AAU/TAAA.

### 3.2.7 Additional Modules

Furthermore, the mRNA molecule may comprise additional 5'-moieties, preferably a promoter or a promoter containing-sequence. The promoter may drive and or regulate transcription of the optimized nucleic acid molecule according to the present invention, for example of an artificial DNA-molecule according to the present invention.

Suitable promoters are known in the art.

Alternatively, any polynucleotide may be selected which is characterized by at least 80 % identity, at least 85 % identity, preferably at least 90 % identity, and more preferably at least 95 % identity to any of the promoter sequences.

### 3.2.8 Hairpin moieties:

The mRNA molecule according to the present invention may further comprise at least one hairpin moiety. Hairpin moieties can support RNA folding, protect mRNA from degradation, or serve as a recognition motif for RNA binding proteins etc. Hairpin moieties may be derived from naturally occurring hairpin structures (e.g., as present in UTR regions).

### 3.2.8 Moieties for RNA binding proteins:

The mRNA molecule according to the present invention may further comprise at least one moiety for RNA binding proteins. In essence, RNA-binding proteins (often abbreviated as RBPs) are proteins that bind to single stranded RNA in cells and participate in forming ribonucleoprotein complexes. RBPs contain various structural motifs, such as RNA recognition motif (RRM), dsRNA binding domain, zinc finger and others. RBPs have crucial roles in various cellular processes such as: cellular function, transport and localization. They especially play a major role in post-transcriptional control of RNAs, such as: splicing, polyadenylation, mRNA stabilization, mRNA localization and translation. Such moieties may be incorporated into the optimized nucleic acid to increase the translation rate of the construct into protein. Furthermore, moieties for RNA binding proteins may be introduced into the optimized nucleic acid to increase the cellular stability of the construct. Such optimized nucleic acids may have a prolonged half-life which may result in a prolonged protein expression *in vivo.*

### 3.2.8 Moiety that prevents 3'-5' degradation:

The mRNA molecule according to the present invention may further comprise a moiety that prevents 3'-5' degradation. Such moieties may comprise tailored oligonucleotides, potentially comprising modified nucleotides. Such moiety that prevents 3'-5' degradation may be incorporated into an optimized nucleic acid to increase the cellular stability of the construct. Such optimized nucleic acids may have a prolonged half-life which may result in a prolonged protein expression in vivo.

### 3.2.9 Moieties that regulate RNA decay rates:

The mRNA molecule according to the present invention may further comprise moieties that regulate RNA decay rates. For example, AU-rich elements located on the 3'UTR of mRNAs modulate mRNA stability, both as stabilizing and destabilizing elements. Elements that destabilize the optimized nucleic acid construct may be introduced into the RNA for application where a fast decay of the RNA is desired, e.g., when the expression of the encoded target protein has to be restricted. Elements that stabilize the optimized nucleic acid construct may be introduced into the RNA for application where a decay of the RNA is not desired, e.g., when the expression of the encoded target protein has to be prolonged.

### 4. Modifications

The mRNA molecule of the present invention may be modified in that a molecular entity (building block) found in a respective starting nucleic acid (e.g. wild-type nucleic acid) is replaced by a different molecular entity (building block). A building block or molecular entity may be a deoxyribonucleotide or ribonucleotide or non-naturally occurring nucleotide.

The replacing molecular entity may be a different - naturally occurring - deoxyribonucleotide or ribonucleotide, e.g. A, C, G, T, U. Embodiments of replacement by a different - but naturally occurring - molecular entity include G/C modification and codon optimization, each as described below.

Alternatively, the replacing molecular entity may be a synthetic entity. Embodiments include the chemical modifications described below.

### 4.1 Substitution by naturally occurring molecular entities

The replacing molecular entity (building block) may be a naturally occurring deoxyribonucleotide or ribonucleotide, so that one naturally occurring nucleotide is replaced by a different deoxyribonucleotide or ribonucleotide. Embodiments thereof include G/C modification and codon optimization:

### 4.1.1 G/C modification

The open reading frame or the mRNA molecule according to the present invention is or comprises a G/C modified nucleic acid sequence. The G/C content of the open reading frame of an mRNA molecule according to the present invention is increased compared to the G/C content of the open reading frame of a corresponding wild-type sequence, preferably by taking advantage of the degeneration of the genetic code. Thus, the amino acid sequence (polypeptide or protein) encoded by the mRNA molecule is preferably not altered, despite the G/C modification. The codons of the coding sequence or the whole mRNA may therefore be varied compared to the wild-type coding sequence, such that they include an increased amount of G/C nucleotides while the translated amino acid sequence is maintained. Due to the fact that several codons code for one and the same amino acid (so-called degeneration of the genetic code), it is feasible to alter codons while not altering the encoded peptide/protein sequence (so-called alternative codon usage). Hence, it is possible to specifically introduce certain codons (in exchange for the respective wild-type codons encoding the same amino acid), which are more favourable with respect to stability of RNA and/or with respect to codon usage in a subject (so-called codon optimization).

Depending on the amino acid to be encoded by the coding region of the mRNA molecule as defined herein, there are various possibilities for modification of the nucleic acid sequence, e.g. the open reading frame, compared to its wild-type coding region. In the case of amino acids, which are encoded by codons which contain exclusively G or C nucleotides, no modification of the codon is necessary. Thus, the codons for Pro (CCC or CCG), Arg (CGC or CGG), Ala (GCC or GCG) and Gly (GGC or GGG) require no modification, since no A or U/T is present.

In contrast, codons which contain A and/or U/T nucleotides may be modified by substitution of other codons which code for the same amino acids but contain no A and/or U/T. For example
the codons for Pro can be modified from CC(U/T) or CCA to CCC or CCG;
the codons for Arg can be modified from CG(U/T) or CGA or AGA or AGG to CGC or CGG;
the codons for Ala can be modified from GC(U/T) or GCA to GCC or GCG;
the codons for Gly can be modified from GG(U/T) or GGA to GGC or GGG.

In other cases, although A or (U/T) nucleotides cannot be eliminated from the codons, it is however possible to decrease the A and (U/T) content by using codons which contain a lower content of A and/or (U/T) nucleotides. Examples of these are:
The codons for Phe can be modified from (U/T)(U/T)(U/T) to (U/T) (U/T)C;
the codons for Leu can be modified from (U/T) (U/T)A, (U/T) (U/T)G, C(U/T) (U/T) or C(U/T)A to C(U/T)C or C(U/T)G;
the codons for Ser can be modified from (U/T)C(U/T) or (U/T)CA or AG(U/T) to (U/T)CC, (U/T)CG or AGC;
the codon for Tyr can be modified from (U/T)A(U/T) to (U/T)AC;
the codon for Cys can be modified from (U/T)G(U/T) to (U/T)GC;
the codon for His can be modified from CA(U/T) to CAC;
the codon for Gln can be modified from CAA to CAG;
the codons for Ile can be modified from A(U/T)(U/T) or A(U/T)A to A(U/T)C;
the codons for Thr can be modified from AC(U/T) or ACA to ACC or ACG;
the codon for Asn can be modified from AA(U/T) to AAC;
the codon for Lys can be modified from AAA to AAG;
the codons for Val can be modified from G(U/T)(U/T) or G(U/T)A to G(U/T)C or G(U/T)G;
the codon for Asp can be modified from GA(U/T) to GAC;
the codon for Glu can be modified from GAA to GAG;
the stop codon (U/T)AA can be modified to (U/T)AG or (U/T)GA.

In the case of the codons for Met (A(U/T)G) and Trp ((U/T)GG), on the other hand, there is no possibility of sequence modification without altering the encoded amino acid sequence.

The substitutions listed above can be used either individually or in all possible combinations to increase the G/C content of the open reading frame of the mRNA molecule of the invention as defined herein, compared to its particular wild-type open reading frame (i.e. the original sequence). Thus, for example, all codons for Thr occurring in the wild-type sequence can be modified to ACC (or ACG).

Preferably, the G/C content of the open reading frame of the mRNA molecule of the invention as defined herein is increased by at least 7%, more preferably by at least 15%, particularly preferably by at least 20%, compared to the G/C content of the wild-type coding region without altering the encoded amino acid sequence, i.e. using the degeneracy of the genetic code. According to a specific embodiment at least 5%, 10%, 20%, 30%, 40%, 50%, 60%, more preferably at least 70 %, even more preferably at least 80% and most preferably at least 90%, 95% or even 100% of the substitutable codons in the open reading frame of the optimized nucleic acid molecule or a fragment, variant or derivative thereof are substituted, thereby increasing the G/C content of said open reading frame.

In this context, it is particularly preferable to increase the G/C content of the open reading frame of the inventive mRNA molecule as defined herein, to the maximum (i.e. 100% of the substitutable codons), compared to the wild-type open reading frame, without altering the encoded amino acid sequence.

### 4.1.2 Adaptation of the codon-usage

Furthermore, the open reading frame is preferably at least partially codon-optimized. Codonoptimization is based on the finding that the translation efficiency may be determined by a different frequency in the occurrence of transfer RNAs (tRNAs) in cells. Thus, if so-called "rare codons" are present in the coding region of the mRNA molecule as defined herein, to an increased extent, the translation of the corresponding modified nucleic acid sequence is less efficient than in the case where codons coding for relatively "frequent" tRNAs are present.

Thus, the open reading frame of the mRNA molecule is preferably modified compared to the corresponding wild-type coding region such that at least one codon of the wild-type sequence which codes for a tRNA which is relatively rare in the cell is exchanged for a codon which codes for a tRNA which is comparably frequent in the cell and carries the same amino acid as the relatively rare tRNA. By this modification, the open reading frame of the mRNA molecule as defined herein, is modified such that codons for which frequently occurring tRNAs are available may replace codons which correspond to rare tRNAs. In other words, according to the invention, by such a modification all codons of the wild-type open reading frame which code for a rare tRNA may be exchanged for a codon which codes for a tRNA which is more frequent in the cell and which carries the same amino acid as the rare tRNA. Which tRNAs occur relatively frequently in the cell and which, in contrast, occur relatively rarely is known to a person skilled in the art; cf. e.g. Akashi, Curr. Opin. Genet. Dev. 2001, 11(6): 660-666. Accordingly, preferably, the open reading frame is codon-optimized, preferably with respect to the system in which the mRNA molecule according to the present invention is to be expressed, preferably with respect to the system in which the mRNA molecule according to the present invention is to be translated. Preferably, the codon usage of the open reading frame is codon-optimized according to mammalian codon usage, more preferably according to human codon usage. Preferably, the open reading frame is codon-optimized and G/C-content modified.

### 4.2 Chemical Modification

The polynucleotide of the present invention can comprise one or more chemical modification(s). The chemical modification is present as an alternative or in addition (preferably in addition) to the modules of the mRNA molecule as described above. Chemical modification, generally, refers to a modified nucleotide, so that a modified nucleotide is a structural feature of such mRNA molecule. In such case, at least one nucleotide of a nucleic acid molecule (e.g. deoxyribonucleic acid molecule or ribonucleic acid molecule) is altered. Typically, chemical modification is introduced into a nucleic acid molecule by incorporating a chemically modified building block at the stage of synthesizing (*in vivo* or *in vitro*) the respective nucleic acid molecule.

In general, in order to further improve degradation resistance, e.g. resistance to *in vivo* (or *in vitro* as defined herein) degradation by an exo- or endonuclease, and/or for further improving stability of protein expression from the optimized nucleic acid molecule according to the present invention, the optimized nucleic acid molecule may further comprise modifications, such as backbone modifications, sugar modifications and/or base modifications, e.g., lipidmodifications or the like.

The term "modification" as used herein with regard to the claimed mRNA molecule may refer to chemical modifications comprising backbone modifications as well as sugar modifications or base modifications.

In this context, the RNA molecule as defined in the claims may contain nucleotide analogues/modifications, e.g. backbone modifications, sugar modifications or base modifications. A backbone modification in connection with the present invention is a modification, in which phosphates of the backbone of the nucleotides contained in a nucleic acid molecule as defined herein are chemically modified. A sugar modification in connection with the present invention is a chemical modification of the sugar of the nucleotides of the nucleic acid molecule as defined herein. Furthermore, a base modification in connection with the present invention is a chemical modification of the base moiety of the nucleotides of the nucleic acid molecule. In this context, nucleotide analogues or modifications are preferably selected from nucleotide analogues which are applicable for transcription and/or translation.

Preferably, the transcription and/or the translation of the optimized nucleic acid molecule according to the present invention is not significantly impaired by the modifications. Generally, the mRNA molecule of the present invention may comprise any native (= naturally occurring) nucleotide, e.g. guanosine, uracil, adenosine, and/or cytosine or an analogue thereof. In this respect, nucleotide analogues are defined as natively and non-natively occurring variants of the naturally occurring nucleotides adenosine, cytosine, thymidine, guanosine and uridine. Accordingly, analogues are e.g. chemically derivatized nucleotides with non-natively occurring functional groups, which are preferably added to or deleted from the naturally occurring nucleotide or which substitute the naturally occurring functional groups of a nucleotide. Accordingly, each component of the naturally occurring nucleotide may be modified, namely the base component, the sugar (ribose or deoxyribose) component and/or the phosphate component forming the backbone (see above) of the nucleic acid molecule. Analogues of guanosine, uridine, adenosine, thymidine and cytosine include, without implying any limitation, any natively occurring or non-natively occurring guanosine, uridine, adenosine, thymidine or cytosine that has been altered e.g. chemically, for example by acetylation, methylation, hydroxylation, etc., including 1-methyl-adenosine, 1-methyl-guanosine, 1-methyl-inosine, 2,2-dimethyl-guanosine, 2,6-diaminopurine, 2'-Amino-2'-deoxyadenosine, 2'-Amino-2'-deoxycytidine, 2'-Amino-2'-deoxyguanosine, 2'-Amino-2'-deoxyuridine, 2-Amino-6-chloropurineriboside, 2-Aminopurine-riboside, 2'-Araadenosine, 2'-Aracytidine, 2'-Arauridine, 2'-Azido-2'-deoxyadenosine, 2'-Azido-2'-deoxycytidine, 2'-Azido-2'-deoxyguanosine, 2'-Azido-2'-deoxyuridine, 2-Chloroadenosine, 2'-Fluoro-2'-deoxyadenosine, 2'-Fluoro-2'-deoxycytidine, 2'-Fluoro-2'-deoxyguanosine, 2'-Fluoro-2'-deoxyuridine, 2'-Fluorothymidine, 2-methyl-adenosine, 2-methyl-guanosine, 2-methyl-thio-N6-isopenenyl-adenosine, 2'-O-Methyl-2-aminoadenosine, 2'-O-Methyl-2'-deoxyadenosine, 2'-O-Methyl-2'-deoxycytidine, 2'-O-Methyl-2'-deoxyguanosine, 2'-O-Methyl-2'-deoxyuridine, 2'-O-Methyl-5-methyluridine, 2'-O-Methylinosine, 2'-O-Methylpseudouridine, 2-Thiocytidine, 2-thio-cytosine, 3-methyl-cytosine, 4-acetyl-cytosine, 4-Thiouridine, 5-(carboxyhydroxymethyl)-uracil, 5,6-Dihydrouridine, 5-Aminoallylcytidine, 5-Aminoallyl-deoxy-uridine, 5-Bromouridine, 5-carboxymehtylaminomethyl-2-thio-uracil, 5-carboxymethylamonomethyl-uracil, 5-Chloro-Ara-cytosine, 5-Fluoro-uridine, 5-lodouridine, 5-methoxycarbonylmethyl-uridine, 5-methoxy-uridine, 5-methyl-2-thio-uridine, 6-Azacytidine, 6-Azauridine, 6-Chloro-7-deaza-guanosine, 6-Chloropurineriboside, 6-Mercapto-guanosine, 6-Methyl-mercaptopurine-riboside, 7-Deaza-2'-deoxy-guanosine, 7-Deazaadenosine, 7-methyl-guanosine, 8-Azaadenosine, 8-Bromo-adenosine, 8-Bromo-guanosine, 8-Mercapto-guanosine, 8-Oxoguanosine, Benzimidazole-riboside, Beta-D-mannosyl-queosine, Dihydro-uracil, Inosine, N1-Methyladenosine, N6-([6-Aminohexyl]carbamoylmethyl)-adenosine, N6-isopentenyl-adenosine, N6-methyl-adenosine, N7-Methyl-xanthosine, N-uracil-5-oxyacetic acid methyl ester, Puromycin, Queosine, Uracil-5-oxyacetic acid, Uracil-5-oxyacetic acid methyl ester, Wybutoxosine, Xanthosine, and Xylo-adenosine. The preparation of such analogues is known to a person skilled in the art, for example from US Patents 4,373,071, US 4,401,796, US 4,415,732, US 4,458,066, US 4,500,707, US 4,668,777, US 4,973,679, US 5,047,524, US 5,132,418, US 5,153,319, US 5,262,530 and 5,700,642. In the case of an analogue as described above, particular preference may be given according to certain embodiments of the invention to those analogues that increase the protein expression of the encoded peptide or protein or that increase the immunogenicity of the mRNA molecule of the invention and/or do not interfere with a further modification of the mRNA molecule that has been introduced.

In a particularly preferred embodiment, the mRNA molecule according to the invention may further comprise one or more of the modifications described in the following:

### 4.2.1. Sugar Modifications:

The modified nucleosides and nucleotides, which may be incorporated into the mRNA as defined in the claims, can be modified in the sugar moiety. For example, the 2' hydroxyl group (OH) of an RNA molecule can be modified or replaced with a number of different "oxy" or "deoxy" substituents. Examples of "oxy" -2' hydroxyl group modifications include, but are not limited to, alkoxy or aryloxy (-OR, e.g., R = H, alkyl, cycloalkyl, aryl, aralkyl, heteroaryl or sugar); polyethyleneglycols (PEG), -O(CH₂CH₂O)ₙCH₂CH₂OR; "locked" nucleic acids (LNA) in which the 2' hydroxyl is connected, e.g., by a methylene bridge, to the 4' carbon of the same ribose sugar; and amino groups (-O-amino, wherein the amino group, e.g., NRR, can be alkylamino, dialkylamino, heterocyclyl, arylamino, diarylamino, heteroarylamino, or diheteroaryl amino, ethylene diamine, polyamino) or aminoalkoxy.

"Deoxy" modifications include hydrogen, amino (e.g. NH2; alkylamino, dialkylamino, heterocyclyl, arylamino, diaryl amino, heteroaryl amino, diheteroaryl amino, or amino acid); or the amino group can be attached to the sugar through a linker, wherein the linker comprises one or more of the atoms C, N, and O.

The sugar group can also contain one or more carbons that possess the opposite stereochemical configuration than that of the corresponding carbon in ribose. Thus, a modified mRNA molecule can include nucleotides containing, for instance, arabinose as the sugar.

### 4.2.2 Backbone Modifications:

The phosphate backbone may further be modified in the modified nucleosides and nucleotides, which may be incorporated into the mRNA as defined in the claims. The phosphate groups of the backbone can be modified by replacing one or more of the oxygen atoms with a different substituent. Further, the modified nucleosides and nucleotides can include the full replacement of an unmodified phosphate moiety with a modified phosphate as described herein. Examples of modified phosphate groups include, but are not limited to, phosphorothioate, phosphoroselenates, borano phosphates, borano phosphate esters, hydrogen phosphonates, phosphoroamidates, alkyl or aryl phosphonates and phosphotriesters. Phosphorodithioates have both non-linking oxygens replaced by sulfur. The phosphate linker can also be modified by the replacement of a linking oxygen with nitrogen (bridged phosphoroamidates), sulfur (bridged phosphorothioates) and carbon (bridged methylene-phosphonates).

### 4.2.3 Base Modifications:

The modified nucleosides and nucleotides, which may be incorporated into the mRNA molecule as defined in the claims, can further be modified in the nucleobase moiety. Examples of nucleobases found in RNA include, but are not limited to, adenine, guanine, cytosine and uracil. For example, the nucleosides and nucleotides described herein can be chemically modified on the major groove face. In some embodiments, the major groove chemical modifications can include an amino group, a thiol group, an alkyl group, or a halo group.

In particularly preferred embodiments of the present invention, the nucleotide analogues/modifications are selected from base modifications, which are preferably selected from 2-amino-6-chloropurineriboside-5'-triphosphate, 2-Aminopurine-riboside-5'-triphosphate; 2-aminoadenosine-5'-triphosphate, 2'-Amino-2'-deoxycytidine-triphosphate, 2-thiocytidine-5'-triphosphate, 2-thiouridine-5'-triphosphate, 2'-Fluorothymidine-5'-triphosphate, 2'-O-Methyl inosine-5'-triphosphate 4-thiouridine-5'-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-Bromo-2'-deoxycytidine-5'-triphosphate, 5-Bromo-2'-deoxyuridine-5'-triphosphate, 5-iodocytidine-5'-triphosphate, 5-lodo-2'-deoxycytidine-5'-triphosphate, 5-iodouridine-5'-triphosphate, 5-lodo-2'-deoxyuridine-5'-triphosphate, 5-methylcytidine-5'-triphosphate, 5-methyluridine-5'-triphosphate, 5-Propynyl-2'-deoxycytidine-5'-triphosphate, 5-Propynyl-2'-deoxyuridine-5'-triphosphate, 6-azacytidine-5'-triphosphate, 6-azauridine-5'-triphosphate, 6-chloropurineriboside-5'-triphosphate, 7-deazaadenosine-5'-triphosphate, 7-deazaguanosine-5'-triphosphate, 8-azaadenosine-5'-triphosphate, 8-azidoadenosine-5'-triphosphate, benzimidazole-riboside-5'-triphosphate, N1-methyladenosine-5'-triphosphate, N1-methylguanosine-5'-triphosphate, N6-methyladenosine-5'-triphosphate, O6-methylguanosine-5'-triphosphate, pseudouridine-5'-triphosphate, or puromycin-5'-triphosphate, xanthosine-5'-triphosphate. Particular preference is given to nucleotides for base modifications selected from the group of base-modified nucleotides consisting of 5-methylcytidine-5'-triphosphate, 7-deazaguanosine-5'-triphosphate, 5-bromocytidine-5'-triphosphate, and pseudouridine-5'-triphosphate.

In some embodiments, modified nucleosides include pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, I-taurinomethyl-4-thio-uridine, 5-methyl-uridine, 1-methyl-pseudouridine, 4-thio- 1-methyl-pseudouridine, 2-thio-l-methyl-pseudouridine, 1 -methyl- 1-deaza-pseudouridine, 2-thio- 1 -methyl- 1 -deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, and 4-methoxy-2-thio-pseudouridine.

In some embodiments, modified nucleosides include 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio- 1-methyl-pseudoisocytidine, 4-thio- 1 -methyl- 1 -deaza-pseudoisocytidine, 1 -methyl-1 -deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy-l-methyl-pseudoisocytidine .

In other embodiments, modified nucleosides include 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, and 2-methoxy-adenine.

In other embodiments, modified nucleosides include inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, l-methyl-6-thio-guanosine, N2-methyl-6-thio-guanosine, and N2,N2-dimethyl-6-thio-guanosine.

In some embodiments, the nucleotide can be modified on the major groove face and can include replacing hydrogen on C-5 of uracil with a methyl group or a halo group.

In specific embodiments, a modified nucleoside is 5'-O-(1-Thiophosphate)-Adenosine, 5'-O-(1-Thiophosphate)-Cytidine, 5'-O-(1-Thiophosphate)-Guanosine, 5'-O-(1-Thiophosphate)-Uridine or 5'-O-(1-Thiophosphate)-Pseudouridine.

In further specific embodiments the claimed mRNA molecule may comprise nucleoside modifications selected from 6-aza-cytidine, 2-thio-cytidine, alpha-thio-cytidine, Pseudo-iso-cytidine, 5-aminoallyl-uridine, 5-iodo-uridine, N1-methyl-pseudouridine, 5,6-dihydrouridine, alpha-thio-uridine, 4-thio-uridine, 6-aza-uridine, 5-hydroxy-uridine, deoxythymidine, 5-methyl-uridine, Pyrrolo-cytidine, inosine, alpha-thio-guanosine, 6-methyl-guanosine, 5-methyl-cytdine, 8-oxo-guanosine, 7-deaza-guanosine, N1-methyl-adenosine, 2-amino-6-Chloro-purine, N6-methyl-2-amino-purine, Pseudo-iso-cytidine, 6-Chloropurine, N6-methyl-adenosine, alpha-thio-adenosine, 8-azido-adenosine, 7-deaza-adenosine.

### 4.2.4 Lipid modification:

According to a further embodiment, the mRNA as defined in the claims can contain a lipid modification. Such a lipid-modified RNA typically comprises an mRNA as claimed. Such a lipid-modified RNA molecule as defined herein typically further comprises at least one linker covalently linked with that RNA molecule, and at least one lipid covalently linked with the respective linker. Alternatively, the lipid-modified RNA molecule comprises at least one mRNA molecule as defined in the claims and above and at least one (bifunctional) lipid covalently linked (without a linker) with that RNA molecule. According to a third alternative, the lipid-modified RNA molecule comprises an mRNA molecule, as defined in the claims, at least one linker covalently linked with that RNA molecule, and at least one lipid covalently linked with the respective linker, and also at least one (bifunctional) lipid covalently linked (without a linker) with that RNA molecule. In this context, it is particularly preferred that the lipid modification is present at the terminal ends of a linear RNA sequence.

### 4.2.5 Modification of the 5'-end of the mRNA:

According to another preferred embodiment of the invention, the mRNA molecule, as defined herein and in the claims, can be modified by the addition of a so-called "5' cap" structure.

A 5'-cap is an entity, typically a modified nucleotide entity, which generally "caps" the 5'-end of a mature mRNA. A 5'-cap may typically be formed by a modified nucleotide, particularly by a derivative of a guanine nucleotide. Preferably, the 5'-cap is linked to the 5'-terminus via a 5'-5'-triphosphate linkage. A 5'-cap may be methylated, e.g. m7GpppN, wherein N is the terminal 5' nucleotide of the nucleic acid carrying the 5'-cap, typically the 5'-end of an RNA. m7GpppN is the 5'-cap structure which naturally occurs in mRNA transcribed by polymerase II and is therefore not considered as modification comprised in the modified RNA according to the invention. This means the mRNA molecule according to the present invention may comprise an m7GpppN as 5'-cap, but additionally the mRNA molecule comprises at least one further modification as defined herein.

Further examples of 5'cap structures include glyceryl, inverted deoxy abasic residue (moiety), 4',5' methylene nucleotide, 1-(beta-D-erythrofuranosyl) nucleotide, 4'-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3,5 dihydroxypentyl nucleotide, 3'-3'-inverted nucleotide moiety, 3'-3'-inverted abasic moiety, 3'-2'-inverted nucleotide moiety, 3'-2'-inverted abasic moiety, 1,4-butanediol phosphate, 3'-phosphoramidate, hexylphosphate, aminohexyl phosphate, 3'-phosphate, 3'phosphorothioate, phosphorodithioate, or bridging or non-bridging methylphosphonate moiety. These modified 5'-cap structures are regarded as at least one modification comprised in mRNA molecule according to the present invention.

Particularly preferred modified 5'-cap structures are CAP1 (methylation of the ribose of the adjacent nucleotide of m7G), cap2 (additional methylation of the ribose of the 2^{nd} nucleotide downstream of the m7G), cap3 (additional methylation of the ribose of the 3^{rd} nucleotide downstream of the m7G), cap4 (additional methylation of the ribose of the 4^{th} nucleotide downstream of the m7G), ARCA (anti-reverse cap analogue, modified ARCA (e.g. phosphothioate modified ARCA), inosine, N1-methyl-guanosine, 2'-fluoro-guanosine, 7-deaza-guanosine, 8-oxo-guanosine, 2-amino-guanosine, LNA-guanosine, and 2-azido-guanosine.

### 5. Combinations; Methods for Making Combinations

The methods of making the mRNA molecule of the invention (i.e. as defined in the claims) comprise at least the following key step (2):
(2) Combining of at least two ribonucleic acid modules (moieties), to form an mRNA molecule. The mRNA molecule is preferably a chimeric molecule.

At least one of these moieties encodes a protein or polypeptide of interest. The mRNA molecule comprises the two moieties in functional relationship to each other. For example, when a further moiety encoding a polypeptide or protein of interest is combined with said protein or polypeptide of interest, then the combination occurs such that the mRNA molecule encodes the two protein elements or polypeptide elements in functional relationship to each other, e.g. as fusion protein. For example, when a non-coding moiety, i.e. not encoding any polypeptide or protein, is combined with said protein or polypeptide of interest, then the combination occurs such that the mRNA molecule encodes protein or polypeptide of interest in functional relationship to the non-coding moiety, e.g. such that the non-coding moiety beneficially influences translation of the protein or polypeptide of interest, or beneficially influences any other functional property, such as RNA stability. The at least one additional polypeptide or protein element is an element that extends protein half-life and is characterized by at least 80% identity to SEQ ID NO: 1705. Methods for making the claimed mRNA molecules are well established in the art, e.g. Current Protocols in Molecular Biology, Ausubel et al. (ed.), 2003, John Wiley & Sons, Inc., and can be used in step (2). Multiple same or different steps (2) can be performed, either sequentially, or simultaneously.

Optionally, a further step (3) is present in addition to step (2). Step (3) relates to a chemical modification as described herein. Typically, chemical modification is introduced into a nucleic acid molecule by incorporating a chemically modiefied building block at the stage of synthesizing (*in vivo* or *in vitro*) the respective nucleic acid molecule. Step (3) may be characterized as follows:
(3) Modifying the mRNA molecule by (i) substituting at least one building block of the mRNA molecule by at least one different building block, or (ii) adding a further building block to the mRNA molecule.

Preferably, the step of substituting is characterized in that one building block of the mRNA molecule is replaced by a different building block, prefereably selected from the following:
(i-a) a sugar building block of the nucleic acid molecule is replaced by a different sugar building block, or
(i-b) a backbone building block of the nucleic acid molecule is replaced by a different backbone building block, or
(i-c) a base building block of the nucleic acid molecule is replaced by a different base building block.

Preferably, the step of adding is characterized in that a further building block is added to the mRNA molecule, prefereably selected from the following:
(ii-a) a lipid building block is added to the mRNA molecule, or
(ii-b) a 5'-cap is added to the nucleic acid molecule.

In addition to the general principle that chemical modification is typically introduced into a mRNA at the stage of synthesizing (*in vivo* or *in vitro*) the respective mRNA molecule, the 5'-cap according to (ii-b) can also be introduced post-transcriptionally (e.g., after RNA in vitro transcription using viral or eukaryotic capping enzymes).

All these embodiments of adding and substituting are combinable with each other.

Any embodiment of substituting a building block or of adding a building block described throughout this specification can be realized in this method step.

For illustrative purposes, a substitution can consist of substitution by a naturally occurring building block; respective embodiments are realized as G/C modification, as described below, or as codon optimization, as described below. Alternatively, such substitution can consist of the introduction of a chemical modification (sugar modification, backbone modification, base modification, lipid modification, and introduction of a 5'-cap, all as described below. Methods for making such substitutions are well established in the art, e.g. Current Protocols in Molecular Biology, Ausubel et al. (ed.), 2003, John Wiley & Sons, Inc.), and can be used in step (3). Multiple same or different steps (3) can be performed, either sequentially, or simultaneously.

Optionally, the steps (2) and (3) are performed simultaneously.

Alternativly, the steps (2) and (3) are performed sequentially. In that case, step (2) is preferably performed first.

Optionally, step (2) is preceded by the following step (1):
(1) Designing (*in silico*) of a protein or polypeptide with desired properties, or of a nucleic acid molecule having desired properties.

Preferably, the method of designing is carried out as follows:
(i) designing a nucleic acid molecule having desired properties, or
(ii) in a a first step: designing a protein or polypeptide having desired properties, and, in a subsequent second step: deducing a nucleic acid sequence that encodes said protein or polypeptide, thereby designing a nucleic acid molecule encoding desired properties.

The designing of the nucleic acid molecule according to (i) or (ii) is followed by physically preparing the designed nucleic acid molecule, as defiend herein. Thereby, an mRNA molecule is obtained.

The step of designing (rational design) is not limited as such and will comprise any considerations deemed suitable by the skilled person performing said step. Optionally, said step can be implemented or aided by a computer program. In any event, the inclusion of step (1) enables the making of nucleic acid molecules having any combination of desired properties, or the indirect making of polypeptides or proteins (e.g. fusion proteins) having any combinations having any combination of desired properties. Any functional property of a nucleic acid molecule or of a polypeptide may be, under some circumstances, a desired property. Functional properties associated with nucleic acid moieties or with polypeptide elements are described throughout the present disclosure. The rational design of step (1) enables the targeted combination of any two or more such functional properties.

The methods of the invention can also be partially or completely be carried out by a machine or apparatus, based on the guidance provided herein. A respectively suitable apparatus is also comprised by the present invention, in particular, an apparatus suitable for providing an optimized nucleic acid molecule according to the invention.

As an example, the mRNA molecule of the present invention may comprise the following moieties in the following order:
5'-cap - 5'-UTR - ORF - 3'-UTR - histone stem-loop - poly(A)/(C) sequence;
5'-cap - 5'-UTR - ORF - 3'-UTR- poly(A)/(C) sequence - histone stem-loop;
5'-cap - 5'-UTR - ORF - IRES - ORF - 3'-UTR - histone stem-loop - poly(A)/(C) sequence;
5'-cap - 5'-UTR- ORF - IRES - ORF - 3'-UTR - histone stem-loop - poly(A)/(C) sequence - poly(A)/(C) sequence;
5'-cap - 5'-UTR - ORF - IRES - ORF - 3'-UTR- poly(A)/(C) sequence - histone stem-loop;
5'-cap - 5'-UTR - ORF - IRES - ORF - 3'-UTR- poly(A)/(C) sequence - poly(A)/(C) sequence - histone stem-loop;
5'-cap - 5'-UTR- ORF - 3'-UTR - poly(A)/(C) sequence - poly(A)/(C) sequence;
5'-cap - 5'-UTR - ORF - 3'-UTR - poly(A)/(C) sequence - poly(A)/(C) sequence - histone stem loop; etc.

In the above, "ORF" stands for one or more open reading frames, each comprised of one or more coding moieties, as described herein. Typically, at least one moiety of said ORF encodes a polypeptide or protein of interest (also referred to as coding sequence or cds).

In some embodiments, the mRNA molecule comprises further moieties such as a 5'-cap, a poly(C) sequence and/or an IRES-motif. A 5'-cap may be added, during transcription or post-transcriptionally, to the 5'end of an RNA. Furthermore, the mRNA molecule as claimed may be modified by a sequence of at least 10 cytidines, preferably at least 20 cytidines, more preferably at least 30 cytidines (so-called "poly(C) sequence"). In particular, the mRNA molecule as claimed may contain a poly(C) sequence of typically about 10 to 200 cytidine nucleotides, preferably about 10 to 100 cytidine nucleotides, more preferably about 10 to 70 cytidine nucleotides or even more preferably about 20 to 50 or even 20 to 30 cytidine nucleotides. Most preferably, the mRNA molecule as claimed comprises a poly(C) sequence of 30 cytidine residues. Thus, preferably the mRNA molecule as claimed comprises, preferably in 5'-to-3' direction, at least one 5'-UTR moiety as described above, an ORF, at least one 3'-UTR moiety as described above, a poly(A) sequence or a polyadenylation signal, and a poly(C) sequence or, in 5'-to-3' direction, optionally a further 5'-UTR, an ORF, at least one 3'-UTR moiety as described above, a poly(A) sequence or a polyadenylation signal, and a poly(C) sequence, or, in 5'-to-3' direction, at least one 5'-UTR moiety as described above, an ORF, optionally a further 3'-UTR, a poly(A) sequence or a polyadenylation signal, and a poly(C) sequence.

### 6. Use of the optimized nucleic acid

The present invention also provides an mRNA molecule obtainable by a method for generating an mRNA molecule as claimed.

The mRNA molecule as claimed is useful e.g. in the context of a vector, of a cell, of a pharmaceutical composition and of medical methods and uses, as described herein below:

### 6.1 Vector

In one aspect, the present invention provides a vector suitabe for producing an mRNA molecule as defined in the claims. In particular, the preferred embodiments described above for an mRNA molecule as claimed also apply for an mRNA molecule, which is produced by a vector as claimed. For example, in the inventive vector the at least one 3'-UTR moiety and/or the at least one 5'-UTR moiety and the ORF are as claimed, including the preferred embodiments.

The vector suitably comprises a cloning site. The cloning site may be any sequence that is suitable for introducing an open reading frame or a sequence comprising an open reading frame, such as one or more restriction sites. Thus, the vector comprising a cloning site is preferably suitable for inserting an open reading frame into the vector, preferably for inserting an open reading frame 3' to the 5'-UTR moiety and/or 5' to the 3'-UTR moiety. Preferably the cloning site or the ORF is located 3' to the 5'-UTR moiety and/or 5' to the 3'-UTR moiety, preferably in close proximity to the 3'-end of the 5'-UTR moiety and/or to the 5'-end of the 3'-UTR moiety. For example, the cloning site or the ORF may be directly connected to the 3'-end of the 5'-UTR moiety and/or to the 5'-end of the 3'-UTR moiety or they may be connected via a stretch of nucleotides, such as by a stretch of 2, 4, 6, 8, 10, 20 etc. nucleotides as described above for the mRNA molecule according to the present invention.

In a particularly preferred embodiment, the vector as claimed is suitable for producing the mRNA molecule as claimed, preferably for producing an mRNA as defined in the claims, for example, by optionally inserting an open reading frame or a sequence comprising an open reading frame into the vector and transcribing the vector. Thus, preferably, the vector comprises moieties needed for transcription, such as a promoter, e.g. an RNA polymerase promoter. Preferably, the vector is suitable for transcription using eukaryotic, prokaryotic, viral or phage transcription systems, such as eukaryotic cells, prokaryotic cells, or eukaryotic, prokaryotic, viral or phage *in vitro* transcription systems. Thus, for example, the vector may comprise a promoter sequence, which is recognized by a polymerase, such as by an RNA polymerase, e.g. by a eukaryotic, prokaryotic, viral, or phage RNA polymerase. In a preferred embodiment, the vector comprises a phage RNA polymerase promoter such as an SP6, T3 or T7, preferably a T7 promoter. Preferably, the vector is suitable for *in vitro* transcription using a phageenzyme based *in vitro* transcription system, such as a T7 RNA polymerase based *in vitro* transcription system.

In another preferred embodiment, the vector may be used directly for expression of the encoded peptide or protein in cells or tissue. For this purpose, the vector comprises particular moieties, which are necessary for expression in those cells/tissue e.g. particular promoter sequences, such as a CMV promoter.

The vector may further comprise a poly(A) sequence and/or a polyadenylation signal as described above for the mRNA molecule as claimed.

The vector may be an RNA vector or a DNA vector. Preferably, the vector is a DNA vector. The vector may be any vector known to the skilled person, such as a viral vector or a plasmid vector. Preferably, the vector is a plasmid vector, preferably a DNA plasmid vector. Preferably, an RNA vector according to the present invention comprises a sequence selected from the group consisting of the sequences according to RNA sequences corresponding to DNA sequences described above in relation to the DNA vector according to the present invention.

Preferably, the vector is a circular molecule. Preferably, the vector is a double-stranded molecule, such as a double-stranded DNA molecule. Such circular, preferably double stranded DNA molecule may be used conveniently as a storage form for the inventive optimized nucleic acid molecule. Furthermore, it may be used for transfection of cells, for example, cultured cells. Also it may be used for *in vitro* transcription for obtaining an artificial RNA molecule according to the invention.

Preferably, the vector, preferably the circular vector, is linearizable, for example, by restriction enzyme digestion. In a preferred embodiment, the vector comprises a cleavage site, such as a restriction site, preferably a unique cleavage site, located immediately 3' to the ORF, or - if present - located immediately 3' to the 3'-UTR moiety, or - if present - located 3' to the poly(A) sequence or polyadenylation signal, or - if present - located 3' to the poly(C) sequence, or - if present - located 3' to the histone stem-loop. Thus, preferably, the product obtained by linearizing the vector terminates at the 3'end with the 3'-end of the ORF, or - if present - with the 3'-end of the 3'-UTR moiety, or - if present - with the 3'-end of the poly(A) sequence or polyadenylation signal, or - if present - with the 3'-end of the poly(C) sequence. In the embodiment, wherein the vector according to the present invention comprises the optimized nucleic acid molecule according to the present invention, a restriction site, preferably a unique restriction site, is preferably located immediately 3' to the 3'-end of the mRNA molecule.

### 6.2 Cell

In a further aspect, the present invention relates to a cell comprising the mRNA molecule according to the present invention or the vector according to the present invention. The cell may be any cell, such as a bacterial cell, insect cell, plant cell, vertebrate cell, e.g. a mammalian cell. Such cell may be, e.g., used for replication of the vector of the present invention, for example, in a bacterial cell. Furthermore, the cell may be used for transcribing the mRNA molecule or the vector as claimed and/or translating the open reading frame of the mRNA molecule or the vector as claimed. For example, the cell may be used for recombinant protein production.

The cells according to the present invention are, for example, obtainable by standard nucleic acid transfer methods, such as standard transfection, transduction or transformation methods. For example, the mRNA molecule or the vector according to the present invention may be transferred into the cell by electroporation, lipofection, e.g. based on cationic lipids and/or liposomes, calcium phosphate precipitation, nanoparticle based transfection, virus based transfection, or based on cationic polymers, such as DEAE-dextran or polyethylenimine etc.

Preferably, the cell is a mammalian cell, such as a cell of human subject, a domestic animal, a laboratory animal, such as a mouse or rat cell. Cells include in particular cell lines, primary cells, cells in tissue or subjects. In specific embodiments cell types allowing cell culture may be suitable for the present invention. The cell may be a cell of an established cell line, such as a CHO, BHK, 293T, COS-7, HeLa, HEPG2 and HEK, etc. or the cell may be a primary cell, such as a human dermal fibroblast (HDF) cell etc., preferably a cell isolated from an organism. In a preferred embodiment, the cell is an isolated cell of a mammalian subject, preferably of a human subject. For example, the cell may be an immune cell, such as a dendritic cell, a cancer or tumor cell, or any somatic cell etc., preferably of a mammalian subject, preferably of a human subject.

### 6.3 Pharmaceutical composition

In a further aspect, the present invention provides a pharmaceutical composition comprising the mRNA molecule according to the present invention, the vector according the present invention, or the cell according to the present invention. The pharmaceutical composition according to the invention may be used, e.g., as a vaccine, for example, for genetic vaccination. Thus, the ORF may, e.g., encode an antigen to be administered to a patient for vaccination. Thus, in a preferred embodiment, the pharmaceutical composition according to the present invention is a vaccine. Furthermore, the pharmaceutical composition according to the present invention may be used, e.g., for gene therapy.

Preferably, the pharmaceutical composition further comprises one or more pharmaceutically acceptable vehicles, diluents and/or excipients and/or one or more adjuvants. In the context of the present invention, a pharmaceutically acceptable vehicle typically includes a liquid or non-liquid basis for the inventive pharmaceutical composition. In one embodiment, the pharmaceutical composition is provided in liquid form. In this context, preferably, the vehicle is based on water, such as pyrogen-free water, isotonic saline or buffered (aqueous) solutions, e.g phosphate, citrate etc. buffered solutions. The buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of mammalian cells due to osmosis or other concentration effects. Reference media are e.g. liquids occurring in *"in vivo"* methods, such as blood, lymph, cytosolic liquids, or other body liquids, or e.g. liquids, which may be used as reference media in *"in vitro"* methods, such as common buffers or liquids. Such common buffers or liquids are known to a skilled person. Ringer-Lactate solution is particularly preferred as a liquid basis.

One or more compatible solid or liquid fillers or diluents or encapsulating compounds suitable for administration to a patient may be used as well for the inventive pharmaceutical composition. The term "compatible" as used herein preferably means that these components of the inventive pharmaceutical composition are capable of being mixed with the mRNA molecule, vector or cells as defined herein in such a manner that no interaction occurs which would substantially reduce the pharmaceutical effectiveness of the inventive pharmaceutical composition under typical use conditions.

The pharmaceutical composition according to the present invention may optionally further comprise one or more additional pharmaceutically active components. A pharmaceutically active component in this context is a compound that exhibits a therapeutic effect to heal, ameliorate or prevent a particular indication or disease. Such compounds include, without implying any limitation, peptides or proteins, nucleic acids, (therapeutically active) low molecular weight organic or inorganic compounds (molecular weight less than 5000, preferably less than 1000), sugars, antigens or antibodies, therapeutic agents already known in the prior art, antigenic cells, antigenic cellular fragments, cellular fractions, cell wall components (e.g. polysaccharides), modified, attenuated or de-activated (e.g. chemically or by irradiation) pathogens (virus, bacteria etc.).

Furthermore, the pharmaceutical composition according to the invention may comprise a carrier for the mRNA molecule or the vector. Such a carrier may be suitable for mediating dissolution in physiological acceptable liquids, transport and cellular uptake of the pharmaceutical active mRNA molecule or the vector. Accordingly, such a carrier may be a component which may be suitable for depot and delivery of an mRNA molecule or vector according to the invention. Such components may be, for example, cationic or polycationic carriers or compounds which may serve as transfection or complexation agent.

Particularly preferred transfection or complexation agents in this context are cationic or polycationic compounds, including protamine, nucleoline, spermine or spermidine, or other cationic peptides or proteins, such as poly-L-lysine (PLL), poly-arginine, basic polypeptides, cell penetrating peptides (CPPs), including HIV-binding peptides, HIV-1 Tat (HIV), Tat-derived peptides, Penetratin, VP22 derived or analog peptides, HSV VP22 (Herpes simplex), MAP, KALA or protein transduction domains (PTDs), PpT620, proline-rich peptides, arginine-rich peptides, lysine-rich peptides, MPG-peptide(s), Pep-1, L-oligomers, Calcitonin peptide(s), Antennapedia-derived peptides (particularly from *Drosophila antennapedia*)*,* pAntp, plsl, FGF, Lactoferrin, Transportan, Buforin-2, Bac715-24, SynB, SynB(1), pVEC, hCT-derived peptides, SAP, or histones.

Further preferred cationic or polycationic compounds may include cationic polysaccharides, for example chitosan, polybrene, cationic polymers, e.g. polyethyleneimine (PEI), cationic lipids, e.g. DOTMA: N[1-(2,3-sioleyloxy)propyl)]-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-(-trimethylammonioacetyl)diethanolamine chloride, CLIP1: rac-[(2,3-dioctadecyloxypropyl)(2-hydroxyethyl)]-dimethylammonium chloride, CLIP6: rac-[2(2,3-dihexadecyloxypropyl-oxymethyloxy)ethyl]-trimethylammonium, CLIP9: rac-[2(2,3-dihexadecyloxypropyl-oxysuccinyloxy)ethyl]-trimethylammonium, oligofectamine, or cationic or polycationic polymers, e.g. modified polyaminoacids, such as alpha-aminoacid-polymers or reversed polyamides, etc., modified polyethylenes, such as PVP (poly(N-ethyl-4-vinylpyridinium bromide)), etc., modified acrylates, such as pDMAEMA (poly(dimethylaminoethyl methylacrylate)), etc., modified Amidoamines such as pAMAM (poly(amidoamine)), etc., modified polybetaaminoester (PBAE), such as diamine end modified 1,4 butanediol diacrylate-co-5-amino-1-pentanol polymers, etc., dendrimers, such as polypropylamine dendrimers or pAMAM based dendrimers, etc., polyimine(s), such as PEI: poly(ethyleneimine), poly(propyleneimine), etc., polyallylamine, sugar backbone based polymers, such as cyclodextrin based polymers, dextran based polymers, Chitosan, etc., silan backbone based polymers, such as PMOXA-PDMS copolymers, etc., Blockpolymers consisting of a combination of one or more cationic blocks (e.g. selected of a cationic polymer as mentioned above) and of one or more hydrophilic- or hydrophobic blocks (e.g polyethyleneglycole); etc.

Additionally, preferred cationic or polycationic proteins or peptides, which can be used as an adjuvant by complexing the mRNA molecule as claimed, may be selected from following proteins or peptides having the following total formula (VII): (Arg)ₗ;(Lys)ₙ; (His)ₙ;(Orn)ₒ;(Xaa)ₓ, wherein l + m + n +o + x = 8-15, and l, m, n or o independently of each other may be any number selected from 0, 1, 2, 3, 4, 5, 6, 7, 8, 9, 10, 11, 12, 13, 14 or 15, provided that the overall content of Arg, Lys, His and Orn represents at least 50% of all amino acids of the oligopeptide; and Xaa may be any amino acid selected from native (= naturally occurring) or non-native amino acids except of Arg, Lys, His or Orn; and x may be any number selected from 0, 1, 2, 3 or 4, provided, that the overall content of Xaa does not exceed 50 % of all amino acids of the oligopeptide. Particularly preferred oligoarginines in this context are e.g. Arg7, Arg8, Arg9, Arg7, H3R9, R9H3, H3R9H3, YSSR9SSY, (RKH)4, Y(RKH)2R, etc.

Furthermore, such cationic or polycationic compounds or carriers may be cationic or polycationic peptides or proteins, which preferably comprise or are additionally modified to comprise at least one -SH moiety. Preferably, a cationic or polycationic carrier is selected from cationic peptides having the following sum formula (VII):

{(Arg)ₗ;(Lys)ₘ;(His)ₙ;(Orn)ₒ;(Xaa)ₓ}; formula (VII)

wherein l + m + n + o + x = 3-100, and l, m, n or o independently of each other is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90 and 91-100 provided that the overall content of Arg (Arginine), Lys (Lysine), His (Histidine) and Orn (Ornithine) represents at least 10% of all amino acids of the oligopeptide; and Xaa is any amino acid selected from native (= naturally occurring) or non-native amino acids except of Arg, Lys, His or Orn; and x is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80, 81-90, provided, that the overall content of Xaa does not exceed 90 % of all amino acids of the oligopeptide. Any of amino acids Arg, Lys, His, Orn and Xaa may be positioned at any place of the peptide. In this context cationic peptides or proteins in the range of 7-30 amino acids are particular preferred.

Further, the cationic or polycationic peptide or protein, when defined according to formula {(Arg)ₗ;(Lys)ₘ;(His)ₙ;(Orn)ₒ;(Xaa)ₓ} (formula (VII) as shown above and which comprise or are additionally modified to comprise at least one -SH moeity, may be, without being restricted thereto, selected from subformula (Vlla):

{(Arg)ₗ;(Lys)ₘ;(His)ₙ;(Orn)ₒ;(Xaa')ₓ(Cys)_{y}} subformula (Vlla)

wherein (Arg)ₗ;(Lys)ₘ;(His)ₙ;(Orn)ₒ; and x are as defined herein, Xaa' is any amino acid selected from native (= naturally occurring) or non-native amino acids except of Arg, Lys, His, Orn or Cys and y is any number selected from 0, 1, 2, 3, 4, 5, 6, 7, 8, 9, 10, 11, 12, 13, 14, 15, 16, 17, 18, 19, 20, 21-30, 31-40, 41-50, 51-60, 61-70, 71-80 and 81-90, provided that the overall content of Arg (Arginine), Lys (Lysine), His (Histidine) and Orn (Ornithine) represents at least 10% of all amino acids of the oligopeptide. Further, the cationic or polycationic peptide may be selected from subformula (VIIb):

Cys₁ {(Arg)ₗ;(Lys)ₘ;(His)ₙ;(Orn)ₒ;(Xaa)ₓ} Cys₂ subformula (VIIb)

wherein empirical formula {(Arg)ₗ;(Lys)ₘ;(His)ₙ;(Orn)ₒ;(Xaa)ₓ} (formula (VIII)) is as defined herein and forms a core of an amino acid sequence according to (semiempirical) formula (VIII) and wherein Cys₁ and Cys₂ are Cysteines proximal to, or terminal to (Arg)ₗ;(Lys)ₘ;(His)ₙ;(Orn)ₒ;(Xaa)ₓ.

Disulfide-linked polyethyleneglycol/peptide conjugates for the transfection of nucleic acids are disclosed in WO2011/026641. Such conjugates are also encompassed by the present invention. Thus, the polypeptide or protein element of the present invention may be selected to allow preparation of a disulfide-linked polyethyleneglycol/peptide conjugate. In a particular embodiment, the polymeric carrier which may be used to complex the mRNA molecule or the vector may be selected from a polymeric carrier molecule according to generic formula (IV):

L-P1-S-[S-P2-S] n-S-P3-L formula (IV),

wherein
- P1 and P3: are different or identical to each other and represent a linear or branched hydrophilic polymer chain, each P1 and P3 exhibiting at least one -SH group, capable to form a disulfide linkage upon condensation with component P2, or alternatively with (AA), (AA)x, or [(AA)x]z if such components are used as a linker between P1 and P2 or P3 and P2) and/or with further components (e.g. (AA), (AA)x, [(AA)x]z or L), the linear or branched hydrophilic polymer chain selected independent from each other from polyethylene glycol (PEG), poly-N-(2-hydroxypropyl)methacrylamide, poly-2-(methacryloyloxy)ethyl phosphorylcholines, poly(hydroxyalkyl L-asparagine), poly(2-(methacryloyloxy)ethyl phosphorylcholine), hydroxyethylstarch or poly(hydroxyalkyl L-glutamine), wherein the hydrophilic polymer chain exhibits a molecular weight of about 1 kDa to about 100 kDa, preferably of about 2 kDa to about 25 kDa; or more preferably of about 2 kDa to about 10 kDa, e.g. about 5 kDa to about 25 kDa or 5 kDa to about 10 kDa;
- P2: is a cationic or polycationic peptide or protein, e.g. as defined above for the polymeric carrier formed by disulfide-crosslinked cationic components, and preferably having a length of about 3 to about 100 amino acids, more preferably having a length of about 3 to about 50 amino acids, even more preferably having a length of about 3 to about 25 amino acids, e.g. a length of about 3 to 10, 5 to 15, 10 to 20 or 15 to 25 amino acids, more preferably a length of about 5 to about 20 and even more preferably a length of about 10 to about 20; or is a cationic or polycationic polymer, e.g. as defined above for the polymeric carrier formed by disulfide-crosslinked cationic components, typically having a molecular weight of about 0.5 kDa to about 30 kDa, including a molecular weight of about 1 kDa to about 20 kDa, even more preferably of about 1.5 kDa to about 10 kDa, or having a molecular weight of about 0.5 kDa to about 100 kDa, including a molecular weight of about 10 kDa to about 50 kDa, even more preferably of about 10 kDa to about 30 kDa; each P2 exhibiting at least two -SH-moieties, capable to form a disulfide linkage upon condensation with further components P2 or component(s) P1 and/or P3 or alternatively with further components (e.g. (AA), (AA)x, or [(AA)x]z);
- -S-S-: is a (reversible) disulfide bond (the brackets are omitted for better readability), wherein S preferably represents sulphur or a -SH carrying moiety, which has formed a (reversible) disulfide bond. The (reversible) disulfide bond is preferably formed by condensation of -SH-moieties of either components P1 and P2, P2 and P2, or P2 and P3, or optionally of further components as defined herein (e.g. L, (AA), (AA)x, [(AA)x]z, etc); The -SH group may be part of the structure of these components or added by a modification as defined below;
- L: is an optional ligand, which may be present or not, and may be selected independent from the other from RGD, Transferrin, Folate, a signal peptide or signal sequence, a localization signal or sequence, a nuclear localization signal or sequence (NLS), an antibody, a cell penetrating peptide, (e.g. TAT or KALA), a ligand of a receptor (e.g. cytokines, hormones, growth factors etc), small molecules (e.g. carbohydrates like mannose or galactose or synthetic ligands), small molecule agonists, inhibitors or antagonists of receptors (e.g. RGD peptidomimetic analogues), or any further protein as defined herein, etc.;
- n: is an integer, typically selected from a range of about 1 to 50, preferably from a range of about 1, 2 or 3 to 30, more preferably from a range of about 1, 2, 3, 4, or 5 to 25, or a range of about 1, 2, 3, 4, or 5 to 20, or a range of about 1, 2, 3, 4, or 5 to 15, or a range of about 1, 2, 3, 4, or 5 to 10, including e.g. a range of about 4 to 9, 4 to 10, 3 to 20, 4 to 20, 5 to 20, or 10 to 20, or a range of about 3 to 15, 4 to 15, 5 to 15, or 10 to 15, or a range of about 6 to 11 or 7 to 10. Most preferably, n is in a range of about 1, 2, 3, 4, or 5 to 10, more preferably in a range of about 1, 2, 3, or 4 to 9, in a range of about 1, 2, 3, or 4 to 8, or in a range of about 1, 2, or 3 to 7.

With respect to disulfide-linked polyethyleneglycol/peptide conjugates for the transfection of nucleic acids, the disclosure of WO 2011/026641.

Each of hydrophilic polymers P1 and P3 typically exhibits at least one -SH group, wherein the at least one -SH group is capable to form a disulfide linkage upon reaction with component P2 or with component (AA) or (AA)ₓ, if used as linker between P1 and P2 or P3 and P2 as defined below and optionally with a further component, e.g. L and/or (AA) or (AA)x, e.g. if two or more -SH-moieties are contained. The following sub-formulae "P1-S-S-P2" and "P2-S-S-P3" within generic formula (IV) above (the brackets are omitted for better readability), wherein any of S, P1 and P3 are as defined herein, typically represent a situation, wherein one-SH group of hydrophilic polymers P1 and P3 was condensed with one -SH group of component P2 of generic formula (IV) above, wherein both sulphurs of these -SH groups form a disulfide bond -S-S- as defined herein in formula (IV). These -SH groups are typically provided by each of the hydrophilic polymers P1 and P3, e.g. via an internal cysteine or any further (modified) amino acid or compound which carries a -SH moiety. Accordingly, the sub-formulae "P1-S-S-P2" and "P2-S-S-P3" may also be written as "P1-Cys-Cys-P2" and "P2-Cys-Cys-P3", if the -SH- moiety is provided by a cysteine, wherein the term Cys-Cys represents two cysteines coupled via a disulfide bond, not via a peptide bond. In this case, the term "-S-S-" in these formulae may also be written as "-S-Cys", as "-Cys-S" or as "-Cys-Cys-". In this context, the term "-Cys-Cys-" does not represent a peptide bond but a linkage of two cysteines via their -SH groups to form a disulfide bond. Accordingly, the term "-Cys-Cys-" also may be understood generally as "-(Cys-S)-(S-Cys)-", wherein in this specific case S indicates the sulphur of the -SH group of cysteine. Likewise, the terms "-S-Cys" and "-Cys-S" indicate a disulfide bond between a -SH containing moiety and a cysteine, which may also be written as "-S-(S-Cys)" and "-(Cys-S)-S". Alternatively, the hydrophilic polymers P1 and P3 may be modified with a -SH moiety, preferably via a chemical reaction with a compound carrying a -SH moiety, such that each of the hydrophilic polymers P1 and P3 carries at least one such -SH moiety. Such a compound carrying a -SH moiety may be e.g. an (additional) cysteine or any further (modified) amino acid, which carries a -SH moiety. Such a compound may also be any non-amino compound or moiety, which contains or allows to introduce a - SH moiety into hydrophilic polymers P1 and P3 as defined herein. Such non-amino compounds may be attached to the hydrophilic polymers P1 and P3 of formula (IV) of the polymeric carrier via chemical reactions or binding of compounds, e.g. by binding of a 3-thio propionic acid or thioimolane, by amide formation (e.g. carboxylic acids, sulphonic acids, amines, etc), by Michael addition (e.g maleinimide moieties, α, β unsatured carbonyls, etc), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow Sn-type substitution reactions (e.g halogenalkans, thiols, alcohols, amines, hydrazines, hydrazides, sulphonic acid esters, oxyphosphonium salts) or other chemical moieties which can be utilized in the attachment of further components. A particularly preferred PEG derivate in this context is alpha-Methoxy-omega-mercapto poly(ethylene glycol). In each case, the SH group, e.g. of a cysteine or of any further (modified) amino acid or compound, may be present at the terminal ends or internally at any position of hydrophilic polymers P1 and P3. As defined herein, each of hydrophilic polymers P1 and P3 typically exhibits at least one -SH-group preferably at one terminal end, but may also contain two or even more -SH groups, which may be used to additionally attach further components as defined herein, preferably further functional peptides or proteins e.g. a ligand, an amino acid component (AA) or (AA)x, antibodies, cell penetrating peptides or enhancer peptides (e.g. TAT, KALA), etc.

The polymeric carrier molecule can additionally contain an amino acid component (AA)ₓ, wherein x is an integer selected from a range of about 1 to 100. The amino acid component (AA)ₓ can comprise an aromatic amino acid component, a hydrophilic amino acid component, a lipophilic amino acid component, a weak basic amino acid component, a signal peptide, localization signal or sequence, a nuclear localization signal or sequence, a cell penetrating peptide, a therapeutically active protein or peptide, an antigen or an antigenic epitope, a tumour antigen, a pathogenic antigen (an animal antigen, a viral antigen, a protozoal antigen, a bacterial antigen, an allergic antigen), an autoimmune antigen, or a further antigen, an allergen, an antibody, an immunostimulatory protein or peptide, an antigen-specific T-cell receptor, or another protein or peptide suitable for a specific (therapeutic) application. The amino acid component (AA)ₓ can occur as a mixed repetitive amino acid component [(AA)ₓ]₂, wherein z is an integer selected from a range of about 1 to 30. Above formula (IV) can be modified according to formula (IVa)

L-P¹-S-{[S-P²-S]ₐ[_{S}-(AA)ₓ-S]_{b}}-S-P³-L,

wherein x, z, S, L, AA, P¹, P² and P³ are as defined before and
a + b = n, wherein
- n: is as defined before, preferably in a range of about 1, 2, 3, 4, or 5 to 10;
- a: is an integer, selected independent from integer b from a range of about 1 to 50, preferably in a range of about 1, 2, 3, 4, or 5 to 10, and
- b: is an integer, selected independent from integer a from a range of about 1 to 50, preferably in a range of about 1, 2, 3, 4, or 5 to 10,
and wherein the single components [S-P²-S] and [S-(AA)ₓ-S] occur in any order in the subformula {[S-P²-S]ₐ[S-(AA)ₓ-S]_{b}}.

Component P² can be a cationic or polycationic peptide selected from protamine, nucleoline, spermine or spermidine, poly-L-lysine (PLL), basic polypeptides, poly-arginine, cell penetrating peptides (CPPs), chimeric CPPs, Transportan, or MPG peptides, HIV-binding peptides, Tat, HIV-1 Tat (HIV), Tat-derived peptides, oligoarginines, members of the penetratin family, Penetratin, Antennapedia-derived peptides (particularly from *Drosophila antennapedia*)*,* pAntp, plsl, etc., antimicrobial-derived CPPs, Buforin-2, Bac715-24, SynB, SynB(1), pVEC, hCT-derived peptides, SAP, MAP, KALA, PpTG20, Proline-rich peptides, Loligomere, Arginine-rich peptides, Calcitonin-peptides, FGF, Lactoferrin, histones, VP22 derived or analog peptides, HSV, VP22 (Herpes simplex), MAP, KALA or protein transduction domains (PTDs, PpT620, prolin-rich peptides, lysine-rich peptides, Pep-1, L-oligomers, Calcitonin peptide(s). The -SH group of component(s) P² can be provided by a cysteine.

Such polymeric carrier molecules can be incorporated in a polymeric carrier cargo complex, wherein the polymeric carrier cargo is formed of said polymeric carrier molecule and a nucleic acid. Said nucleic acid can be provided in a molar ratio of about 5 to 10000 of polymeric carrier molecule : nucleic acid.

The polymeric cargo complexes enable expression of a therapeutically active protein or peptide, an antigen, including tumor antigens, pathogenic antigens, animal antigens, viral antigens, protozoal antigens, bacterial antigens, allergic antigens, autoimmune antigens, allergens, antibodies, immunostimulatory proteins or peptides, or antigen-specific T-cell receptors.

The polymeric carrier molecule can be prepared by a method comprising following steps:
a) providing at least one cationic or polycationic protein or peptide as component P² as defined herein and/or at least one cationic or polycationic polymer as component P² as defined according to one of claims 1 to 8, and optionally at least one further component (AA)ₓ, mixing these components, preferably in a basic milieu, preferably in the presence of oxygen or a further starter which leads to mild oxidation conditions, and thereby condensing and thus polymerizing these components with each other via disulfide bonds in a polymerization condensation or polycondensation to obtain a repetitive component H-[S-P²-S]ₙ-H or H{[S-P²-S]ₐ[S-(AA)ₓ-S]_{b}}H;
b) providing a hydrophilic polymer P¹ and/or P³ as defined according to any of claims 1 to 8, optionally modified with a ligand L and/or an amino acid component (AA)ₓ as defined according to any of claims 1 to 8;
c) mixing the hydrophilic polymer P¹ and/or P³ according to step b) with the repetitive component H-[S-P²-S]ₙ-H or H{[S-P²-S]₂[S-(AA)ₓ-S]_{b}}H obtained according to step a) in a ratio of about 2 : 1, and thereby typically terminating the polymerization condensation or polycondensation reaction and obtaining the inventive polmeric carrier molecule according to formula (IV) or (IVa);
d) optionally purifying the polymeric carrier molecule obtained according to step c);
e) optionally adding a nucleic acid as defined herein to the polymeric carrier obtained according to step c) or d) and complexing the nucleic acid with the polymeric carrier obtained according to step c) or d) to obtain a polymeric carrier cargo complex as defined according to any of claims 9 to 12.

The polymeric carrier molecules and methods of WO2011/026641.

Further preferred cationic or polycationic compounds, which can be used as transfection or complexation agent may include cationic polysaccharides, for example chitosan, polybrene, cationic polymers, e.g. polyethyleneimine (PEI), cationic lipids, e.g. DOTMA: [1-(2,3-sioleyloxy)propyl)]-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-(α-trimethylammonioacetyl)diethanolamine chloride, CLIP1: rac-[(2,3-dioctadecyloxypropyl)(2-hydroxyethyl)]-dimethylammonium chloride, CLIP6: rac-[2(2,3-dihexadecyloxypropyl-oxymethyloxy)ethyl]-trimethylammonium, CLIP9: rac-[2(2,3-dihexadecyloxypropyl-oxysuccinyloxy)ethyl]-trimethylammonium, oligofectamine, or cationic or polycationic polymers, e.g. modified polyaminoacids, such as β-aminoacid-polymers or reversed polyamides, etc., modified polyethylenes, such as PVP (poly(N-ethyl-4-vinylpyridinium bromide)), etc., modified acrylates, such as pDMAEMA (poly(dimethylaminoethyl methylacrylate)), etc., modified Amidoamines such as pAMAM (poly(amidoamine)), etc., modified polybetaaminoester (PBAE), such as diamine end modified 1,4 butanediol diacrylate-co-5-amino-1-pentanol polymers, etc., dendrimers, such as polypropylamine dendrimers or pAMAM based dendrimers, etc., polyimine(s), such as PEI: poly(ethyleneimine), poly(propyleneimine), etc., polyallylamine, sugar backbone based polymers, such as cyclodextrin based polymers, dextran based polymers, chitosan, etc., silan backbone based polymers, such as PMOXA-PDMS copolymers, etc., blockpolymers consisting of a combination of one or more cationic blocks (e.g. selected from a cationic polymer as mentioned above) and of one or more hydrophilic or hydrophobic blocks (e.g polyethyleneglycole); etc.

According to another embodiment, the pharmaceutical composition according to the invention may comprise an adjuvant in order to enhance the immunostimulatory properties of the pharmaceutical composition. In this context, an adjuvant may be understood as any compound, which is suitable to support administration and delivery of the components such as the mRNA molecule or vector comprised in the pharmaceutical composition according to the invention. Furthermore, such an adjuvant may, without being bound thereto, initiate or increase an immune response of the innate immune system, i.e. a non-specific immune response. With other words, when administered, the pharmaceutical composition according to the invention typically initiates an adaptive immune response directed to the antigen encoded by the optimized nucleic acid molecule. Additionally, the pharmaceutical composition according to the invention may generate an (supportive) innate immune response due to addition of an adjuvant as defined herein to the pharmaceutical composition according to the invention.

Such an adjuvant may be selected from any adjuvant known to a skilled person and suitable for the present case, i.e. supporting the induction of an immune response in a mammal. Preferably, the adjuvant may be selected from the group consisting of, without being limited thereto, TDM, MDP, muramyl dipeptide, pluronics, alum solution, aluminium hydroxide, ADJUMER^{™} (polyphosphazene); aluminium phosphate gel; glucans from algae; algammulin; aluminium hydroxide gel (alum); highly protein-adsorbing aluminium hydroxide gel; low viscosity aluminium hydroxide gel; AF or SPT (emulsion of squalane (5%), Tween 80 (0.2%), Pluronic L121 (1.25%), phosphate-buffered saline, pH 7.4); AVRIDINE^{™} (propanediamine); BAY R1005^{™} ((N-(2-deoxy-2-L-leucylamino-b-D-glucopyranosyl)-N-octadecyl-dodecanoyl-amide hydroacetate); CALCITRIOL^{™} (1-alpha,25-dihydroxy-vitamin D3); calcium phosphate gel; CAP^{™} (calcium phosphate nanoparticles); cholera holotoxin, cholera-toxin-A1-protein-A-D-fragment fusion protein, sub-unit B of the cholera toxin; CRL 1005 (block copolymer P1205); cytokine-containing liposomes; DDA (dimethyldioctadecylammonium bromide); DHEA (dehydroepiandrosterone); DMPC (dimyristoylphosphatidylcholine); DMPG (dimyristoylphosphatidylglycerol); DOC/alum complex (deoxycholic acid sodium salt); Freund's complete adjuvant; Freund's incomplete adjuvant; gamma inulin; Gerbu adjuvant (mixture of: i) N-acetylglucosaminyl-(P1-4)-N-acetylmuramyl-L-alanyl-D-glutamine (GMDP), ii) dimethyldioctadecylammonium chloride (DDA), iii) zinc-L-proline salt complex (ZnPro-8); GM-CSF); GMDP (N-acetylglucosaminyl-(b1-4)-N-acetylmuramyl-L-alanyl-D-isoglutamine); imiquimod (1-(2-methypropyl)-1H-imidazo[4,5-c]quinoline-4-amine); ImmTher^{™} (N-acetylglucosaminyl-N-acetylmuramyl-L-Ala-D-isoGlu-L-Ala-glycerol dipalmitate); DRVs (immunoliposomes prepared from dehydration-rehydration vesicles); interferon-gamma; interleukin-1beta; interleukin-2; interleukin-7; interleukin-12; ISCOMS^{™}; ISCOPREP 7.0.3. ^{™}; liposomes; LOXORIBINE^{™} (7-allyl-8-oxoguanosine); LT oral adjuvant (E.coli labile enterotoxin-protoxin); microspheres and microparticles of any composition; MF59^{™}; (squalene-water emulsion); MONTANIDE ISA 51^{™} (purified incomplete Freund's adjuvant); MONTANIDE ISA 720^{™} (metabolisable oil adjuvant); MPL^{™} (3-Q-desacyl-4'-monophosphoryl lipid A); MTP-PE and MTP-PE liposomes ((N-acetyl-L-alanyl-D-isoglutaminyl-L-alanine-2-(1,2-dipalmitoyl-sn-glycero-3-(hydroxyphosphoryloxy))-ethylamide, monosodium salt); MURAMETIDE^{™} (Nac-Mur-L-Ala-D-Gln-OCH3); MURAPALMITINE^{™} and D-MURAPALMITINE^{™} (Nac-Mur-L-Thr-D-isoGln-sn-glyceroldipalmitoyl); NAGO (neuraminidase-galactose oxidase); nanospheres or nanoparticles of any composition; NISVs (non-ionic surfactant vesicles); PLEURAN^{™} (β-glucan); PLGA, PGA and PLA (homo- and co-polymers of lactic acid and glycolic acid; microspheres/nanospheres); PLURONIC L121^{™}; PMMA (polymethyl methacrylate); PODDS^{™} (proteinoid microspheres); polyethylene carbamate derivatives; poly-rA: poly-rU (polyadenylic acid-polyuridylic acid complex); polysorbate 80 (Tween 80); protein cochleates (Avanti Polar Lipids, Inc., Alabaster, AL); STIMULON^{™} (QS-21); Quil-A (Quil-A saponin); S-28463 (4-amino-otec-dimethyl-2-ethoxymethyl-1H-imidazo[4,5 c]quinoline-1-ethanol); SAF-1^{™} ("Syntex adjuvant formulation"); Sendai proteoliposomes and Sendai-containing lipid matrices; Span-85 (sorbitan trioleate); Specol (emulsion of Marcol 52, Span 85 and Tween 85); squalene or Robane^{®} (2,6,10,15,19,23-hexamethyltetracosan and 2,6,10,15,19,23-hexamethyl-2,6,10,14,18,22-tetracosahexane); stearyltyrosine (octadecyltyrosine hydrochloride); Theramid^{®} (N-acetylglucosaminyl-N-acetylmuramyl-L-Ala-D-isoGlu-L-Ala-dipalmitoxypropylamide); Theronyl-MDP (Termurtide^{™} or [thr 1]-MDP; N-acetylmuramyl-L-threonyl-D-isoglutamine); Ty particles (Ty-VLPs or virus-like particles); Walter-Reed liposomes (liposomes containing lipid A adsorbed on aluminium hydroxide), and lipopeptides, including Pam3Cys, in particular aluminium salts, such as Adju-phos, Alhydrogel, Rehydragel; emulsions, including CFA, SAF, IFA, MF59, Provax, TiterMax, Montanide, Vaxfectin; copolymers, including Optivax (CRL1005), L121, Poloaxmer4010), etc.; liposomes, including Stealth, cochleates, including BIORAL; plant derived adjuvants, including QS21, Quil A, Iscomatrix, ISCOM; adjuvants suitable for costimulation including Tomatine, biopolymers, including PLG, PMM, Inulin,; microbe derived adjuvants, including Romurtide, DETOX, MPL, CWS, Mannose, CpG nucleic acid sequences, CpG7909, ligands of human TLR 1-10, ligands of murine TLR 1-13, ISS-1018, IC31, Imidazoquinolines, Ampligen, Ribi529, IMOxine, IRIVs, VLPs, cholera toxin, heat-labile toxin, Pam3Cys, Flagellin, GPI anchor, LNFPIII/Lewis X, antimicrobial peptides, UC-1V150, RSV fusion protein, cdiGMP; and adjuvants suitable as antagonists including CGRP neuropeptide.

Suitable adjuvants may also be selected from cationic or polycationic compounds wherein the adjuvant is preferably prepared upon complexing the mRNA molecule or the vector of the pharmaceutical composition with the cationic or polycationic compound. Association or complexing the mRNA molecule or the vector of the pharmaceutical composition with cationic or polycationic compounds as defined herein preferably provides adjuvant properties and confers a stabilizing effect to the mRNA molecule or the vector of the pharmaceutical composition.

The ratio of nucleic acid (the optimized nucleic acid or vector comprising the same) to cationic or polycationic compound may be calculated on the basis of the nitrogen/phosphate ratio (N/P-ratio) of the entire nucleic acid complex. For example, 1 µg RNA typically contains about 3 nmol phosphate residues, provided the RNA exhibits a statistical distribution of bases. Additionally, 1 µg peptide typically contains about x nmol nitrogen residues, dependent on the molecular weight and the number of basic amino acids. When exemplarily calculated for (Arg)9 (molecular weight 1424 g/mol, 9 nitrogen atoms), 1 µg (Arg)9 contains about 700 pmol (Arg)9 and thus 700 x 9=6300 pmol basic amino acids = 6.3 nmol nitrogen atoms. For a mass ratio of about 1:1 RNA/(Arg)9 an N/P ratio of about 2 can be calculated. When exemplarily calculated for protamine (molecular weight about 4250 g/mol, 21 nitrogen atoms, when protamine from salmon is used) with a mass ratio of about 2:1 with 2 µg RNA, 6 nmol phosphate are to be calculated for the RNA; 1 µg protamine contains about 235 pmol protamine molecules and thus 235 x 21 = 4935 pmol basic nitrogen atoms = 4.9 nmol nitrogen atoms. For a mass ratio of about 2:1 RNA/protamine an N/P ratio of about 0.81 can be calculated. For a mass ratio of about 8:1 RNA/protamine an N/P ratio of about 0.2 can be calculated. In the context of the present invention, an N/P-ratio is preferably in the range of about 0.1-10, preferably in a range of about 0.3-4 and most preferably in a range of about 0.5-2 or 0.7-2 regarding the ratio of nucleic acid:peptide in the complex, and most preferably in the range of about 0.7-1.5.

Patent application WO2010/037539 describes an immunostimulatory composition and methods for the preparation of an immunostimulatory composition. Accordingly, in a preferred embodiment of the invention, the composition is obtained in two separate steps in order to obtain both, an efficient immunostimulatory effect and efficient translation of the mRNA molecule according to the invention. Therein, a so called "adjuvant component" is prepared by complexing - in a first step - the mRNA of the adjuvant component with a cationic or polycationic compound in a specific ratio to form a stable complex. In this context, it is important, that no free cationic or polycationic compound or only a neglibly small amount remains in the adjuvant component after complexing the nucleic acid. Accordingly, the ratio of the nucleic acid and the cationic or polycationic compound in the adjuvant component is typically selected in a range that the nucleic acid is entirely complexed and no free cationic or polycationic compound or only a small amount remains in the composition. Preferably the ratio of the adjuvant component, i.e. the ratio of the nucleic acid to the cationic or polycationic compound is selected from a range of about 6:1 (w/w) to about 0,25:1 (w/w), more preferably from about 5:1 (w/w) to about 0,5:1 (w/w), even more preferably of about 4:1 (w/w) to about 1:1 (w/w) or of about 3:1 (w/w) to about 1:1 (w/w), and most preferably a ratio of about 3:1 (w/w) to about 2:1 (w/w).

According to a preferred embodiment, the mRNA molecule or vector is added in a second step to the complexed mRNA molecule of the adjuvant component in order to form the (immunostimulatory) composition of the invention. Therein, the artificial mRNA molecule or vector as claimed is added as free nucleic acid, i.e. nucleic acid, which is not complexed by other compounds. Prior to addition, the free mRNA molecule or vector is not complexed and will preferably not undergo any detectable or significant complexation reaction upon the addition of the adjuvant component.

Suitable adjuvants may furthermore be selected from nucleic acids having the formula (V): GₗXₘGₙ, wherein: G is guanosine (guanine), uridine (uracil) or an analogue of guanosine (guanine) or uridine (uracil); X is guanosine (guanine), uridine (uracil), adenosine (adenine), thymidine (thymine), cytidine (cytosine) or an analogue of the above-mentioned nucleotides (nucleosides); l is an integer from 1 to 40, wherein when l = 1 G is guanosine (guanine) or an analogue thereof, when l > 1 at least 50% of the nucleotides (nucleosides) are guanosine (guanine) or an analogue thereof; m is an integer and is at least 3; wherein when m = 3 X is uridine (uracil) or an analogue thereof, when m > 3 at least 3 successive uridines (uracils) or analogues of uridine (uracil) occur; n is an integer from 1 to 40, wherein when n = 1 G is guanosine (guanine) or an analogue thereof, when n > 1 at least 50% of the nucleotides (nucleosides) are guanosine (guanine) or an analogue thereof.

Other suitable adjuvants may furthermore be selected from nucleic acids having the formula (VI): CₗXₘCₙ, wherein: C is cytidine (cytosine), uridine (uracil) or an analogue of cytidine (cytosine) or uridine (uracil); X is guanosine (guanine), uridine (uracil), adenosine (adenine), thymidine (thymine), cytidine (cytosine) or an analogue of the above-mentioned nucleotides (nucleosides); l is an integer from 1 to 40, wherein when l = 1 C is cytidine (cytosine) or an analogue thereof, when l > 1 at least 50% of the nucleotides (nucleosides) are cytidine (cytosine) or an analogue thereof; m is an integer and is at least 3; wherein when m = 3 X is uridine (uracil) or an analogue thereof, when m > 3 at least 3 successive uridines (uracils) or analogues of uridine (uracil) occur; n is an integer from 1 to 40, wherein when n = 1 C is cytidine (cytosine) or an analogue thereof, when n > 1 at least 50% of the nucleotides (nucleosides) are cytidine (cytosine) or an analogue thereof.

The pharmaceutical composition as claimed preferably comprises a "safe and effective amount" of the components of the pharmaceutical composition, particularly of the claimed mRNA molecule, the vector and/or the cells as defined herein. As used herein, a "safe and effective amount" means an amount sufficient to significantly induce a positive modification of a disease or disorder as defined herein. At the same time, however, a "safe and effective amount" preferably avoids serious side-effects and permits a sensible relationship between advantage and risk. The determination of these limits typically lies within the scope of sensible medical judgment.

The compounds and ingredients of the pharmaceutical composition of the invention may also be manufactured and traded separately of each other. Thus, also disclosed is a kit or kit of parts comprising an mRNA molecule, a vector, a cell, and/or a pharmaceutical composition as claimed. Preferably, such kit or kits of parts may, additionally, comprise instructions for use, cells for transfection, an adjuvant, a means for administration of the pharmaceutical composition, a pharmaceutically acceptable carrier and/or a pharmaceutically acceptable solution for dissolution or dilution of the optimized nucleic acid molecule, the vector, the cells or the pharmaceutical composition.

### 6.4 Suitability of the present invention for in vivo applications

The mRNA molecules of the present invention are suitable for *in vivo* administration to humans and animals, particularly in medical methods. Gene therapy and genetic vaccination belong to the most promising and quickly developing medical methods of our modern times. They may provide highly specific and individual options for therapy of a large variety of diseases. Particularly, inherited genetic diseases, infectious diseases, neoplasms (e.g. cancer or tumour diseases), autoimmune diseases, inflammatory diseases, diseases of the blood and blood-forming organs, endocrine, nutritional and metabolic diseases, diseases of the nervous system, diseases of the circulatory system, diseases of the respiratory system, diseases of the digestive system, diseases of the skin and subcutaneous tissue, diseases of the musculoskeletal system and connective tissue, and diseases of the genitourinary system, independently if they are inherited or acquired, may be the subject of such treatment approaches. Also, it is envisaged to prevent early onset of such diseases by these approaches.

### 6.5 Medical uses

In a further aspect, the present invention provides the mRNA molecule, the vector, the cell, or the pharmaceutical composition as claimed for use as a medicament, for example, as vaccine (in genetic vaccination) or in gene therapy.

The use can comprise the administration of the mRNA molecule, the vector, the cell, or the pharmaceutical composition as claimed to a patient in need thereof.

The mRNA molecule, the vector, the cell, or the pharmaceutical composition as claimed are particularly suitable for any medical application which makes use of the therapeutic action or effect of peptides, polypeptides or proteins, or where supplementation of a particular peptide or protein is needed or beneficial. Thus, the present invention provides the mRNA molecule, the vector, the cell, or the pharmaceutical composition as claimed for use in the treatment or prevention of diseases or disorders amenable to treatment by the therapeutic action or effect of peptides, polypeptides or proteins or amenable to treatment by supplementation of a particular peptide, polypeptide or protein. For example, the mRNA molecule, the vector, the cell, or the pharmaceutical composition as claimed may be used for the treatment or prevention of genetic diseases, autoimmune diseases, cancerous or tumour-related diseases, infectious diseases, chronic diseases or the like, e.g., by genetic vaccination or gene therapy.

In particular, such therapeutic treatments which benefit from an increased and prolonged presence of therapeutic peptides, polypeptides or proteins or from more immunogenic properties of the therapeutic peptides, polypeptides or proteins in a subject to be treated are especially suitable as medical application in the context of the present invention.. Thus, a particularly suitable medical application for mRNA molecule, the vector according to the present invention, the cell according to the present invention, or the pharmaceutical composition according to the present invention is vaccination. Thus, the present invention provides the mRNA molecule, the vector, the cell, or the pharmaceutical composition as claimed for vaccination of a subject, preferably a mammalian subject, more preferably a human subject. Preferred vaccination treatments are vaccination against infectious diseases, such as bacterial, protozoal or viral infections, and anti-tumour-vaccination. Such vaccination treatments may be prophylactic or therapeutic.

Depending on the disease to be treated or prevented, the protein of interest encoded by the mRNA molecule may be selected. For example, the open reading frame may code for a protein that has to be supplied to a patient suffering from total lack or at least partial loss of function of a protein, such as a patient suffering from a genetic disease. Additionally the open reading frame may be chosen from an ORF coding for a peptide or protein which beneficially influences a disease or the condition of a subject. Furthermore, the open reading frame may code for a peptide or protein which effects down-regulation of a pathological overproduction of a natural peptide or protein or elimination of cells expressing pathologically a protein or peptide. Such lack, loss of function or overproduction may, e.g., occur in the context of tumour and neoplasia, autoimmune diseases, allergies, infections, chronic diseases or the like. Furthermore, the open reading frame may code for an antigen or immunogen, e.g. for an epitope of a pathogen or for a tumour antigen. Thus, in preferred embodiments, the mRNA molecule or the vector as claimed comprises an ORF encoding an amino acid sequence comprising or consisting of an antigen or immunogen, e.g. an epitope of a pathogen or a tumour-associated antigen, a 3'-UTR moiety as described above and/or a 5'-UTR moiety as described above, and optional further components, such as a poly(A) sequence etc.

In the context of medical application, in particular, in the context of vaccination, it is preferred that the mRNA molecule according to the present invention is mRNA since DNA harbours the risk of eliciting an anti-DNA immune response and tends to insert into genomic DNA.

### 6.5.1 Gene Therapy

The main conceptual rationale behind gene therapy (or molecular therapy) is appropriate modulation of impaired gene expression associated with pathological conditions of specific diseases. Pathologically altered gene expression may result in lack or overproduction of essential gene products, for example, signalling factors such as hormones, housekeeping factors, metabolic enzymes, structural proteins or the like. Altered gene expression may not only be due to mis-regulation of transcription and/or translation, but also due to mutations within the ORF coding for a particular protein. Pathological mutations may be caused by e.g. chromosomal aberration, or by more specific mutations, such as point or frame-shift-mutations, all of them resulting in limited functionality and, potentially, total loss of function of the gene product. However, misregulation of transcription or translation may also occur, if mutations affect genes encoding proteins which are involved in the transcriptional or translational machinery of the cell. Such mutations may lead to pathological up- or down-regulation of genes which are - as such - functional. Genes encoding gene products which exert such regulating functions, may be, e.g., transcription factors, signal receptors, messenger proteins or the like. However, loss of function of such genes encoding regulatory proteins may, under certain circumstances, be reversed by artificial introduction of other factors acting further downstream of the impaired gene product. Such gene defects may also be compensated by gene therapy via substitution of the affected gene itself.

The mRNA molecule as claimed can be used as vector for gene therapy.

In particular, the mRNA molecule can be used to encode any kind of protein suitable for use in molecular therapy. Illustrative examples comprise insulin, EPO and the like.

### 6.5.2 Genetic Vaccination

Genetic vaccination allows evoking a desired immune response to selected antigens, such as components of bacterial surfaces, viral particles, tumour antigens or the like. Generally, vaccination is one of the pivotal achievements of modern medicine. However, effective vaccines are currently available only for a limited number of diseases. Accordingly, infections that are not preventable by vaccination still affect millions of people every year.

Commonly, vaccines may be subdivided into "first", "second" and "third" generation vaccines. "First generation" vaccines are, typically, whole-organism vaccines. They are based on either live and attenuated or killed pathogens, e.g. viruses, bacteria or the like. The major drawback of live and attenuated vaccines is the risk for a reversion to life-threatening variants. Thus, although attenuated, such pathogens may still intrinsically bear unpredictable risks. Killed pathogens may not be as effective as desired for generating a specific immune response. In order to minimize these risks, "second generation" vaccines were developed. These are, typically, subunit vaccines, consisting of defined antigens or recombinant protein components which are derived from pathogens.

Genetic vaccines, i.e. vaccines for genetic vaccination, are usually understood as "third generation" vaccines. They are typically composed of genetically engineered nucleic acid molecules which allow expression of peptide or protein (antigen) fragments characteristic for a pathogen or a tumor antigen *in vivo.* Genetic vaccines are expressed upon administration to a patient after uptake by target cells. Expression of the administered nucleic acids results in production of the encoded proteins. In the event these proteins are recognized as foreign by the patient's immune system, an immune response is triggered.

As can be seen from the above, both methods, gene therapy and genetic vaccination, are essentially based on the administration of nucleic acid molecules to a patient and subsequent transcription and/or translation of the encoded genetic information. Alternatively, genetic vaccination or gene therapy may also comprise methods which include isolation of specific body cells from a patient to be treated, subsequent *in ex vivo* transfection of such cells, and re-administration of the treated cells to the patient.

### 6.5.3 Route of administration

The mRNA molecule, the vector, the cell, or the pharmaceutical composition as defined in the claims may be administered orally, parenterally, by inhalation spray, topically, rectally, nasally, buccally, vaginally, via an implanted reservoir or via jet injection. The term parenteral as used herein includes subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, and sublingual injection or infusion techniques. In a preferred embodiment, the mRNA molecule, the vector, the cell, or the pharmaceutical composition as defined in the claims is administered via needle-free injection (e.g. jet injection).

Preferably, the mRNA molecule, the vector, the cell, or the pharmaceutical composition as defined in the claims is administered parenterally, e.g. by parenteral injection, more preferably by subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, sublingual injection or via infusion techniques. Particularly preferred is intradermal and intramuscular injection. Sterile injectable forms of the inventive pharmaceutical composition may be aqueous or oleaginous suspension. These suspensions may be formulated according to techniques known in the art using suitable dispersing or wetting agents and suspending agents. Preferably, the solutions or suspensions are administered via needle-free injection (e.g. jet injection).

The mRNA molecule, the vector, the cell, or the pharmaceutical composition as defined in the claims may also be administered orally in any orally acceptable dosage form including, but not limited to, capsules, tablets, aqueous suspensions or solutions.

The mRNA molecule, the vector, the cell, or the pharmaceutical composition as defined in the claims may also be administered topically, especially when the target of treatment includes areas or organs readily accessible by topical application, e.g. including diseases of the skin or of any other accessible epithelial tissue. Suitable topical formulations are readily prepared for each of these areas or organs. For topical applications, the mRNA molecule, the vector, the cell, or the pharmaceutical composition as claimed may be formulated in a suitable ointment suspended or dissolved in one or more carriers.

In one embodiment, the use as a medicament comprises the step of transfection of mammalian cells, preferably *in vitro* or *ex vivo* transfection of mammalian cells, more preferably *in vitro* transfection of isolated cells of a subject to be treated by the medicament. If the use comprises the *in vitro* transfection of isolated cells, the use as a medicament may further comprise the readministration of the transfected cells to the patient. The use of the inventive optimized nucleic acid molecules or the vector as a medicament may further comprise the step of selection of successfully transfected isolated cells. Thus, it may be beneficial if the vector further comprises a selection marker. Also, the use as a medicament may comprise *in vitro transfection* of isolated cells and purification of an expression-product, i.e. the encoded peptide or protein from these cells. This purified peptide or protein may subsequently be administered to a subject in need thereof.

The following Figures, Sequences and Examples are intended to illustrate the invention further. The the subject matter of the invention is defined in the claims.

### Brief description of the Figures

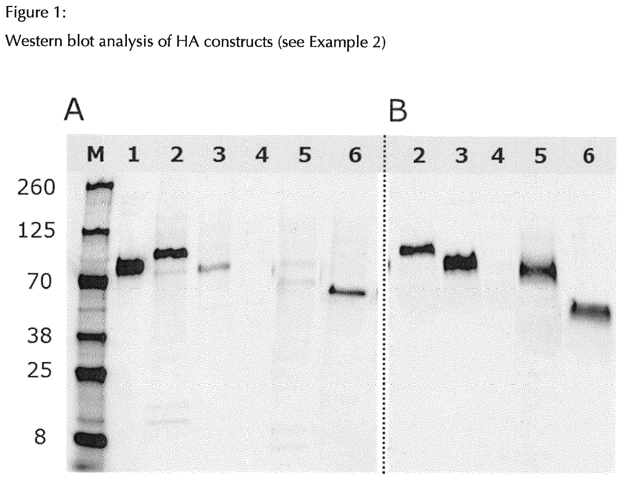

- Figure 1:: shows a western blot to detect HA proteins in cell lysates (A) and cell culture supernatant (B) using an anti HA (H1N1) protein specific antibody. M: protein marker lane; 1: recombinant HA protein (positive control); 2: HA_{ΔTM}-SGG-ferritin; 3: HAΔ_{TM}; 4: negative control; 5: HA_{ΔTM}-C3d_P28; 6: HA_{ΔTM}- foldon. The size ladder (kDa) of the protein marker is shown on the left of panel (A). See Example 2.

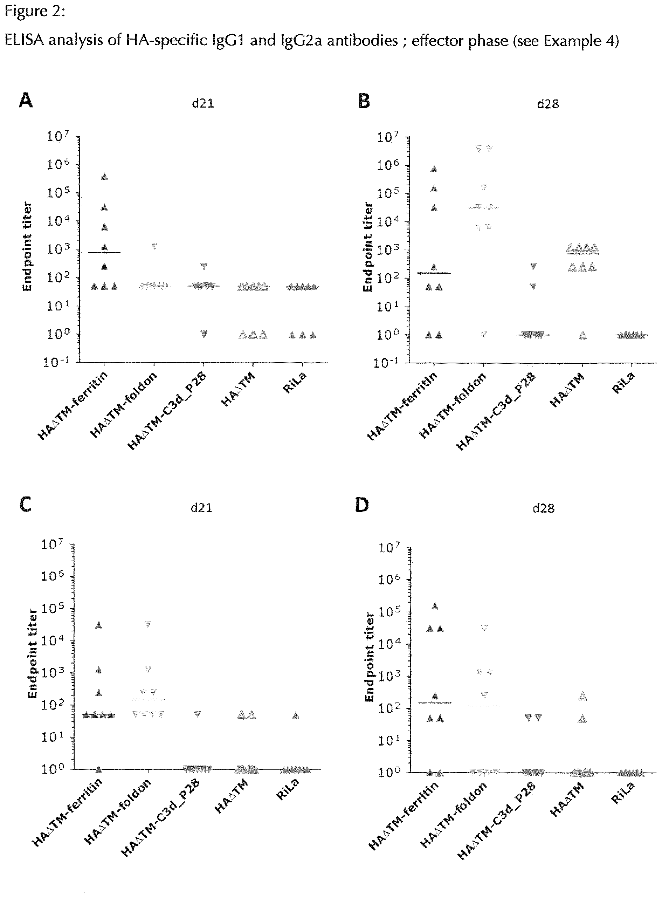

- Figure 2:: shows IgG1 and IgG2a titers of mice immunized with the indicated formulated HA mRNA vaccines. RiLa served as a negative control. Antibody titers were measured at day 21 and day 28. (A) and (B) shows HA-specific IgG1 antibody titers; (C) and (D) shows HA-specific IgG2a antibody titers. The horizontal bar indicates the median. Every data point represents one individual specimen. See Example 4.

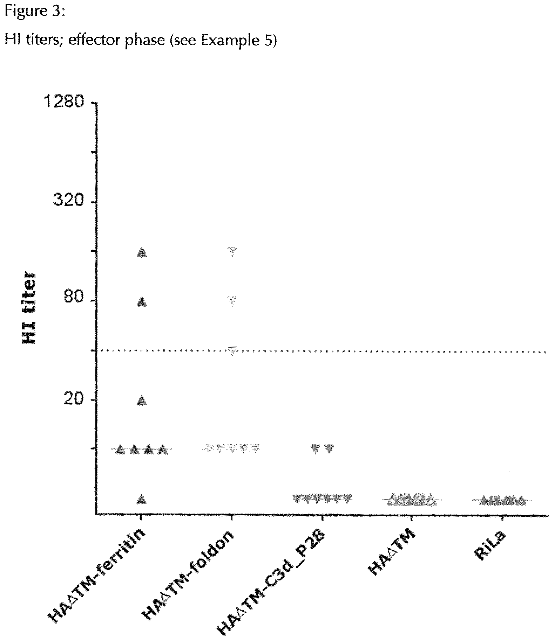

- Figure 3:: shows HI titers of mice immunized with the indicated formulated HA mRNA vaccines. RiLa served as a negative control. HI titers were determined at day 28 (1 week after boost immunization). HI titers of >40 are associated with a protection from influenza virus infection (indicated by dashed line). Every data point represents one individual specimen. See Example 5.
- Figure 4:: shows IgG1 and IgG2a titers of mice immunized with the indicated formulated HA mRNA vaccines. RiLa served as a negative control. Antibody titers were measured at day 35 and day 49. (A) and (B) shows HA-specific IgG1 antibody titers; (C) and (D) shows HA-specific IgG2a antibody titers. The horizontal bar indicates the median. Every data point represents one individual specimen. See Example 6.

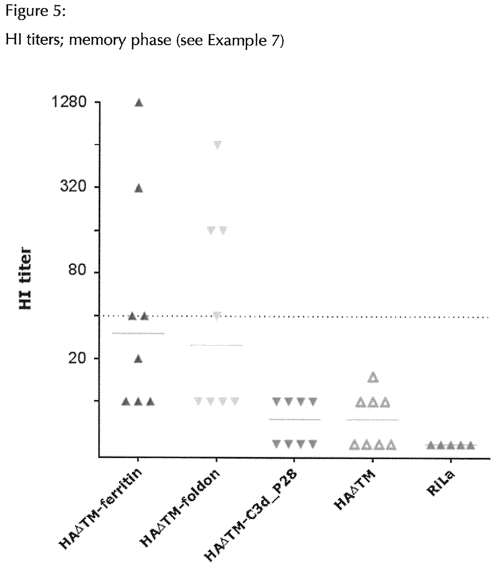

- Figure 5:: shows HI titers of mice immunized with the indicated formulated HA mRNA vaccines. RiLa served as a negative control. HI titers were determined at day 49 (4 weeks after boost immunization). HI titers of >40 are associated with a protection from influenza virus infection (indicated by dashed line). Every data point represents one individual specimen. See Example 7.

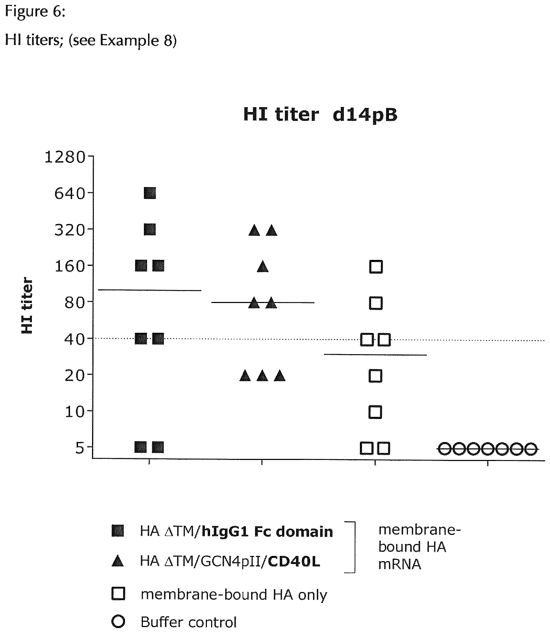

- Figure 6:: shows HI titers of mice immunized with the indicated formulated HA mRNA vaccines. RiLa served as a negative control. HI titers were determined at day 14 (2 weeks after boost immunization (pB)). HI titers of >40 are associated with a protection from influenza virus infection (indicated by dashed line). Every data point represents one individual specimen. See Example 8.

### Examples

### Example 1: Preparation of mRNA HA constructs for in vitro and in vivo experiments

### 1.1. Explanation of the HA constructs:

For the present example, the target protein was the antigen hemagglutinin of *Influenza A virus* (A/Netherlands/602/2009(H1N1); Gl:228860929). The C-terminal transmembrane domain (TM) of the protein was removed (amino acids 531 - 566), hereinafter referred to as HA_{ΔTM}. To potentially promote oligomerization, a non-heme ferritin of *Helicobacter pylori* was fused to the C-terminus of HA_{ΔTM}, separated by a "SGG" spacer sequence, hereinafter referred as HA_{ΔTM}-SGG-ferritin. To potentially promote trimerization, a foldon domain of the fibritin/foldon protein of the bacteriophage T4T was fused to the C-terminus of HA_{ΔTM}, hereinafter referred to as HA_{ΔTM}- foldon. As immunologic adjuvant element, C3d_P28 was fused to the C-terminus of HA_{ΔTM}, hereinafter referred to as HA_{ΔTM}-C3d_P28. To potentially promote multimerization, a human IgG1 Fc domain was fused to the C-terminus of HA_{ΔTM}, hereinafter referred to as HA_{ΔTM}-IgG FC. To obtain targeting of dendritic cells, a CD40 ligand domain was fused to a HA_{ΔTM}, additionally comprising a GCN4pll for trimerization, hereinafter referred to as HA_{ΔTM}-GCN4pll-CD40L). The fusion constructs used in the present example as well as the control constructs are listed with their respective SEQ ID NOs in Table 1.

### 1.2. Preparation of DNA and mRNA constructs

DNA sequences encoding the target element HA_{ΔTM} fused to respective additional elements were prepared and used for subsequent RNA *in vitro* transcription reactions. The constructs are listed in Table 1.

**Table 1: Prepared mRNA HA-fusion constructs**

| Protein construct description | Protein SEQ ID NO | mRNA SEQ ID NO |
|---|---|---|
| HA_{ΔTM} | 1667 | 1663 |
| HA_{ΔTM}-SGG-ferritin | 1670 | 1666 |
| HA_{ΔTM}-foldon | 1669 | 1665 |
| HA_{ΔTM}-C3d_P28 | 1668 | 1664 |
| HA membrane bound | 1735 | 1732 |
| HA_{ΔTM}-lgG FC | 1736 | 1733 |
| HA_{ΔTM}-GCN4pil-CD40L | 1737 | 1734 |

The DNA sequences were prepared by modifying the wild type encoding DNA sequences by introducing a GC-optimized sequence for stabilization. Sequences were introduced into a derived pUC19 vector and modified to comprise stabilizing UTR sequences derived from alpha-globin-3'-UTR (muag (mutated alpha-globin-3'-UTR)), a histone-stem-loop structure, and a stretch of 70 × adenosine at the 3'-terminal end.

### 1.3. RNA in vitro transcription

The respective DNA plasmids were transcribed *in vitro* using DNA dependent T7 RNA polymerase in the presence of a CAP analog (m7GpppG) and a nucleotide mixture. Subsequently, the *in* vitr*o* transcribed mRNA was purified using PureMessenger^{®} (CureVac, Tübingen, Germany; WO2008/077592A1). The obtained mRNA (naked, unformulated mRNA) was used for *in vitro* expression analysis.

### 1.4. Preparation of protamine complexed mRNA vaccine

Prior to use in *in vivo* vaccination experiments, naked mRNA constructs were complexed with protamine. The mRNA complexation consisted of a mixture of 50% naked mRNA and 50% mRNA complexed with protamine at a weight ratio of 2:1. First, mRNA was complexed with protamine by addition of protamine-Ringer's lactate solution to mRNA. After incubation for 10 minutes, when the complexes were stably generated, naked mRNA was added, and the final concentration of the vaccine was adjusted with Ringer's lactate solution. The obtained formulated mRNA vaccine was used for *in vivo* experiments.

### Example 2: Expression of HA constructs in HEK 293T cells and analysis using western blot

The aim of these experiments was to analyse the expression of the HA mRNA constructs (see Table 1) and to determine the release of the HA protein into the supernatant of transfected HEK 293T cells. All HA mRNA vaccine candidates contained an endogenous secretory signal peptide (N-terminus of the HA protein) that should promote the release from the producing cells into the supernatant. Cell lysates were also analyzed for HA protein expression.

### 2.1. Transfection of HEK 293T cells

HEK 293T cells were seeded in a 24-well plate at a density of 70,000 cells/well in cell culture medium (DMEM complete), 48h prior to transfection. Cells were transfected with and 5 µg naked, unformulated mRNA HA constructs (see Table 1) using Lipofectamine 2000 (Invitrogen).

48 hours post transfection, transfection supernatants were collected. Additionally, cells were harvested and lysed with RIPA lysis buffer (50 mM Tris-HCl pH 7.4, 150 mM NaCl, 1 % TritonX-100, 0.1 % SDS). The respective supernatants and cell lysates were stored at -20 °C.

### 2.2. Analysis for HA expression using western blot

An SDS-PAGE was performed with supernatants and whole cell lysates from all samples with Mini-PROTEAN^{®} TGX Precast Mini Gels 7.5% (Bio-Rad). Untransfected cells were used as a negative control. 0.5 µg recombinant H1N1 HA (A/California/04/2009; Sino Biological) was used as a positive control. The blotting on a nitrocellulose membrane was performed for 2h in the presence of a blotting buffer. After blocking the membrane in a respective buffer, antibody incubation (primary and secondary antibodies) and signal detection (LI-COR measurement) was performed. The presence of HA was analyzed using a commercially available mouse monoclonal anti influenza A virus H1N1 specific antibody (Clone 2C10H2, Sino Biological, C) in combination with a goat anti mouse IgG1 IRDye^{®} 800 coupled secondary antibody (LI-COR Biosciences). The presence of tubulin was analyzed either in cell lysates as a loading control or in supernatants to check for cellular contamination using a rabbit anti α/β tubulin antibody (Cell signaling Technology) in combination with a goat anti rabbit IgG IRDye^{®} 680 coupled secondary antibody (LI-COR Biosciences). The approximate protein sizes (without taking post-translational protein modifications into account) are shown in table 2. Western blot results are shown in Figure 1.

**Table 2: Expected protein sizes (approximations) of the HA monomers**

| Protein construct description | Protein size monomer [kDa] |
|---|---|
| HA_{ΔTM} | 59.4 |
| HA_{ΔTM}-SGG-ferritin | 80.7 |
| HA_{ΔTM}-foldon | 64.6 |
| HA_{ΔTM}-C3d_P28 | 75 |

### Results:

For all four tested mRNA constructs, HA protein monomers were detected in cell lysates and/or supernatants (see Figure 1), showing that mRNA constructs were translated into protein. The band sizes were in accordance to the expected band sizes. The majority of protein for all four mRNA constructs was detected in the respective supernatants (see Figure 1B). Since no tubulin protein was detectable in the analyzed supernatants (data not shown), the presence of HA protein was considered to be mediated by secretion triggered by the endogenous secretory signal peptide and not via release by cell death associated with the transfection method. Taken together, all tested mRNA constructs were translated and secreted in HEK 293T cells.

### Example 3: Vaccination of mice with HA constructs

### Immunization

Female BALB/c mice were injected intramuscularly (i.m.) with formulated mRNAs vaccines encoding HA protein constructs with doses indicated in Table 3. As a negative control, one group of mice was vaccinated with buffer (ringer lactate, RiLa). All animals received boost injections on day 21. Blood samples were collected on day 21 and 28 for the analysis of the immune response in the effector phase (see Examples 4-5) and additionally on day 35 and 49 for the analysis of the immune response in the memory phase (see Examples 6-7).

**Table 3: Vaccination regimen for indicated animal groups**

| Group | Number of mice | Injected mRNA HA construct | dose | Vaccination on day |
|---|---|---|---|---|
| 1 | 8 | HA_{ΔTM} | 40µg | 0/21 |
| 2 | 8 | HA_{ΔTM}-SGG-ferritin | 40µg | 0/21 |
| 3 | 8 | HA_{ΔTM}-foldon | 40µg | 0/21 |
| 4 | 8 | HA_{ΔTM}-C3d_P28 | 40µg | 0/21 |
| 5 | 8 | RiLa buffer | | 0/21 |

### Example 4: ELISA analysis of an antigen specific humoral immune response in the effector phase

The aim of this experiment was to assess the antigen specific humoral immune response in vaccinated mice for the used mRNA vaccines and to compare the detected immune response evoked by HA fusion constructs (with additional element) with the immune response evoked by the target HA antigen without additional element. HA protein specific IgG1 and IgG2a antibodies were detected by ELISA using sera obtained at day 21 and day 28 (effector phase).

### Determination of anti HA protein specific IgG1 and IgG2a antibodies by ELISA:

Assessment of an antigen specific immune response was carried out by detecting HA protein specific IgG1 and IgG2 antibodies. MaxiSorp^{®} plates (Nalgene Nunc International) were coated with HA protein (Charles River Laboratories). After blocking with 1×PBS containing 0.05% Tween-20 and 1% BSA the coated plates were incubated with respective mouse serum dilutions. Binding of specific antibodies to the HA antigens was detected using biotinylated isotype specific anti-mouse antibodies followed by streptavidin-HRP (horse radish peroxidase) with ABTS as substrate. For the analysis of an antigen specific immune response in the effector phase, sera obtained at day 21 (three weeks after prime vaccination) and day 28 (one week after boost vaccination) were used. Vaccination was performed according to Example 3. The results are shown in Figure 2.

### Results:

Assessment of the humoral immune response after immunizations revealed that 40 µg of the respective mRNA vaccines induced HA specific IgG1 and IgG2a antibody titers for the HA_{ΔTM}-SGG-ferritin and the HA_{ΔTM}- foldon constructs. The HA_{ΔTM} (without additional element) vaccine induced IgG1 antibodies but not IgG2a antibodies at day 28 (Figure 2B). The HA_{ΔTM}-C3d_P28 vaccine did not induce substantial antibody titers.

Taken together, the addition of ferritin and foldon elements to HA_{ΔTM} substantially improved the induction of a HA specific humoral immune response in the effector phase, whereas the addition of a C3d_P28 element to HA_{ΔTM} had no positive effect.

### Example 5: Hemagglutination inhibition assay to determine virus neutralizing titers in the effector phase

The aim of this experiment was to determine virus neutralizing titers in the collected mice sera (see Example 3) and to compare virus neutralizing titers of mice vaccinated with HA fusion constructs to the titers of mice vaccinated with the target HA antigen without additional element.

### Hemagglutination inhibition assay (HI)

In a 96-well plate, the obtained sera were mixed with HA H1N1 antigen (A/California/07/2009 (H1N1); NIBSC) and red blood cells (4% erythrocytes; Lohmann Tierzucht). In the presence of HA neutralizing antibodies, an inhibition of hemagglutination of erythrocytes can be observed. The lowest level of titered serum that resulted in a visible inhibition of hemagglutination was the assay result. For the analysis of an antigen specific immune response in the effector phase, sera obtained at day 28 (one week after boost vaccination) were used. Vaccination was performed according to Example 3. The results are shown in Figure 3.

### Results:

The results show that potentially protective virus neutralizing titers (>40) were detected for mice vaccinated with the ferritin (2 out of 8 mice) and foldon (3 out of 8 mice) HA fusion constructs, indicating that vaccination with these constructs induced protective neutralizing antibodies in the effector phase. The HA vaccine without additional element and the HA vaccine with a C3d_P28 could not induce virus neutralizing titers.

Taken together, the addition of ferritin and foldon elements to HA_{ΔTM} substantially increased the protective antibody titers in the effector phase, whereas the addition of a C3d_P28 element to HA_{ΔTM} had no positive effect.

### Example 6: ELISA analysis of an antigen specific humoral immune response in the memory phase

### Determination of anti HA protein specific IgG1 and IgG2a antibodies by ELISA:

ELISA was performed according to example 4. Vaccination was performed according to example 3. For the analysis of an antigen specific immune response in the memory phase, sera obtained at day 35 (two week after boost vaccination) and day 49 (four weeks after boost vaccination) were used. The results are shown in Figure 4.

### Results:

Assessment of the humoral immune response after immunizations revealed that 40 µg of the respective mRNA vaccines induced strong HA specific IgG1 and IgG2a antibody titers for the HA_{ΔTM}-SGG-ferritin and the HA_{ΔTM}- foldon constructs. The HA_{ΔTM} vaccine (without additional element) and also the HA_{ΔTM}-C3d_P28 vaccine only induced IgG1 antibodies in few mice. Taken together, the addition of ferritin and foldon elements to HA_{ΔTM} substantially improved the induction of a HA specific humoral immune response in the memory phase, whereas the addition of a C3d_P28 element to HA_{ΔTM} had no positive effect.

### Example 7: Hemagglutination inhibition assay (HI) to determine virus neutralizing titers in the memory phase

The HI assay was performed according to example 5. Vaccination was performed according to example 3. For the analysis virus neutralizing titers in the memory phase, sera obtained at day 49 (four weeks after boost vaccination) was used. The results are shown in Figure 5.

### Results:

The results show that potentially protective virus neutralizing titers (>40) were detected for mice vaccinated with the ferritin (4 out of 8 mice) and foldon (4 out of 8 mice) fusion HA mRNA constructs. As the measurement was performed 4 weeks after boost vaccination, the results suggest that the ferritin and foldon HA fusion constructs were able to induce protective neutralizing titers in the memory phase. Protective HI titers were not detected in HA_{ΔTM}-C3d_P28 and the HA_{ΔTM} vaccinated mice.

Taken together, the addition of ferritin and foldon elements to HA_{ΔTM} could substantially increase the protective antibody titers in the effector phase, whereas the addition of a C3d_P28 element to HA_{ΔTM} had no positive effect.

### Example 8: Hemagglutination inhibition assay to determine functional antibody titers

The Hemagglutination inhibition assay (HI) assay was performed according to example 5. Vaccination was performed according to example 3 on day 0 and day 21. For the HI assay, sera obtained 14 days after boost vaccination were used (day 35). In the experiment, mice were vaccinated with HA_{ΔTM}-IgG1 FC and HA_{ΔTM}-GCN4pll-CD40L, both in combination with a membrane-bound HA. As control, mice were vaccinated only with the membrane-bound HA. The vaccination schemes as well as the used concentrations are provided in Table 4. The results are shown in Figure 6.

**Table 4: Vaccination regimen for indicated animal groups**

| Group | Number of mice | HA fusion construct ( 20 µg) | membrane bound HA ( 20 µg) | Vaccination on day |
|---|---|---|---|---|
| A | 8 | HA_{ΔTM}-IgG FC | Membrane bound HA | 0/21 |
| B | 8 | HA_{ΔTM}-GCN4pll-CD40L | Membrane bound HA | 0/21 |
| C | 8 | ---- | Membrane bound HA | 0/21 |
| D | 8 | RiLa buffer control | | 0/21 |

### Results:

The results show that potentially protective antibody titers (>40) were detected for mice vaccinated with the HA TM-IgG1 FC (6 out of 8 mice) and HA TM-GCN4pll-CD40L (5 out of 8 mice) fusion mRNA constructs in combination with membrane-bound HA (Groups A and B). Compared with a single treatment with membrane bound HA vaccine (4 out of 8) this led to an increase in protective antibody titers (see Figure 6).

### Example 9: EPO half-life extension using EPO fusion constructs

### 9.1. Explanation of the EPO constructs:

For the present example, the target protein is the mice EPO protein (MmEPO; GI:
21389309; NM_007942.2; Uniprot ID P07321; SEQ ID NO: 1738). To extend the half life of the EPO protein, several elements that extend the half life of the protein are C-terminally fused to the EPO protein. The fusion constructs of the present example as well as the control constructs are listed with their respective SEQ ID NOs (fusion proteins and respective RNA coding sequences) in Table 5.

### 9.2. Preparation of EPO DNA and mRNA constructs

The DNA sequences encoding the target EPO (SEQ ID NO: 1771) fused to respective additional half life extension elements are prepared by modifying the wild type encoding DNA sequences by introducing a GC-optimized sequence and/or codon optimized sequence for stabilization and optimized expression. Sequences were introduced into a vector and modified to additionally comprise stabilizing UTR sequences (3' UTR and 5' UTR), a histone-stem-loop structure, a poly-A stretch, and a poly-C stretch at the 3'-terminal end.

The DNA constructs are used as templates for subsequent RNA *in vitro* transcription reactions (see Example 1). Subsequently, the *in vitro* transcribed mRNA is purified using PureMessenger^{®} (CureVac, Tübingen, Germany; WO2008/077592A1).

**Table 5: Prepared EPO-fusion constructs**

| Protein construct description | SEQ ID NOs of EPO-fusion proteins | SEQ ID NOs of RNA coding sequences (cds) |
|---|---|---|
| MmEPO-CgB | 1739 | 1772 |
| MmEPO-Xten | 1740 | 1773 |
| MmEPO-PAS600 | 1741 | 1774 |
| MmEPO-PAS200 | 1742 | 1775 |
| MmEPO-HAP200 | 1743 | 1776 |
| MmEPO-ELP | 1744 | 1777 |
| MmEPO-MmAlb(25-608) | 1745 | 1778 |
| MmEPO-HsAlb(25-609) | 1746 | 1779 |
| MmEPO-HsAlb(25-609_K597P) | 1747 | 1780 |
| MmEPO-linkerG4S-MmAlb(404-608) | 1748 | 1781 |
| MmEPO-ABP-SA21 | 1749 | 1782 |
| MmEPO-SSG148_ABD_SpG_high | 1750 | 1783 |
| MmEPO-HslgG1 | 1751 | 1784 |
| MmEPO-MmIgG1 | 1752 | 1785 |
| MmEPO-MmIgG2 | 1753 | 1786 |
| MmEPO-MmTf(20-697) | 1754 | 1787 |
| MmEPO-HsTf(20-698) | 1755 | 1788 |
| MmEPO-Sa_SpA (121-270) | 1756 | 1789 |
| MmEPO-Hs_monolgG1 | 1757 | 1790 |
| MmEPO-Hs_2x-monolgG1 | 1758 | 1791 |
| MmEPO-IgBD | 1759 | 1792 |
| MmEPO-linkerG4S-lgBP | 1760 | 1793 |
| MmEPO-E-XTEN | 1761 | 1794 |
| MmEPO-ELP(420) | 1762 | 1795 |
| MmEPO-ABP SA15 | 1763 | 1796 |
| MmEPO-ABD SPG | 1764 | 1797 |
| MmEPO-HsAlbDIII (P02768 ; 404-609) | 1765 | 1798 |
| MmEPO-monomeric Mm Fc | 1766 | 1799 |
| MmEPO-tandem monomeric MmFc | 1767 | 1800 |
| MmEPO-HslgG2 | 1768 | 1801 |
| MmEPO-MmlgG2b | 1769 | 1802 |
| MmEPO-HslgG4 | 1770 | 1803 |

### 9.3. Expression of EPO constructs in HEK 293T cells and HeLa cells and analysis using western blot

To characterize the expression of EPO mRNA constructs and to determine the release of the EPO protein into the supernatant of transfected HEK 293T cells and transfected HeLa cells, *in vitro* expression analysis is performed. A detailed description of the experimnets is provided below.

### 9.3.1. Transfection of cells

HEK 293T cells and HeLa cells are seeded in a 24-well plate at a density of 70,000 cells/well in cell culture medium 48h prior to transfection. Cells are transfected with 5 µg naked, unformulated mRNA EPO constructs (see Table 4) using Lipofectamine 2000 (Invitrogen). As a control, full length EPO mRNA construct is used (without half-life extending element). 24 hours post transfection, cell culture supernatants are collected. Additionally, cells are harvested and lysed with RIPA lysis buffer (50 mM Tris-HCl pH 7.4, 150 mM NaCl, 1 % TritonX-100, 0.1 % SDS) or harvested using SDS lysis buffer.

### 9.3.2. Analysis of EPO expression and secretion using western blot and EPO-ELISA

An SDS-PAGE is performed with supernatants and whole cell lysates from all samples with Mini-PROTEAN^{®} TGX Precast Mini Gels 4 - 20 % (Gradient gel; Bio-Rad). Untransfected cells are used as a negative control. The blotting on a nitrocellulose membrane is performed for 2h in the presence of a blotting buffer. After blocking the membrane in a respective buffer, antibody incubation (primary and secondary antibodies) and signal detection (LI-COR measurement) is performed. The presence of EPO is analyzed using a commercially available anti EPO specific antibody in combination with a suitable IgG1 IRDye^{®} 800 coupled secondary antibody (LI-COR Biosciences). Additionally, EPO levels in the culture medium are quantitatively measured 24 hours post transfection using a commercially available mouse EPO ELISA kit (R&D Systems, Wiesbaden, Germany). The constructs showing suitable expression and secretion characteristics are used in *in* vivo experiments.

### 9.3. In vivo characterization of TransIT formulated EPO constructs

To characterize the half-life of the generated EPO-fusion proteins, mRNA encoding said fusion proteins is formulated for in vivo application using TransIT and injected intraperitoneal or intraveneously into female BALB/c mice in equimolar amounts. As control, EPO protein and TransIT formulated EPO mRNA (without half-life extending element) are used. 6 hours, 1 day, 4 days and 7 days after injection, a few microliters of blood are collected, heparinized, and centrifuged. EPO levels in the supernatant are measured using a mouse EPO ELISA kit (R&D Systems). In addition, reticulocytes are analysed using a commercially available Retic-COUNT kit (BD Biosciences, Heidelberg, Germany) according to the manufacturer's instructions. Stained cells are analyzed on a FACS Canto (BD Biosciences). Reticulocyte levels are given as percentage of total red blood cells.

## Claims

1. mRNA molecule suitable for application in gene therapy and/or genetic vaccination, the mRNA molecule comprising a 5'-untranslated region (5'-UTR) and a 3'-untranslated region (3'-UTR), wherein the mRNA comprises at least two modules,
wherein each module is a ribonucleic acid moiety,
wherein at least one module is an open reading frame (ORF) encoding a polypeptide or protein of interest, and
wherein at least one module is a further coding ribonucleic acid moiety encoding at least one additional polypeptide or protein element encoded in the same open reading frame as the polypeptide or protein of interest, wherein the at least one additional polypeptide or protein element is an element that extends protein half-life, and wherein the polypeptide or protein element is **characterized by** at least 80% identity to SEQ ID NO. 1705, and
wherein the open reading frame is or comprises a G/C-modified nucleic acid sequence, wherein the number of G and/or C nucleotides is increased as compared to the wild-type sequence.

2. mRNA molecule according to claim 1, wherein the codon usage of the open reading frame is adapted by modifying the coding region compared to the corresponding wild-type coding region such that at least one codon of the wild-type sequence which codes for a tRNA which is relatively rare in the cell is exchanged for a codon which codes for a tRNA which is comparably frequent in the cell and carries the same amino acid as the relatively rare tRNA.

3. mRNA molecule according to claim 1 or 2, wherein the polypeptide or protein of interest is selected from the group comprising therapeutic proteins, therapeutic polypeptides, allergens, autoimmune antigens, pathogenic antigens, and tumour antigens.

4. mRNA molecule according to any one of the preceding claims, wherein the mRNA molecule comprises at least one chemical modification selected from a sugar modification, a backbone modification, a base modification, a lipid modification and/or a modification of the 5'-end of the mRNA molecule.

5. mRNA molecule according to any one of the preceding claims, comprising at least one non-coding moiety, selected from one or more miRNA moieties, one or more IRES moieties, a histone stem loop, a 5'-Cap, a poly(C) sequence, a polyadenylation signal and/or a poly(A) sequence.

6. mRNA molecule according to any one of the preceding claims, wherein the 5'-UTR is derived from the 5'-UTR of a TOP gene or from a fragment, homologue or variant of the 5'-UTR of a TOP gene, and/or
wherein the 3'-UTR is derived from a 3'-UTR of a gene selected from the group consisting of an albumin gene, an α-globin gene, a β-globin gene, a tyrosine hydroxylase gene, a lipoxygenase gene, and a collagen alpha gene.

7. mRNA molecule according to any one of the preceding claims, wherein the mRNA molecule encodes at least 2, at least 3, at least 4, at least 5, at least 6, at least 7, at least 8, at least 9, or at least 10 polypeptide elements or protein elements, encoded by the open reading frame (ORF) encoding the polypeptide or protein of interest.

8. Method for preparing an mRNA molecule suitable for application in gene therapy and/or genetic vaccination, comprising at least the step of combining at least two modules, wherein each module is a ribonucleic acid moiety, and thereby preparing an mRNA molecule comprising a 5'-untranslated region (5'-UTR), a 3'-untranslated region (3'-UTR) and said at least two modules,
wherein at least one module is an open reading frame encoding a polypeptide or protein of interest,
wherein at least one module is a further coding ribonucleic acid moiety encoding at least one polypeptide or protein element encoded in the same open reading frame as the polypeptide of interest, wherein the at least one polypeptide or protein element is an element that extends protein half-life, wherein the polypeptide or protein element is **characterized by** at least 80% identity to SEQ ID NO. 1705, and
wherein the open reading frame is or comprises a G/C-modified nucleic acid sequence, wherein the number of G and/or C nucleotides is increased as compared to the wild-type sequence.

9. Method according to claim 8, whereby at least one ribonucleotide is altered by substitution or addition,
(i) wherein substitution is **characterized in that** one building block of the nucleic acid molecule is replaced by a different building block, preferably selected from the following:
(i-a) a sugar building block of the nucleic acid molecule is replaced by a different sugar building block, or
(i-b) a backbone building block of the nucleic acid molecule is replaced by a different backbone building block, or
(i-c) a base building block of the nucleic acid molecule is replaced by a different base building block,
or
(ii) wherein adding is **characterized in that**
(ii-a) a lipid building block is added to the nucleic acid molecule, or
(ii-b) a 5'-Cap is added to the nucleic acid molecule.

10. Method according to claim 9, wherein the at least one ribonucleotide is substituted or added at the stage of preparing the mRNA molecule.

11. Method according to any one of claims 8 to 10, additionally comprising
(i) a step of designing an mRNA molecule having desired properties, or
(ii) a first step of designing a protein or polypeptide having desired properties, followed by a second step of deducing a nucleic acid sequence that encodes said protein or polypeptide, thereby designing an mRNA molecule encoding desired properties;
and wherein the designing of the mRNA molecule according to (i) or (ii) is followed by preparing the designed mRNA molecule, comprising the method steps defined in claim 9 or claim 10.

12. Method according to any one of claims 8 - 11, wherein the mRNA molecule resulting from the preparation is an mRNA molecule as defined in any one of claims 1 - 7.

13. Vector encoding an mRNA molecule of any one of claims 1 - 7.

14. Cell comprising an mRNA molecule of any one of claims 1 - 7, or a vector according to claim 13.

15. Pharmaceutical composition comprising:
an mRNA molecule according to any one of claims 1 - 7, a vector according to claim 13, or a cell according to claim 14,
and a pharmaceutically acceptable carrier.

16. mRNA molecule according to any one of claims 1 - 7, vector according to claim 13, cell according to claim 14 or pharmaceutical composition according to claim 15 for use in a method of treatment of the human or animal body by therapy.

17. mRNA molecule according to any one of claims 1 - 7, vector according to claim 13, cell according to claim 14 or pharmaceutical composition according to claim 15 for use in a method of gene therapy or for use in a method of genetic vaccination.

## Patentansprüche

1. mRNA-Molekül, das zur Anwendung in der Gentherapie und/oder genetischen Impfung geeignet ist, wobei das mRNA-Molekül eine 5'-nicht-translatierte Region (5'-UTR) und eine 3'-nicht-translatierte Region (3'-UTR) umfasst, wobei die mRNA mindestens zwei Module umfasst,
wobei jedes Modul eine Ribonukleinsäure-Einheit ist,
wobei mindestens ein Modul ein offener Leserahmen (ORF) ist, der für ein Polypeptid oder Protein von Interesse kodiert, und
wobei mindestens ein Modul eine weitere kodierende Ribonukleinsäure-Einheit ist, die für mindestens ein zusätzliches Polypeptid- oder Proteinelement kodiert, das in demselben offenen Leserahmen wie das Polypeptid oder Protein von Interesse kodiert wird, wobei das mindestens eine zusätzliche Polypeptid- oder Proteinelement ein Element ist, das die Protein-Halbwertszeit verlängert, und wobei das Polypeptid- oder Proteinelement durch mindestens 80 % Identität mit SEQ ID NO. 1705 gekennzeichnet ist, und
wobei der offene Leserahmen eine G/C-modifizierte Nukleinsäuresequenz ist oder umfasst, wobei die Anzahl der G- und/oder C-Nukleotide im Vergleich zur Wildtyp-Sequenz erhöht ist.

2. mRNA-Molekül nach Anspruch 1, wobei die Codon-Nutzung des offenen Leserahmens angepasst wird, indem die kodierende Region im Vergleich zur entsprechenden Wildtyp-kodierenden Region so modifiziert wird, dass mindestens ein Codon der Wildtyp-Sequenz, das für eine tRNA kodiert, die in der Zelle relativ selten ist, gegen ein Codon ausgetauscht wird, das für eine tRNA kodiert, die in der Zelle vergleichsweise häufig ist und die gleiche Aminosäure trägt wie die relativ seltene tRNA.

3. mRNA-Molekül nach Anspruch 1 oder 2, wobei das Polypeptid oder Protein von Interesse aus der Gruppe ausgewählt ist, die therapeutische Proteine, therapeutische Polypeptide, Allergene, Autoimmun-Antigene, pathogene Antigene und Tumor-Antigene umfasst.

4. mRNA-Molekül nach einem der vorhergehenden Ansprüche, wobei das mRNA-Molekül mindestens eine chemische Modifikation umfasst, die aus einer Zucker-Modifikation, einer Rückgrat-Modifikation, einer Basen-Modifikation, einer Lipid-Modifikation und/oder einer Modifikation des 5'-Endes des mRNA-Moleküls ausgewählt ist.

5. mRNA-Molekül nach einem der vorhergehenden Ansprüche, umfassend mindestens eine nicht-kodierende Einheit, ausgewählt aus einer oder mehreren miRNA-Einheiten, einer oder mehreren IRES-Einheiten, einer Histon-Stammschleife, einem 5'-Cap, einer Poly(C)-Sequenz, einem Polyadenylierungssignal und/oder einer Poly(A)-Sequenz.

6. mRNA-Molekül nach einem der vorhergehenden Ansprüche, wobei die 5'-UTR von der 5'-UTR eines TOP-Gens oder von einem Fragment, Homolog oder einer Variante der 5'-UTR eines TOP-Gens abgeleitet ist, und/oder
wobei die 3'-UTR von einer 3'-UTR eines Gens abgeleitet ist, das aus der Gruppe ausgewählt ist, die aus einem Albumin-Gen, einem α-Globin-Gen, einem β-Globin-Gen, einem Tyrosin-Hydroxylase-Gen, einem Lipoxygenase-Gen und einem Kollagen-alpha-Gen besteht.

7. mRNA-Molekül nach einem der vorhergehenden Ansprüche, wobei das mRNA-Molekül für mindestens 2, mindestens 3, mindestens 4, mindestens 5, mindestens 6, mindestens 7, mindestens 8, mindestens 9 oder mindestens 10 Polypeptidelemente oder Proteinelemente kodiert, die von dem offenen Leseraster (ORF) kodiert werden, der für das Polypeptid oder Protein von Interesse kodiert.

8. Verfahren zur Herstellung eines mRNA-Moleküls, das zur Anwendung in der Gentherapie und/oder genetischen Impfung geeignet ist, umfassend mindestens den Schritt des Kombinierens von mindestens zwei Modulen, wobei jedes Modul eine Ribonukleinsäure-Einheit ist, und dadurch Herstellen eines mRNA-Moleküls, umfassend eine 5'-nicht-translatierte Region (5'-UTR), eine 3'-nicht-translatierte Region (3'-UTR) und die mindestens zwei Module,
wobei mindestens ein Modul ein offener Leserahmen ist, der für ein Polypeptid oder Protein von Interesse kodiert,
wobei mindestens ein Modul eine weitere kodierende Ribonukleinsäure-Einheit ist, die für mindestens ein Polypeptid- oder Proteinelement kodiert, das in demselben offenen Leserahmen wie das Polypeptid von Interesse kodiert wird, wobei das mindestens eine Polypeptid- oder Proteinelement ein Element ist, das die Protein-Halbwertszeit verlängert, wobei das Polypeptid- oder Proteinelement durch mindestens 80 % Identität mit SEQ ID NO. 1705 gekennzeichnet ist, und
wobei der offene Leserahmen eine G/C-modifizierte Nukleinsäuresequenz ist oder umfasst, wobei die Anzahl der G- und/oder C-Nukleotide im Vergleich zur Wildtypsequenz erhöht ist.

9. Verfahren nach Anspruch 8, wobei mindestens ein Ribonukleotid durch Substitution oder Addition verändert wird,
(i) wobei die Substitution **dadurch gekennzeichnet ist, dass** ein Baustein des Nukleinsäuremoleküls durch einen anderen Baustein, vorzugsweise ausgewählt aus den folgenden, ersetzt wird:
(i-a) ein Zucker-Baustein des Nukleinsäuremoleküls durch einen anderen Zucker-Baustein ersetzt wird, oder
(i-b) ein Rückgrat-Baustein des Nukleinsäuremoleküls durch einen anderen Rückgrat-Baustein ersetzt wird, oder
(i-c) ein Basen-Baustein des Nukleinsäuremoleküls durch einen anderen Basen-Baustein ersetzt wird,
oder
(ii) wobei das Hinzufügen **dadurch gekennzeichnet ist, dass**
(ii-a) dem Nukleinsäuremolekül ein Lipid-Baustein hinzugefügt wird, oder
(ii-b) dem Nukleinsäuremolekül ein 5'-Cap hinzugefügt wird.

10. Verfahren nach Anspruch 9, wobei das mindestens eine Ribonukleotid auf der Stufe der Herstellung des mRNA-Moleküls substituiert oder hinzugefügt wird.

11. Verfahren nach einem der Ansprüche 8 bis 10, zusätzlich umfassend
(i) einen Schritt des Designs eines mRNA-Moleküls mit gewünschten Eigenschaften, oder
(ii) einen ersten Schritt des Designs eines Proteins oder Polypeptids mit gewünschten Eigenschaften, gefolgt von einem zweiten Schritt der Ableitung einer Nukleinsäuresequenz, die für dieses Protein oder Polypeptid kodiert, wodurch ein mRNA-Molekül entworfen wird, das für gewünschte Eigenschaften kodiert;
und wobei auf das Design des mRNA-Moleküls gemäß (i) oder (ii) die Herstellung des entworfenen mRNA-Moleküls folgt, umfassend die in Anspruch 9 oder Anspruch 10 definierten Verfahrensschritte.

12. Verfahren nach einem der Ansprüche 8 bis 11, wobei das aus der Herstellung resultierende mRNA-Molekül ein mRNA-Molekül ist, wie in einem der Ansprüche 1 bis 7 definiert.

13. Vektor, der für ein mRNA-Molekül nach einem der Ansprüche 1 bis 7 kodiert.

14. Zelle, die ein mRNA-Molekül nach einem der Ansprüche 1 bis 7 oder einen Vektor nach Anspruch 13 umfasst.

15. Pharmazeutische Zusammensetzung, umfassend:
ein mRNA-Molekül nach einem der Ansprüche 1 bis 7, einen Vektor nach Anspruch 13 oder eine Zelle nach Anspruch 14,
und einem pharmazeutisch akzeptablen Träger.

16. mRNA-Molekül nach einem der Ansprüche 1 bis 7, Vektor nach Anspruch 13, Zelle nach Anspruch 14 oder pharmazeutische Zusammensetzung nach Anspruch 15 zur Verwendung in einem Verfahren zur Behandlung des menschlichen oder tierischen Körpers durch Therapie.

17. mRNA-Molekül nach einem der Ansprüche 1 bis 7, Vektor nach Anspruch 13, Zelle nach Anspruch 14 oder pharmazeutische Zusammensetzung nach Anspruch 15 zur Verwendung in einem gentherapeutischen Verfahren oder zur Verwendung in einem Verfahren zur genetischen Impfung.

## Revendications

1. Molécule d'ARNm appropriée pour une application en thérapie génique et/ou en vaccination génétique, la molécule d'ARNm comprenant une région 5' non traduite (5'-UTR) et une région 3' non traduite (3'-UTR), dans laquelle l'ARNm comprend au moins deux modules,
dans laquelle chaque module est une fraction d'acide ribonucléique,
dans laquelle au moins un module est un cadre de lecture ouvert (ORF) codant pour un polypeptide ou une protéine d'intérêt, et
dans laquelle au moins un module est une autre fraction d'acide ribonucléique codante, codant pour au moins un élément polypeptidique ou protéique supplémentaire codé dans le même cadre de lecture ouvert que le polypeptide ou la protéine d'intérêt, dans laquelle ledit au moins un élément polypeptidique ou protéique supplémentaire est un élément qui prolonge la demi-vie de protéine, et dans laquelle ledit élément polypeptidique ou protéique est **caractérisé par** une identité d'au moins 80 % avec SEQ ID NO. 1705, et
dans laquelle le cadre de lecture ouvert est ou comprend une séquence d'acide nucléique modifiée par G/C, dans laquelle le nombre de nucléotides G et/ou C est augmenté par rapport à la séquence de type sauvage.

2. Molécule d'ARNm selon la revendication 1, dans laquelle l'utilisation des codons du cadre de lecture ouvert est adaptée par modification de la région codante par rapport à la région codante de type sauvage correspondante de telle sorte qu'au moins un codon de la séquence de type sauvage qui code pour un ARNt qui est relativement rare dans la cellule est échangé contre un codon qui code pour un ARNt qui est comparativement fréquent dans la cellule et porte le même acide aminé que l'ARNt relativement rare.

3. Molécule d'ARNm selon la revendication 1 ou 2, dans laquelle le polypeptide ou la protéine d'intérêt est choisi dans le groupe comprenant les protéines thérapeutiques, les polypeptides thérapeutiques, les allergènes, les antigènes auto-immuns, les antigènes pathogènes et les antigènes tumoraux.

4. Molécule d'ARNm selon l'une quelconque des revendications précédentes, dans laquelle la molécule d'ARNm comprend au moins une modification chimique choisie parmi une modification de sucre, une modification de squelette, une modification de base, une modification de lipide et/ou une modification de l'extrémité 5' de la molécule d'ARNm.

5. Molécule d'ARNm selon l'une quelconque des revendications précédentes, comprenant au moins une fraction non codante, choisie parmi une ou plusieurs fractions de miARN, une ou plusieurs fractions IRES, une tige-boucle d'histone, une 5'-Cap, une séquence poly(C), un signal de polyadénylation et/ou une séquence poly(A).

6. Molécule d'ARNm selon l'une quelconque des revendications précédentes, dans laquelle la 5'-UTR est dérivée de la 5'-UTR d'un gène TOP ou d'un fragment, d'un homologue ou d'un variant de la 5'-UTR d'un gène TOP, et/ou dans laquelle la 3'-UTR est dérivée d'une 3'-UTR d'un gène choisi dans le groupe constitué par un gène d'albumine, un gène d'α-globine, un gène de β-globine, un gène de tyrosine hydroxylase, un gène de lipoxygénase et un gène de collagène alpha.

7. Molécule d'ARNm selon l'une quelconque des revendications précédentes, dans laquelle la molécule d'ARNm code pour au moins 2, au moins 3, au moins 4, au moins 5, au moins 6, au moins 7, au moins 8, au moins 9 ou au moins 10 éléments polypeptidiques ou éléments protéiques, codés par le cadre de lecture ouvert (ORF) codant pour le polypeptide ou la protéine d'intérêt.

8. Procédé de préparation d'une molécule d'ARNm appropriée pour une application en thérapie génique et/ou en vaccination génétique, comprenant au moins l'étape de combinaison d'au moins deux modules, dans lequel chaque module est une fraction d'acide ribonucléique, et préparation ainsi d'une molécule d'ARNm comprenant une région 5' non traduite (5'-UTR), une région 3' non traduite (3'-UTR) et lesdits au moins deux modules,
dans lequel au moins un module est un cadre de lecture ouvert codant pour un polypeptide ou une protéine d'intérêt,
dans lequel au moins un module est une autre fraction d'acide ribonucléique codante, codant pour au moins un élément polypeptidique ou protéique codé dans le même cadre de lecture ouvert que le polypeptide d'intérêt, dans lequel ledit au moins un élément polypeptidique ou protéique est un élément qui prolonge la demi-vie de protéine, dans lequel l'élément polypeptidique ou protéique est **caractérisé par** une identité d'au moins 80 % avec SEQ ID NO. 1705, et
dans lequel le cadre de lecture ouvert est ou comprend une séquence d'acide nucléique modifiée par G/C, dans laquelle le nombre de nucléotides G et/ou C est augmenté par rapport à la séquence de type sauvage.

9. Procédé selon la revendication 8, grâce auquel au moins un ribonucléotide est modifié par substitution ou addition,
(i) dans lequel la substitution est **caractérisée en ce qu'**un bloc de construction de la molécule d'acide nucléique est remplacé par un bloc de construction différent, choisi de préférence parmi les suivants :
(i-a) un bloc de construction sucre de la molécule d'acide nucléique est remplacé par un bloc de construction sucre différent, ou
(i-b) un bloc de construction squelette de la molécule d'acide nucléique est remplacé par un bloc de construction squelette différent, ou
(i-c) un bloc de construction base de la molécule d'acide nucléique est remplacé par un bloc de construction base différent,
ou
(ii) dans lequel l'addition est **caractérisée en ce que**
(ii-a) un bloc de construction lipide est ajouté à la molécule d'acide nucléique, ou
(ii-b) une 5'-Cap est ajoutée à la molécule d'acide nucléique.

10. Procédé selon la revendication 9, dans lequel l'au moins un ribonucléotide est substitué ou ajouté au stade de préparation de la molécule d'ARNm.

11. Procédé selon l'une quelconque des revendications 8 à 10, comprenant en outre
(i) une étape de conception d'une molécule d'ARNm présentant des propriétés souhaitées, ou
(ii) une première étape de conception d'une protéine ou d'un polypeptide présentant des propriétés souhaitées, suivie d'une seconde étape de déduction d'une séquence d'acide nucléique qui code pour ladite protéine ou ledit polypeptide, concevant ainsi une molécule d'ARNm codant pour des propriétés souhaitées ;
et dans lequel la conception de la molécule d'ARNm selon (i) ou (ii) est suivie de la préparation de la molécule d'ARNm conçue, comprenant les étapes de procédé définies dans la revendication 9 ou la revendication 10.

12. Procédé selon l'une quelconque des revendications 8 à 11, dans lequel la molécule d'ARNm résultant de la préparation est une molécule d'ARNm telle que définie dans l'une quelconque des revendications 1 à 7.

13. Vecteur codant pour une molécule d'ARNm selon l'une quelconque des revendications 1 à 7.

14. Cellule comprenant une molécule d'ARNm selon l'une quelconque des revendications 1 à 7, ou un vecteur selon la revendication 13.

15. Composition pharmaceutique comprenant :
une molécule d'ARNm selon l'une quelconque des revendications 1 à 7, un vecteur selon la revendication 13, ou une cellule selon la revendication 14,
et un support pharmaceutiquement acceptable.

16. Molécule d'ARNm selon l'une quelconque des revendications 1 à 7, vecteur selon la revendication 13, cellule selon la revendication 14 ou composition pharmaceutique selon la revendication 15 pour une utilisation dans une méthode de traitement du corps humain ou animal par thérapie.

17. Molécule d'ARNm selon l'une quelconque des revendications 1 à 7, vecteur selon la revendication 13, cellule selon la revendication 14 ou composition pharmaceutique selon la revendication 15 pour une utilisation dans une méthode de thérapie génique ou pour une utilisation dans une méthode de vaccination génétique.
